# EUROPEAN PATENT APPLICATION

(11) **EP 3 750 997 A1**
(43) Date of publication of application: **16.12.2020**
(21) Application number: 20172762.5
(22) Date of filing: 02.12.2014
(51) Int. Cl.: C12N 15/113, A61K 31/7088

(54) **ANTISENSE COMPOUNDS AND USES THEREOF**

(30) Priority: 02.12.2013 US 201361910871 P
(62) Divisional of application: 14867872.5
(71) Applicant: Ionis Pharmaceuticals, Inc., Carlsbad, CA 92010 (US); Rosalind Franklin University of Medicine and Science, North Chicago, IL 60064 (US)
(72) Inventor: RIGO, Frank, Carlsbad, CA 92010 (US); HASTINGS, Michelle, L., North Chicago, IL 60064 (US)
(74) Representative: Chapman, Desmond Mark

(57) **Abstract**

The present invention provides compounds comprising oligonucleotides complementary to a CLN3 transcript. Certain such compounds are useful for hybridizing to a CLN3 transcript, including but not limited to a CLN3 transcript in a cell. In certain embodiments, such hybridization results in modulation of splicing of the CLN3 transcript. In certain embodiments, such compounds are used to treat one or more symptoms associated with Batten Disease.

SEQUENCE LISTING

<110>  Isis Pharmaceuticals, Inc.
       Rosalind Franklin University of Medicine and Science

<120>  ANTISENSE COMPOUNDS AND USES THEREOF

<130>  CORE0120WO

<150>  61/910,871
<151>  2013-12-03

<160>  127

<170>  PatentIn version 3.5

<210>  1
<211>  17021
<212>  DNA
<213>  Homo sapiens

<400>  1
agcactttgg gaagccaagg caggtggatt gcttgagctc aggagtttga ggccaccctg      60

ggcaacgtgg caaaatccag tctctacaaa aaacacaaaa attagctggg cacagtggtg     120

cgcacctgct gtcccagcta ctcgggaggc tgaggtggga ggatcacctg agcctgggag     180

gtcaaggttg cagcaaacca agatcctgcc actgcactcc agcctgggca acagagcaaa     240

accctgtcaa aaaaaaaaaa aaaatctggt ctaagcacca cctttgatgg aggcaggaat     300

cctgcagcag cctggagcca acccaatggc tccctgcctc tggtgctcta tttcttatca     360

ccatacaggt ctctaaggtc acctttaggt ctaacattgt atgaatgatt gtcagcaact     420

ttccaatttt tttcatatac atatattttt tttattttat tttttacttt ttcttttttt     480

gagacaggat ctcactctgt cacccaggct ggagtgcagt ggcgtgatca tagctcactg     540

tagcttcaac ctcctgggca caagtgatcc tcccacctca gcttccaagg tacctgtgac     600

tacaggaatg cgccaccatg cccggctaat tttgttatat atatatatat atatatttgt     660

accttggttt tctcatctgt aacaccaggg gttgaagtgc tgtgatattt tttggttcta     720

ggataatttg gaaataggga tctcttaaca tcttggatac tgccaataga tcattcacga     780

aatcccttta gatgagtgtt atgtttactt aggtctctaa aacaatttgt aaggtcaaca     840

gatggggata agaatgacct gaagctggtc gtctttgttc gcaaccagaa acagtcctgc     900

ctcaaaaaaa aaaaaaaaaa aaagggagta ggcaggtggc catatttgtt tctggcaagt     960

gagtgctgaa ggaaaggagc tgaagcctcc cacagtcata actggtgctg gcaggctact    1020

gtctcggtct tgggcgccac tgatctaagg tcacggctct gcttgctgct cccacccgct    1080

ccagtttaaa acctgcggtt ccagggttct ccagcccctc cctttttcac gctccgaagc    1140

cgagaaggcc caaagcgaag acagagagga cccggaagta gggaaaacct ctgagcacgt    1200

gatgggggaa cacgcgggtg ctgtcacgtg atccgacaaa cggcctctgc atagtgcaga    1260

acattctgct gctcttaaag gtacaggcct cagggtccct gctgtagacg gggcggggga 1320

gagtacgatg ggtggggcgt ggtgggtcgt agggcgctcg agatggagcc cccagcttcc 1380

ttgatggatc gcggggcgcg agtgccctag acaagccgga gctgggaccg gcaatcgggc 1440

gttgatcctt gtcacctgtc gcagaccctc atccctcccg tgggagcccc ctttggacac 1500

tctatgaccc tggaccctcg ggggacctga acttgatgcg atgggaggct gtgcaggctc 1560

gcggcggcgc ttttcggatt ccgagggtga gtattcccgc ccaccctcat ggaacgacca 1620

cctctggctc gcagcccacc tgaggggaat gagagctgac tcggccccgg gaggacacgc 1680

aaggagcggt cgtgcacctg agagtaggtg ggggtcatgc cctcttctcg ctctcccagg 1740

ggaggagacc gtcccggagc cccggctccc tctgttggac catcagggcg cgcattggaa 1800

gaacgcggtg ggcttctggt gagtggcctg agacttcagc gagtgacaat ggcatgagaa 1860

gagggaagtg accggaggaa agggggaaga aagagttaag ctgcgcaaag gagctgaacc 1920

cacaaatatt tactgaatcc actggtttgc cccaggggca gcatgtaaaa tttccaaact 1980

tcactcctta tcaccttccg gatttagcaa gatcctcata cctaccacct gtaggattat 2040

ttactttttt cttccagcca attcagcctt aacgtttttt agtgcacata tttttaaaaa 2100

gaaaacttta aatcacgact ccacttggaa aaccagcgtt ttcctagtga acgtttaaat 2160

aaatgcatca ctactaaaac ttttttattt ttttaattta ttttatttta tcttattttt 2220

ttttcttttt gagacagggt cttgctctgt ttcccaggtt ggaatgcagt agtgcaatca 2280

cagctccctg cagcctggaa ctcctgggct caagtgattc tcccacctca gcctccaagt 2340

aggtgtggct acaggtgtgc accaccgcat ctgcctaatt tttatatttt tttatagaga 2400

tgggggtctc actatgggac ctgggctggt ctcaaactcg tggcctcaag tgatcctcct 2460

acctcaacct tccaaagtgc tgagattaca agcatgagcc accatgccgg gcctgaacct 2520

tttttttttt tttttttttt gagacggagt ctctctctgt cctccaggct ggagtgcagt 2580

ggcgcaatct cggctcactg caaactccgc ctcccaggtt cacaccattc ttttgcctca 2640

gcctccggag tagctgagac tacaggcgcc tgccaccacg cccggctatt tttttgtatt 2700

tttagtagag atggggtttc accgcgttat ccaggatggt ctcgatctcc tgacctcgtg 2760

atccgcccgc ctcggcctcc caaagtgctg ggattacagg tgtgagccac cgcgcccagc 2820

tttttttttt ttttttttta attttatttt atttttgaga cagagtctca ctctatcacc 2880

caggctggag agcagtggca caatctcggc tcactgaaac ctccacctcc aaggttcaag 2940

ctattctcct gtctcagctc cccgagtagc tgggattaca ggtgcgtgcc accagacaca 3000

gctaattttt tgtattttta gtaaagacag gatttcacca tgttggtcag gctggtctag 3060

aactcctgac cttaggtgat ccgcttgcct ccacttccca aagtgctggg attacaggcg 3120

tgagccaccg tgccccgccc tgacataggg gctttgggat cacagacttg gattcacttc 3180

cagcctccaa ggcctctccc agaaactctc tgtgaccact ctcctttttt tttttttttt 3240

ttgagataga gtctcgctct tgtagcccag gctggagtgc aatggcacaa tctcggctta 3300

ctgcaacctc cgcctttcgg gttcaagtgg ttctcctgcc tcagcctcct gagtagctag 3360

gattacaggc atgtgcaact tcgcccagct aattttgtat tttttagtag agacggggtt 3420

tctccatatt gctcaggctg gtctcgaact cctgacctca ggcgatccgc ccgcctcggc 3480

ccctcaaagt gctggaatta caggtgtgag ccactgcact cggcctcttc ctttttattt 3540

tttatttttg tgacagggtc ttgctctgtc acccaggcta gagtgcagtg gcatgatcac 3600

agttcactgc agcctctgac tcctggactc aagcaatcct cccacctcag cctcccaagt 3660

agctgggact acagtgcaag ccaccacacc tggctaattt ttaaattttt tgtagagatg 3720

ggggtctcac tatcttgccc aagcaccaaa gcctgtattt ttgaccccaa cattgaatga 3780

ggatgggatc tggtgataga gggaaaaaaa agagggggct gattgaaggg cacaggtaag 3840

ggaaggtttg gcacaaaggc tacagatggc tgcaggatga ggagggagtt gagacctgtc 3900

ctgctccttc caggctgctg ggcctttgca acaacttctc ttatgtggtg atgctgagtg 3960

ccgcccacga catccttagc cacaagagga catcgggaaa ccagagccat gtaagtgact 4020

ctctaccacc accaccatgg ttagtccctg tgggaagatg agggggtggg acaaggtggg 4080

gtaaagtttc cagtctctgg ctgggcacag tggctcacac ctgtaatccc agcagtttgg 4140

gaggctgagg cgggcggatc acttgaagtc aggagttcga gaccagcctg gccaacatgg 4200

tgaaactccg actctactaa aaatacaaaa actacctggg tgtggtggca cacacctgta 4260

gtctgagcta ttcaggaggc tgaggcagga gaattgcttg aagccaggag gtggaagttg 4320

caatgagcca agatcacacc actgcactct agcttgggca acagagtgag acaccatctc 4380

aaaaaaaaga aagctcttga atgtgttatc taaacaaagt ccatttacag ggccatctgt 4440

ggcctgtggt ctagtagtgt gagactcata tggaaacccc ctcttcattg tgcatgcaga 4500

gacgctgagg tcctgagaag tcagtcactg ctgacccaaa gccatccttc aagtgaaggc 4560

agagctggga cgggagccag gctctgtgtg tctatccctc tgcctccagg gtgaaagaca 4620

tgcttttctc ccattaggtg gacccaggcc caacgccgat cccccacaac agctcatcac 4680

gatttgactg caactctgtc tctacggctg tgcgtactca tctcacctgg tccttgcctg 4740

acccagggcc cctgggtcta tagaccccac tcccagccct tcactaccca gctgggactg 4800

tcatgattaa aacagttgag acctgggctg ggcgcactgg ctcacacttg taatcccagc 4860

actttgggag accaaggtgg gaggatcact tgagcctagg agtttgagac cagcctgggc 4920

agtatagtga gacccccatc ttaaagaaaa aaattaaaaa atatatatat ataaaataat 4980

tgagacctta atataatttc tgaggctgag gcggatggat cacttgagac caggagttca 5040

agaccagcct ggccaacatg gtgaaacccc atctgtacta aaaatacaaa aattagccag 5100

gcatggtggt gcacgcctgt aatcccagct actcaggagg ctgaggcagg agaatcactt 5160

gaacccagga ggtggaggtt gtagtgagcc aagatcgagc cactacactc cagcctgggt 5220

ggcagaatga gactcactct caaaatatat atatatatag tttctgagtc ctttctgtct 5280

gcaccatata ttagctcatt taagccacac agcagtcctg tgagctaggt gctatgatat 5340

tcccattttc cagatgagga aactgaagct cagagagtat aaactcttga cacacaacta 5400

aggagtggga gagctgagac ttgaacccag gcgtgcctga ctccagagcc tgtgtttgta 5460

gcaggcctgt ttggccagct cctgcctctc cttggccacg tggttgggag ggttgtcccc 5520

tggaagctct gcggtctcac tctattctcc tgtcccaggc tgtgctcctg gcggacatcc 5580

tccccacact cgtcatcaaa ttgttggctc ctcttggcct tcacctgctg ccctacaggt 5640

ctgggtgagg gtagtgggag gcagggtggg caggagctga gaaaggggag gctgggatgg 5700

ctgagatgct gagagtagag accgaccttc cccctccctt cccttctcac cccctcagcc 5760

cccgggttct cgtcagtggg atttgtgctg ctggaagctt cgtcctggtt gccttttctc 5820

attctgtggg gaccagcctg tgtggtgagt gtgtggttct gtgtcagatg gggagccccg 5880

aggaaccaca tcagagcatt tgtgggaaga gtctccccag cctcccagag gaaagggatt 5940

cattctgtca cccttagaag cctgctaggg ctatcagcag taggcgatgg gagactggga 6000

caatttggag gggtaggcag tggaggagat gggagaaaat ggatgaatta gatggagatt 6060

gaggtgaaca aagtcaagac tctgtgatgg accaggcaca gtgactcatg cctataatcc 6120

cagcactttg gaaagccaag gcaggcagat cacctgaggt caggagttcg aaaccagcct 6180

ggccaacatg gagaaacccc gtctctacca aaaatacaaa aattagctgg gtgtggtggc 6240

aggagcctgt aatcccagct actcgggagg ctgaggcagg agaatctctt gaacctggga 6300

ggcagaggtt gcagtgagcc gagatcacgc cactgtactc cagcctgggt gacagggcga 6360

gactccgtct ccaaaaagaa aagaaaagaa aaagactgat gaaggggcag agacatcaag 6420

ggtgcatgtc tgcccctggt ctgataactg ggtggatgga ggtgccactc tccatgaagg 6480

gacacgcagg ggagtggggc tctgcttcag acctggaacc tggcctatgc atgggatcta 6540

ttggagcctc tatgagctga tactgaggag gccatggcca gacacattag aggcctgggc 6600

agtgtggcaa ggtgtggtgt gaccatccca gtgcttgtcc tccccccagg tgtggtcttc 6660

gctagcatct catcaggcct tggggaggtc accttcctct ccctcactgc cttctacccc 6720

aggtaagcag gtggagcagg gagtgtgggg agaggctgtc ccatggtcag cctaggtcct 6780

cctgaatgtt cctgtgttct ccttcccagg gccgtgatct cctggtggtc ctcagggact 6840

gggggagctg ggctgctggg ggccctgtcc tacctgggcc tcacccaggc cggcctctcc 6900

cctcagcaga ccctgctgtc catgctgggt atccctgccc tgctgctggc caggtgagct 6960

gccctgagcc gggagggaga ggggtccaag gagagaaaac ttggccatgg ctgggtgtgg 7020

tggctcacgc ctgtaatccc agcactttgg gaggccaagg agggcagatc gcctaaggtc 7080

aggaaaccag cctggccaac atggtgaaac cccgtctcta ctaaaaatac aacaattagc 7140

caggtgtggt ggcgggtgcc tgtagtccca actactcagg aggctaaggc aggagaatcg 7200

cttgaacccc ggaggcagtg gtggcagtga gccgagatcg tgccattgca ctccagcccg 7260

ggcgacagag ttagactctg tctcaggaag aaaaaaaaaa aaagaaagaa aagaaaactt 7320

gattatgatt gcaatcttca agtccctacc ttgctgtgaa gggaggcgga atctggactc 7380

tgatagcccc aggtgtgagt cctggagctg ccacttttta gctttgtagc gttgaacaag 7440

ttactccacc tctctgaacc ctcagtttcc ccatatctca aatggcagtt gttcttgctt 7500

tccttggagg tgatgagggt aatgcattca gcacagtgtg gttcccaagg tgattagaag 7560

taggatgagg gtggacttta tttgattagt tccttttttt tttttttttt tttttaatca 7620

gtgttgacca ggttggcctc gaatgtgtag ccttgcctgc ctgagtgcca gggcaacagg 7680

cctgaaccat ggcgactccc tttttttttt tttttttttt ttttttttga gacggagtct 7740

catggtgtca ctcaggctgg agtgcagtga ctggtgtgat ctcagctcac tgcagcctcg 7800

gcctcccggg ctctagtgat tctcctgcct cagcctcccg agtagctggg actacaggcc 7860

catgccacca tgcctggcta attttttata tttttagtag agacggggtt tcactgtatt 7920

ggccaggctg gtctcaaact cctgacctca agtgatccac ctgccttggc ctcccataat 7980

gctaggaata caggcgtgag ccaccgcgcc cggcctgatt agttctgtgt attttcatgc 8040

atattacaaa acactttggc cgggcatggt ggctcacatc tgtaatccca gcactttggg 8100

aggccgaggc gggcgcacca cgaggtcagg agtttgagac cagcctggcc aatatggtga 8160

aaccccgtct ctactaaaaa tacaaaaatt agccaggctt ggtggcgctt gcctgtagtc 8220

ccagctactt gggaggctga ggagaagaat cgcttgaacc caggaggcag aggttgcagt 8280

gagccgtgat tgtgccactg cactccaggc tgggcaacag agcgagactc cgtctaaaaa 8340

aaaaaaaaac acacactttg aactagccag acacacctgg cccttcacaa gattagtgct 8400

taggacaagt ctaagtagaa aaagtgagtc catttaaaga aaaatattaa gtaaaaatat 8460

atatatacag gaggaatatg gatatggcaa ataacatgaa gccagaacct aagtgactaa 8520

aatcgaggaa gttctggcct agcacagagt gaacgtataa ttaatgggag ctagagcgac 8580

cattagaata ataatggcca gaaaacagaa ctgactcgct aggtatgagc cagcagtccg 8640

aacaagggcc tgctgagcac agtggctcac agctgtaatc ccagtgcttt aggaggctga 8700

ggctggagga ccactagagg ctagaaattt gagaccagcc tgggcaacat agcgagactc 8760

catttataca aaaaatggaa aatatgaggc gggcttggtg gcacgtgcct gtagtccccg 8820

ctacttggga ggctgaggcg ggaggattgc ttgagcctgg gaggtcgcgg ctgcagtgag 8880

ctatgattgc accactgcat tccagcctgg atgctggaga aagaccctgt ctctgaaaaa 8940

aatcaaaaca aaatgaaggc ccatgaatag gaataaggag ataaattcag gtccaaagaa 9000

agaagccttt gtccacaatt ggagctctcc cgcacagcgt aggagcctta gaggcagtga 9060

gctacccatc tttgaaagtg ttcaaaggta gaggtgttcc ctgggaaaag tggcctagat 9120

ggtccctggg gaccttccca gccagtaggg ttttctgacc ctgccttcat cctactccta 9180

gctatttctt gttgctcaca tctcctgagg cccaggaccc tggaggggaa gaagaagcag 9240

agagcgcagc ccggcagccc ctcataagaa ccgaggcccc ggagtcgaag ccaggtagga 9300

gacacagacc ctcagagagg tcactttctt tctctctggg tttggccttt tcctctctgc 9360

aataggcaaa gttaagaggg gaaagagagt gagtgtactg gttatgggaa aagcctttgt 9420

ctatttgaga aatacttgtt gaggacgagc acagtggctc acgcctgtaa tatcccagca 9480

ctttgggagg ctgaggcagg aggaccactt gagctcagaa gtctgagtcc agcctgggca 9540

acagagcaag accttgtcgc aaaaaaaaaa aaaaaaaaaa aaaaaagaaa agaaaggaag 9600

gaagggaggg agagaagaag ggatattaat tgagtactta ccatgtgcca ggcattccaa 9660

tcacaagtgc tgaggccctg cggtggggat aagctgggat gttctagaac cagagaatgg 9720

ccagtgagac tgggcagggt gggccaatgg ccatctgtgg agctgtacaa gagacattgg 9780

ccgggcgtgg tggttcatac ctgtaatccc agcactttgg gaggctgagg cagatggatc 9840

atttgagatc aagagtttga gaccagtctg gccaacatgg taaaaccccg tctctactaa 9900

aaataaaaaa attagccagg tgtggtggtg catgcctgta gtcccagcta cttgggaagc 9960

tgaggcacga gaatcacttg aacccaggag gcagaggctg cagtgagctg agattgcacc 10020

actgcactgc agcctgggca actgagtgag actctgtctc aaaaaataaa aaaaattaaa 10080

aatcaaagag acgtgaagag gtctcacctg gttagctttt attttcatca tgataaagta 10140

tgtatattac ttgaagttta ccattttatt attttttatc ttactttatt tttttgaggt 10200

ggagtttcgc tcttgttgcc caggctggag tgcaatggcg caatctcagc tcactacaac 10260

ctctgcctcc tgggttcaag tgattctcct gcctcagcca cccgagtagc tgggactaca 10320

gacacctgcc accacacatg gctaattttt gtatttttag tagagatggg gtttctccat 10380

gttggccagg gtggtcttga actcctgacc tcaggtgatc cgcccacctc agtctcccaa 10440

ggtgctggga ttacaagcat gagccactgc gcccagccat tttaaccatt tttaagtgtc 10500

caatccagtg gcatggaagt gagttcccac tgttgcgtgg tctggttaat tctgtcatac 10560

cggtgcctct ttgccgagtc ttcagtgtga aaacttcatc tgcccttctc tctctgttcc 10620

cctgcaggct ccagctccag cctctccctt cgggaaaggt ggacagtgtt caaggttcgg 10680

atgatggctg gggtatgtcc ctgtggggct tgctcacctc caggcccccc gcttatatct 10740

tctgcctttt ccagggtctg ctgtggtaca ttgttccctt ggtcgtagtt tactttgccg 10800

agtatttcat taaccaggga cttgtaagtg aggggtgcta ggaggggtgt ggaggtggcg 10860

attggggctg ggacccacac agccccgtcc atctcccctg tctggtattt gttgcagttt 10920

gaactcctct ttttctggaa cacttccctg agtcacgctc agcaataccg ctggtaagag 10980

gagcgagggc agtgggctgg gagggcgccg tggtgatgca gctgccctgc ccagtaggca 11040

ccggggggag cgggatggtc cctggaggag cctcctctcc tccccccaac cctaacctca 11100

ggtaccagat gctgtaccag gctggcgtct ttgcctcccg ctcttctctc cgctgctgtc 11160

gcatccgttt cacctgggcc ctggccctgc tgcaggtacc aaacccctgc ccctcacttc 11220

actcccacct tggctcccag cttggctccc agcttcccca aaccccctgc ttcccactat 11280

cagtgggaag tgtaaatttt tttttttttt tttgagacag agtctcgctc tgtcgtccag 11340

gctggagtgc agtagcgcaa tcttggctta ctgcaacctc tgcctcccgg gttcaagtga 11400

ctctcctgcc tcagtctcct gagtagctgg gattacaggc gcccgccacc atacctggct 11460

aatttttgta tttttagtag agatgggatt ttgccatatt ggctggtctt gaactcttga 11520

cctcaggtga tctgccggcc tcagcctccc aaagtgctgg gattacaggg aagtgtaaat 11580

tgtacactat catttcttag cacagggaac caaagtccag cagagctcca tgtaaaatgt 11640

atagcctcag gagccaagca aaactgagct tcagtactca gccactgtgt gacttagggc 11700

aggtctcaga acctctctga gcctcaattt cctcatgtat aaattgggtg tggccaatac 11760

ctatttttca gggtagttgt aagaaataga gatgagagaa agaatcgaag aacaagattt 11820

tgtagtgtgt gtgtgtgtgt gtgtgcgtgt gtgtgtgtgt gtgtgtgtgt gtgtgtgtgt 11880

gttttaagca ttgggattgg gccgggccag gtggctcatg cctgtaatcc cagcactatg 11940

ggaggctgag gcaggtggat catttgagct tggtagttca agtccagcct gggcaacatg 12000

gcacaatcac atctctacaa aaattagcca ggtgcagtgg cgcacacctg tagtcttagc 12060

tactgggaag ctgaggtggg aggatcactt gaacccatga ggtcaaggct gcagtaacca 12120

aggtcatgcc actgcactcc agcctaggca atagagcgag accctgtctc ccagaaaaag 12180

aaaaaagaaa ggaaaagtcg gaaccttcct ctgtcgccta ggctggagta cagtggcatg 12240

atcataggtc atttacctca ggcaaagaga caggctttca aataatgttt ctaactataa 12300

gaaaatgtac agttctttta ttctagatag ttaattataa tagaattggt gactttgaag 12360

tcagtgggtt atttatcttg agggtggtga gcaaattcaa ctgcccttgg caaacagtag 12420

tttacagctt taagggcgca aacacccttc cttacaacag tctaataaaa ggagtttcat 12480

ataaccagca ttcattgttt caaggttaca aatccacagc ccctgcaggt aactgttaca 12540

ataaactgtc aactgtgaca ggaaagtctt ctgcctttga aatttaggaa cttggccagt 12600

cctggtggct cacacctgta atcccagcac tttgggaggc caaggcaggt gaatcacctg 12660

aggtcaggat caggagtttg agaccagtct ggccaacaca gtgaaacccc gtctctatta 12720

aaaatgcaaa aattagttgg gcgtggtggc acctgcctgt catctcagct actcgctagg 12780

ctgaggcagg agaattgctt gaactccgga tatggaggtt gcagtgagcc gagattgcag 12840

tactgtactc cagcctgggc cacagagcga gactccatct ccaaaaaaaa agacttagga 12900

actcaattct gtgatagcaa aatacagaaa tagagattat actttaggct aggtgttgtg 12960

gctcacacct gtaatcccaa tgcttttgga ggctagggtg ggaggatcac tcgaagccag 13020

gagcttgaga ccagactgga caacatagtg agacccccca cctctacaaa aaattaaaaa 13080

aaaaaataag ccaggtggga gaattgcttg agcccagaag tttgagagag cagcctgggt 13140

aacatcacga gacctcatct ctacaaaaaa caacaacagg gtgtggtgac attcacctgt 13200

aatcccagct actcgggagg ctgaggcagg aggatcgctt tagcccagga gttccaggct 13260

gcagtgagcc ataatcatgc cactgtaccc cagcctgggt gagaccctat ctcaaaaaaa 13320

aagattacac tttcatatta tggcaaaagc atgtaaatat cctccagcct ccttccttat 13380

ttatctgagc agtgctgcaa gcccagggtt tgtacccaca aaaccccaaa tttataactc 13440

agggtgcttg tagataccac tgaaacatgt agcttatggt tttaagctac atgtttttag 13500

ctataatgtt ttttgttttg ttttgttttt tgagatggag tctcgctctg ttgcccaggc 13560

tggaatgtag cggtgcaatc ttggctcact gcaacccctg cctcccagat tcaagcgatt 13620

ctcaagcctc agccttccga gtagctggga ttacaggtgc gccaccacgc ccagcaaatt 13680

ttttgttttg ttttgttttg ttttgttttt tagagacaga gttttgctct tgtagctcag 13740

gctagagtac aatggcgtga tcttggctca ctgcaacctc cgcctcccag gttcaggcga 13800

ttcttctgcc tcagcctcct gcgtagctgg gattacaggc acgtgccacc acacctggct 13860

aatttttgta ttttttagta gagatggggt ttcgccatgt tggccaggct ggtcttgaac 13920

tcctgacctc aggtgatcca cccgccttgg ccttccaaag tgctgggatt acaggtgtga 13980

gccaccacac ctggctagag gtgtgtcctt tttaatgctc atctttaagt ctacctcaga 14040

tttaatgaat taggacctct agggtaagcc ccgcttgggg catttgtacc tagatcccca 14100

ggcaattctt acatatacga aaagtatgaa ccgccacgag gaggcctaca cactgcacaa 14160

gaggcaatgt ggcgtggtca gtgctgggag gatggcacct ccttgagaga ctggggcaca 14220

gaggtgggac ctacaccctg gggtggggat cgaagaggct tcctggccag gtgcggtggc 14280

tcctgcctat aatcccagca ctttgggagg ctgaggcagg ggggcagcga ctgcttgaga 14340

ccaggagttc gagcccagct gaataggatg cccggctact cccaccttgt tttaccatag 14400

tgaaccaacg gtttttccaa taggggcaat tttgctgccc agcggatgtt tggcaacgtc 14460

tgcaggcatg ctttggttgt cacaactgag ggatgctatg ggcatctagc gggcagaggc 14520

cagggatcct gctaaatgcg ctgccatata caggacagcc ctcaccatgc agaacaacca 14580

gcaccaaacg ccaagagtgc tgcaattgag aaatcctggt ttcgatggtc ccacttcctt 14640

taagaagcct ccattttttt tttttttttt tttttttttt ttatgagaca ggatcttgct 14700

ctgtcaccca ggctggagtg cagtggcaca atcatagctt actgcagcct ccacctccta 14760

ggttcaagca atcctcctgt ctcagactcc cgagtagctg ggactgcagg tgcgtgccac 14820

catgctcaac taatttttaa attttttgta gagacggagt tttgttatgt tgcccaggct 14880

ggtctcgaac tcctggcctc aagtgatcat cccaccttgg tctcccaatg tgctggtatt 14940

acctgcatga gccactgcac caggctgagg ttgagaattt tttttttttt tttttgggat 15000

ggagtctctc tctgttgccc aggctggagt gcagtgctgc gatcttggct cactgcagcc 15060

tctgcctccc aggttcaagt gattctcctg cctcagcctt ccaagtagct gggactacag 15120

aaccactaca cccagataat tttgtatttt ttagttgaga tggggtttta ccatgtttag 15180

tagagaccag gccaggctgg tctcgaaatc ctgacctcag gtgatctgcc cacctcagcc 15240

tcccaaagtg ctgggattac aggtgtgagc caccacaccg ggctggtgtt gagattttat 15300

ctagacctgg cagccctctc cttcaccagt aactcctaaa accagggacc cctggagggg 15360

aggccacctc tccttccctg ccccgccctg gtcccaggct tagcctctcc ccctgttcac 15420

agtgcctcaa cctggtgttc ctgctggcag acgtgtggtt cggctttctg ccaagcatct 15480

acctcgtctt cctgatcatt ctgtatgagg ggctcctggg aggcgcagcc tacgtgaaca 15540

ccttccacaa catcgccctg gaggtcagca ttggccgggc aagggctggg ggtggcctgt 15600

ccagggacac ccagggcagg gatgtctggg actgaagcct cacccctgct ctctgccctc 15660

ccagaccagt gatgagcacc gggagtttgc aatggcggcc acctgcatct ctgacacact 15720

ggggatctcc ctgtcggggc tcctggcttt gcctctgcat gacttcctct gccagctctc 15780

ctgatactcg ggatcctcag gacgcaggtc acattcacct gtgggcagag ggacaggtca 15840

gacacccagg cccaccccag agaccctcca tgaactgtgc tcccagcctt cccggcaggt 15900

ctgggagtag ggaagggctg aagccttgtt tcccttgcag gggggccagc cattgtctcc 15960

cacttgggga gtttcttcct ggcatcatgc cttctgaata aatgccgatt ttatccatgg 16020

acttcttata tcgtttttgt ctctaaaaag aaacttttat tatgaaagta atacatgccc 16080

actttcctat atatagacag cataaagaag gtatgtcagc agatttctcc cttttttgtt 16140

tgtttgtttg tttgtttgtt tgttttgaca gagtctcact ctgtcaccta ggctggagtg 16200

caatggcgtg atcttggctc actgcaacct ccacttcccg gttcaagcaa ttctcctgcc 16260

tcagcctccc gagtagctgg gattacaggt ggtcactacc atgtctggct aatttttata 16320

tttttagtac agacgacatt ttgccatgtt ggccaggctg gtctcaaact catgacctca 16380

ggtgatccgc ccaccttggc ctcccaaagt gctgagatta caggtgtgag ccactgtacc 16440

tggcctctgc catctttgag gccttacgta ttcattcatt catgcattca ttgtttggga 16500

caatctgtaa acaatcatgt tggacattct ttccagcatg aaagttttca gagaagtgga 16560

aagaattgtg cagtgaatca tcctgagtat gcgccaccct gttctatgtt aacattttgt 16620

tacagttgtt ttatcatgta gtccagcccc catcaaatct atgtattcac tgttccatca 16680

gtgagtagat agagctaatg aatggcaggg ttggcacctg aggcatttta ttttattatt 16740

tttttttatt tttagagaca gggttgcact ctgtcaccca ggctgaagta cagtggcaca 16800

gtcatggctc actgtagcct tgacctccta gactcaaaca atcctcacac cttggcctcc 16860

caagcagctg ggactgcaag tgcatgccac caagcccagc taattttttt tttttttttt 16920

gtagagacgc gatcttccta tgttacccag gctggtcttg aactcttgag ctcaagcagt 16980

cctgccttgg cctcccaaag tactgagatt acaagcatga g 17021

<210> 2
<211> 14881
<212> DNA
<213> Mus musculus

<400> 2
tggacgtctg ctaagggacc ccgacaaggc ctagcttgaa taagtgggta cacttgtaga 60

ggcaggagat tgaaacccag gaaggactgg gctaggagtc tggctgactg taggaaaggg 120

agctcaggct tcaggagagg ctgtaaggga gaggaaaggt gagactacga ggaatggtgg 180

aagtttcggg tatctctctg cccctctcac cagtgctccc ctcaaagcct gatgtagccc 240

agggagacga agccggagaa aatccatcct gcaggtctct gtgtgtccag aaagctcaca 300

atgaccatcc gtccccaccc acgaagtcct ccccagcacc caagacactt cctcttcccc 360

tgactctccc tgacaaatct ccaccccagg ccctgtgcac ctcccccctg ctcatggatg 420

acttcatgac aggcaggggg atggcttaac agtgtccagg tgactcagac tcgggtgcac 480

atctggaact aaagggcaga ggatcgggcc agaaagggga aagggaaaaa aaaagtggaa 540

acaggagacc tcagaattgg gtatggggga gggggcattc ggcaagtgca gcctgagtga 600

ctcaggtctt gtattcgcac ctcagccatt tccgcctcct ccaagccaca gccttcagtg 660

agaggaagct agggtttgtg gttcctgatt tatttggggg agaagctgta gaagtgagga 720

taaggccact ggaagttctg ggttcccatt ttcaacgcca ttaagatttt tctggggatg 780

aaaggtctgg attggtgctc aatgcccaca ccctcaccta tggttattct tgtactgtgg 840

tcatgtgtcc tcacagctta ctgtgagttc cttaaggaca gaggctgcag aatttctaga 900

gactcgtttg ggtaggagga tcctttttct cactgtttga gtgatctagg aagaacacca 960

caggagggca tatttcaaac ggcaaatctt aatgacaggt ggttgaaaag acccgggaga 1020

agtgggcaga ggctgctgga atccatagca gaggaatagg cttgatgaag aatggtcaag 1080

atgctgccct gccatcgagt ggaaggtaag ggaaggactc cttatttgga actgtgaggt 1140

gacacattcc aactatcctc tcagcctcat tctcctaact tccaattagg acaaacggct 1200

gacatcacag accaggaaaa acacccatag cagctttctc agtgaactta gcacctttgc 1260

tctatttctt actgctgagt ctctgaagtc agctaaaagt ctagcctctg ttgcacagtg 1320

gggcggaggg accttggttt tctcttgtaa aaccaggggt tggacaactt tcgtggtatt 1380

tgttgttcta ggatagtaag gcaaagggga tttaaccgta acatcgtgaa tattgccaca 1440

gataattcat agaatgctgt tttgtgggtg tttattttta atcgatctac tctcagctac 1500

ttcccagatg cttattatag gtctttacaa taggtctgta aggacaacag ataacacttg 1560

aggctggtcg cctttgcgct caagcagaac ttgtccttgg gacgttgggt ggggtagtgg 1620

gacatccgca gcaaagtagc cataattgtt tcaggcaagt aagagctgca ggaaagaagc 1680

tgaagccttc cgcgccatga ctagtactgg cagatttggt cggcgtctag agcgccgctt 1740

ttcatggctc tacttgtggc tcccttcgac tccaattcaa aatcagtgct tccagcgttc 1800

tctcgaactc tccttctttt attctccgaa gtttgagaag tccaaaggcg aaaaagagaa 1860

aaaaggaccc ggaagtaaga agcaaccctc aagcacgtga tggaaggctc acgggtactg 1920

tcacgtgacc cccaacctgc tcgtctacct tgcggagagt tcagctgctc ttaaaggtac 1980

aggcctgggg gcgggggctt tgctttagag gggcaggggg cggggtcagg cgggtggggc 2040

gtgggtgaac acgagctgga gatctgggga tccttcgacg gacatgtcgt gtatagcaga 2100

cagcggaacc tgggactgac cgcggggcat tgatccttcg cacccacctg tcccagactt 2160

taatctgttt tcttgaagct agctcggaac acacgctgac tttgggccct ttgggggacc 2220

cgaactcaat gttatgggaa gttctgcggg ctcgtggagg cgccttgagg attctgagag 2280

tgagtgctct catcggttgc cttgggaatg accacctctg cccgcggctc tcccgaggag 2340

ttgggatgct ggccctgccc cctcaacacc atgcgggata ggcggggcct tcgtgtaccc 2400

gagtgagtgg ggggtcatgc tttctctacc tcccagggga ggagaccgac tcagagcccc 2460

aggcccctcg gttggatagt cggagtgtcc tttggaagaa tgcagtgggt ttctggtaag 2520

tggtctgaga gttgtgcaaa agtaaaatgc cagctgggcg tggtggcaca cgcctttaat 2580

cccagcattt gggaggcaga ggcaggcgga tttctgagtt cgaggccagc ctggtctaca 2640

gagtgagttc caggacagcc agggctacac agagaaaccc tgtctcgaaa aacaaacaaa 2700

caaacaaaca aacaaagtaa agtgcgattg gagcaaagat gagagccaag ttgtgaaaag 2760

cagttgaata cacagatact taatcaactg tgaactttcc aagttccatt aaattcccac 2820

cttcacctat tgatcagatc ttagtatctg tagagttatt tattaaatgt tttcctccag 2880

ctaactttgt tttttttcct cttccttcct ttctttcttt ctttctttct tcctcttcct 2940

cctcctcctc ttcctcttct ttttcttctt ttttttggaa aggtctttaa accctacaag 3000

cagtaagggc ttactttgac ctttctaatc ctgcttttgc cttccaggtg ctgcagttac 3060

aggccctact gtagcacaat gcccagaaca cccattgttt ctgtttgcag tgcagggtat 3120

taaaagcagg tccttcccgg gctcacgcac tgttgtacaa gtgagctctg tctccagcca 3180

atttgctttt gccaagttct gagcacagct ctgggagatg aaaataagaa caatatgccc 3240

tgctattatt ttggttccct tgagatcagc aggtgaaaat ttgtctccgt tttgcacagg 3300

aatacgctag atatttgcat tgtaccacag tgtttccgtt gatgagaaat gcccagggat 3360

cagaatctta gtgtaagcgt gggagaaata gtgcttagga ggacttggca cttggccatg 3420

agctggtcta atctcaaggc ctgttttgat agtggagaag gaaaaaaaaa aggtgtatct 3480

ttaaggactg taagtggata gaaagccaga cagtgaagat gtgtcctcat ttctaggatc 3540

ttgggtcttt gcaacaattt ctcatatgtg gtgatgctga gcgctgccca tgacatcctc 3600

aagcaggagc aggcgtctgg aaaccagagc catgtaagta agcccctctt caccaccacc 3660

ataggtcagt aagtgagccc ctctgtacca ctaccatagg tcagtccgca tggggaatac 3720

taagaaccca ctcagggcca gaggacccct gaaaaactcc tagagtagct ctgggtggga 3780

ttggggagct aaaggtctta gtctcccaca tcctcatggc aggagggatc ttaaagagag 3840

caacgggtac caggttggtg aactcacata actggctggg taagacctta ttttgtggtg 3900

tgttttgtca ttgtaccctg ttgacatttg aggtcaggtg actgttgtga gtctgtcctg 3960

catgttgtag atgtttagta gcatcccatg ccccttgtca accaaaagtg acagccaaaa 4020

atgtctccag atgttacaaa tgctcctgcc aggtaacatt acccaggtgg agagcctttg 4080

gcggcagtga gctagagcac gcaggcccta agaggcgcag cctccacttt gcaggttcac 4140

taggaaattt gacccaagct gatattttcc agaaacccag atttctatat gaatgctctt 4200

aaacatggtg catccatctt ggggggctgt ctttggcctc tggcccaaag tttgagatgc 4260

ttataaacac tctcttcatt gtggacattg aaaaactaag gccccctaga agccttgcct 4320

acccacagcc atttgtgaag ttaggacaga actggacctg ggccttggtt agcctcctgc 4380

cgctttgtcg gaaagaccct cttctctccc ataggtggaa ccaggcccaa cacccacacc 4440

ccacaacagc tcatctcgat ttgactgcaa ctccatctcc acagctgtga gtgtccatgt 4500

ggggacccca cgatccttgc ctttgcaatc tcgtttcccc acccccaccc ccatccagtt 4560

gggaatgttg ggattgcagt gatgaatata aatatgtaaa tagtaattat tagttcaggt 4620

gccccccttc ccccagcttt gtgagtgaaa cactgtaagt ttccctcttt ggatgaggaa 4680

actgaaggtc agagtgtata aatatgccct gacagccatt tgaggagccc cagagcaggg 4740

aatgaatgga gatctatcta actccagagc ctaacttaca gcaggtctca ctggccggct 4800

ccccggctgg gttgggaagg ttgtctccgg gaaggtctag gctctcactg tgttctcctg 4860

tctcaggcgg tgctcctagc agacatcctt cccacccttg tcatcaaact cctggcgcct 4920

cttggccttc acttgctgcc ttacaggtct gggtcggggt ggcaggaggg aggcagggtg 4980

ggaggaagcg ggagtctgga gagtctcgca tggaggtggc cttctgagtt cctcctcatc 5040

tcttctcagc ccccgggtgc tcgtcagtgg agtttgttct gctgggagct ttgttctggt 5100

tgccttctct cagtcagtgg ggttaagcct gtgtggtgag tacgaagctg ggagaggttg 5160

atggactcaa cagagtgatg tgctgtagtc tccccagctt cctgtgtgtg tgtgtgtgtg 5220

tgtgtgtgtg tgtgtgtgtg tgtgtgtgtg agagagagag agagagagag agagagagac 5280

acacacttgg gagattgtga atttgagatt agttggggcc acatagtgaa ttcaagccta 5340

tcctaagctg cacagcaaaa ccctatctca aaaaacaaag acaaaaaaaa accaaaaaaa 5400

caaggagagg gccagtccag gctgatttac atagcaagtt ccaggccagc ctggactaca 5460

tccccgagac tttgtctcaa agacaaacct cctagagctt ccctaaccag aagtgagctc 5520

taggaacaat tggagggtgt caataggaag gaatggggag actgagtata ggtggggatg 5580

agcagggttg aataattggg aattattaac tgaatgaaga tgggggtaca gtctcggaga 5640

ggacatgtgg aggagggtgt ggcttttcag atgagaccct agcctgtctg ttcagtgaca 5700

gatctgttct tagccctgct gagctgatgg agttgaggct gtaaacaaac caccggggac 5760

tgcggctttc agttaaaggt cactgtttgc tgtttgtcct cccaggagtg gttttggcca 5820

gcatctcctc agggctaggg gaggtcacct tcctctcact gactgccttc taccccaggt 5880

aagcggcctg ggccaggagg actgggagac gagtgccccc tgctggcttc catcttcctc 5940

agtgctcttg tcttcttttc agtgctgtga tctcatggtg gtcttcgggt accgggggtg 6000

cagggcttct tggatcgctg tcttacctgg gactcaccca ggctggcctc tccccgcagc 6060

acaccctact ttctatgttg gggatccctg ttctgctgct agccaggtga gtgtccttag 6120

gcccaaagga tgaaagatgg aaggaggagc ttgatgctaa tccaatcttc agagctcttc 6180

agccttgttg tgaaggggtg tggactctgg actctcgaca cccctcaggt ggaatctcag 6240

agtggggctg tgttagctgt gcgactctgg acaacttcat tcctgacctt cagttgtctc 6300

tgtctcaggc tggttctgtc tcatccgcac aggtcggctc ctgagactgg ggctgggact 6360

gagtgcaccc cggtttattt cttctgtgtg tatttgtata ttttagaaaa cattattaag 6420

ccaggtgtag tggtacatgc ctttcatccc agcactctgg aggcatcatg ttatagctta 6480

gggctattgt ggggtatcca ccagaaagac ggctcagaca tgggtttaaa cccagtggaa 6540

agccgattag tcacctggta actacacact gcatgttcag tggcatttcc attggagtca 6600

gttgtcctaa ggtccggtgg cctgttacaa ggctatcttg aagaaaagga gtcagtatgt 6660

gtgtaatgct gaacacccac agtcctggct cttgggaggt gatggaaaag gctcatgtgt 6720
ttgaagccta cctgggatac acgtggagac cctggctcaa aagctaactg agataattga 6780
acatatggca aatgcctcta atccaagccc tcaggaggca ggaggatctc tgtgagtttg 6840
gggtcagcct ggtctacatc ttgagctcca ggccagccag agctgcatag ttaagaccct 6900
gtctcaaaaa aaaaaaaaaa gaaagaaaga aataaagcaa tcccatgatc atacagaccc 6960
tgggtaaact cagtcacaac acgaaacaaa aagacactga gtatgagaaa gggacctgtg 7020
atgtgggaaa ggtcacaggg tgtggggacc cgagggggtg gggagagtca ccagagggca 7080
gtatatatgt gaataaaaat gtcaagaaac acatttagta agaatctctg tgaagtggtg 7140
aatagatgag aagtactgag aacagggaaa cgagcaggtg ctgtgactga gtcaaatgag 7200
gtccctgggt agaaacaaag aagcttttcc agcacagggt acctagaggc tgggagcacc 7260
ccatgcaagt gtgaagggcc tggaggggcg ggcctgcagg acctctgggg cctttcccga 7320
caagagcccc ctgactcgct tctccattcc atcccagcta tttcttgttg ctcacgtctc 7380
ctgaacccct ggaccctgga ggggaaaacg aggcagagac tgctgcccgg cagcctctca 7440
taggcaccga gaccccagag tcaaagccag gtaggatgca aaggccctcc catcccaggg 7500
ccactttccg ccttggggtt ttggtctcaa cagtggacac agttgaggga aacaggctgc 7560
taggtgtaca ggtctgggtc aaagcagtca tgtatactta ctaactactg actggatgcc 7620
agacattccg attgctggga atggtagtgc tccagatgaa ggaatgagag cattttcaca 7680
ggacataaaa ctgcgatggg gcccagaggg gtggcgcaca ccttgaatcc caacactccg 7740
gaggcagcca caatcagatc tctatgattt caaggccggt tgggctacat agtaagaccc 7800
tgcacagacc cagaaagtaa cggttaagga aatggggtgg ggatggctaa gggagctgct 7860
ttacaggagg ttccaagtgt ggggggtttg ttgttgtttt gtttttcttt caagtttttg 7920
cttattggtt tttaaatagt ctctctgtat agccctagct ggcctagaaa tcactgtgta 7980
ctctcgaatg gcctcaacct cacaaatcca cctgcctctg cctctcaaat gctaggagta 8040
aacgcatatg ccaccataca cctggctagg atttattttt ctatttattg atgtgtatgt 8100
gtatttgtga acttataccg cggatgacca gcagagggca tcagatgcct tggagttgca 8160
gttacaggca gttgggagct ggccgatgtt gccaggattc aaacgtggac cccctcatgg 8220
ttgagcaacc agtgctcgct cgctctttct ttccttttct ctctttcttt ctttctttct 8280
ttctttcttt ctttctttct ttctttcttt ctttctttct ttctttcttt ctttcttcct 8340
tccttccttc cttcctttct ttctttcttt ctttcggatt tatttattta tttaatgtat 8400
atgagcacaa tgtagttgtc ttcagacaca ccagaagagg gcaagagggc atcagatctc 8460
ataacagatg gttgtgagcc accatgtggt tgctgggaat tgaactcagg acctctggaa 8520
gagcagtcag tgctcttaac cactgagcca tctctccagc ccctctttct ttttttttta 8580

ttgtttgttt gttttgtttt tgttttgttt tttgtttttt tgttgttgtt gttgaggcag 8640

agcttctctg tgtagctctg gctgtcctgg aactcactct ctagaccaga agtcaaaaat 8700

tctcctgcct ctgctttcca tgcgctggga ttcagggcgt gtgccgccac ttaaccccaa 8760

gccacctctc caactctgag aaaggctcta cttatttatt taattttgta attatttgtt 8820

tgtttgtttg cttgcttttc gaggaagggt ttctctgtgt agccctggct gtcctggaac 8880

tcacactgta tgtaatccag gctgccctca aactttcaga gatccacctg cctcagcctc 8940

ccaagtgggg attaaaggca tgcgccacca ctgcccagcg atttgtctat ttttatttta 9000

tgtgcattgg tgttttgcct gtgtgcatgt ctatgggagg gtgttggatc ccctggaact 9060

ggagttacag acagttgtga gctgccacgt gggtgctggg aattgaggtc cgctggaaga 9120

gcagccatta ctcttaacca ctgagccatc tcttgagccc ccgagagaac ccccaacaac 9180

aaacctttta tttttgagaa tggagctgtc tgggcaagag cgtgtgcaca agtgtgaggt 9240

ctgacgttct gaggattgag ctctgctcat tcagactcgg cagtgagtgc cctcatccac 9300

cgagcatcca tcagcccaag acagcctttt gaaggatatc tctgggctga aacttggctg 9360

tcaaggtgtc agctttgcaa atgcctaggt gatcatgttc caggcacagg aaacagctca 9420

gggtgaggcc ttatgtgaga aaaggagcct ggttgttcta gaaatgaaag gcttagccag 9480

agtctgggat ggagtcggta gataaaagct gtaccctgat ctatagtctt gctcattaaa 9540

aaaaaaaaaa aaaagtttta taagtatatg tgtgtatata catatgtata cagcgcgaag 9600

tttaccattt tagcgactac taagtgtgca ctaagtggca tttaaatcca cacactgctg 9660

tgctggctgg ttgcttgctg catgctggca ccttagctaa gcgttcagcg tgcaacctcc 9720

tccccttccc cctgctctgt aggtgccagc tgggacctct ccctccagga aaggtggaca 9780

gtgttcaagg tttgaatggt ggctgcgggt gccccgtgag gatcctctgc ctcttgcctt 9840

ccacttacat ctcccgactc ttccagggtc tcttgtggta catcatccct ctggtgctgg 9900

tctactttgc agaatacttt atcaaccagg gacttgtgag tgaggggcgc cggggaggga 9960

tgtgggagga gagatagggc tgaggttccg ccatctccat ctgtctggtc ttcgctgcag 10020

ttcgagctcc tgtttttccg gaacacttcc ctaagccatg ctcagcagta ccgatggtga 10080

gagaagttgg caggtgggca gtaggctggg aggatccctg atgttggggt cctggggttg 10140

accccaaaaa gtgccctgag agcctcttcc tcttctctgc cccaacctta ggtaccagat 10200

gctataccag gctggtgtgt tcgcctcccg ctcttctctc caatgttgcc gaatacggtt 10260

cacctgggtc ctagccctgc tccaggtact gagtgctgcc ctgtttgttc acacccacct 10320

cggcctcctg agtttcttac attagtgtac tccagcgttc ctaagtacag cactaaaggc 10380

cagctggact ccatataaaa tgtgtggtgg caccaggagg aaccagtcat acttgtgggt 10440

aaattccagt ttctgttctc aagagctgtg tgactcagag cagtctctga acctctattg 10500

agcctcagtt ttcctatcag gaagtcagat ggggtggagc aggattgtgc acctagcctg 10560

tctgaggctg tgggtcccat ccctagtact gcaaaaatta aaatatttaa tagctgtggg 10620

caacacctat ttctcagtgt ttgtatagca atgttgagaa aaaaaaggaa gaacaagata 10680

ttgttttgat ttattttggt tttgttcttg tctgctttat ttttttttct ttgtttgttt 10740

ttaaggtagg gtctcactgt gtagtcctgg gtggcctgga atccgatatg tagactaggc 10800

tagcctgaag ctcacagatc tgcttcatga atgcgggcgt tgagggcctg tgcctcgtgc 10860

ctggccgtcg tgctttgttc tcacagtcgt tctgactgca cctggggcct tgttcctgct 10920

ggtccatgct gattgttacg aaacttctta agccctcaag cttgctttca tttacttact 10980

tatttacttt aaagatttgt ttgtttatat ataaacaaac tgtagctgtg ttcagacaca 11040

ccagaagagg gcatcagcta gccgggcgtg gtggcgcacg cctttaatcc cagcactcgg 11100

gaggcagagg caggcggatt tctgagttcg aggccagcct ggtctgaaaa gtgagctcca 11160

ggacagccag ggctacacag agaaaccctg tctcgaaccc ccccccaaaa aaaaaaaaca 11220

aaaaaaaaca aaaaaaaaaa acagaagagg gcatcagatc ccatcataga tggttgtgag 11280

ccaccatgtg gttgctggga atcgaactca ggacctctgg cagagcagtc agtgctctta 11340

accgctgagc catcgctcta gccctattta tttattttat acaatttaaa taaacacact 11400

tatatttata tttatttatt ggtgggaggg agacacttgc caagccgtgt gtatggaggt 11460

cagaggacag cgtgcaggat tggttccctt cttcaacgtg tgggttctgg tgatcaaact 11520

caggtcatac aggcaagtac ctgtagctga gccatctctt cagcccaaac agggtttctc 11580

tgtatagccc tggctgtcct ggaactcact ctgtggacca ggctggcctc gaactcagaa 11640

atctgcctgc ctctgcctcc caagtgctgg gtttaaaggc gtgcgccacc actgcccagc 11700

tcagcccaaa ctttttattt ggaggcaaag tcttcaaagc tggccttgaa cctacatctg 11760

aggcccacat aagccttgac cattgtttag tttatatata cacaaacctt tgtctatgtg 11820

tatatatgtg aaccacctgt gtgcctggtg cccatggagg ccagagaggg tatcagatcc 11880

ctggaactgg aactacagat aggtgtggac cgccctgtgg gtgctgtgga agaacagcgg 11940

gtgcctttaa ctactgagcc atctttccag tccaaaattt tgtactcttg acatttgcag 12000

ctagatcatt ctgatgtgtt acttgctgta ggtgtttagc agtatcccta gctggtgaga 12060

cggcaagtag gtctccagac attgtcaaat gacccattga tttcagtgca gtaattaggg 12120

gagaacaggt gatgttcagg gggtccttgg gtgctgagga taggactctg gtagagcatt 12180

tgcctctgaa tcacaagctc tcgtgttagc tctgcagtgt atgtgtgtgt gtgtgtgtgt 12240

gtgtgtgtgt gtgtgtgtgt gtgtgtttgt gtgtgtgata cacatggtag gggtgtctag 12300

gtgttaggat tcatgccatt aaaaaagaaa caaaaaggca agaaggagag gaggtgactg 12360

atgaccacag tggaatgacc ctggtccaac tgagagctca ccagtgatgg gaagttgagg 12420

aacattacag gctaagagtg acagggacaa gtcaaggaag gggagaaacc cacaaggcat 12480

gtgaggatga ggatggggtc atcgagggcc taccaagatg tcggggtccc acaggcccag 12540

ctgggcactg ggaccctaca cagccctctg cttcagcggc atctcctata acctggcagc 12600

tctgagacct gctgcctccc tgccgtattg ttgcccagtc tcacgcctcc cttccctctg 12660

cccacagtgc ctcaacctgg ccctcctgct ggcagatgtc tgcttgaact tcttgcccag 12720

catctacctc atcttcatca tcattctgta cgaaggctcc ctgggtgggg ccgcttacgt 12780

gaataccttc cacaacattg ctctggaggt ctgtgccagt tggacaaggg ctgtgggtgg 12840

ccttaggagc cttgtggatg gaagccctaa cccttgttct ttgctctccc agaccagtga 12900

caagcaccga gagtttgcca tggaagctgc ctgtatctct gacaccttgg gaatctccct 12960

gtcgggggtc ctggccctgc ctctgcatga cttcctctgt cacctccctt gacaggagtt 13020

gctcgacaca cactgatctg caggcacatg agcagatcac acatcttcga gctctgccac 13080

agcctttccc tgccccactg cagcaaggag cccctgatgt ttcccactcc tgagctggcc 13140

tcagagtttt ctcctaccct ctgcccttct aataaatgct tattttaaca gtttcctttt 13200

tgtgcagtct ctgaaacgga aagtttacat aagcccactg tgaaagatat atgtatttat 13260

gaatgtataa agtatttcag acaaggtgtg gcacacacct gtaattccag cactcagcag 13320

actggagaat ccaagttcac gtctgactgg gatatacggt gagaccctga ggactagggc 13380

tatagttcag tggtagagta cttgcccagg atgtcctggg ctctcagttt tatccctgga 13440

aaacaattca aaagcagcgt atgctaatat aagcctgtag tcccaggatt ggggaggtag 13500

agacaggagg atccagagtt catggtcctc agctacatag gaaaccaagg tcagcctggc 13560

ctacataaga tcttgtttta caataattac attgtgtatt tctggcttct ttataaaaaa 13620

aaatcaaatg tccataggta tgtagattta tatctgggtc ttcgattcca ttgatcaact 13680

tgtctgtttt tatgccaata ctgtgtgggt tttattacta taactctgta gtagtgcttg 13740

aaataaggat ggtgatacct ccaggagttc tattactgta cagagttgtt ttagcaatcc 13800

tgtgtttctt gtttttttca tatgaaattg agtattgttc tttcaaggtc tataaagaat 13860

tgtgttggaa ttttgatgga caactgattt ctttcttcaa tgtcttaaag tttttatcat 13920

ataactcttt gacttgcttg gttagattga ccctacaata ttcatagaag acgaagtggg 13980

tagtagccct gaacgaatgt cacaggagac aacttcctga acagaacacc aatagttcag 14040

gcactaaaat tgacaataaa ccagatctca tgaaactgaa aagcttttgt gaggcaaatg 14100

atattgtcat ctggcagcct acagaataga aaaagatttc taccaactca atatctgata 14160

gaaggctaat atccgtaata tataaagaac tcaaagccgg gcggtggtgg cgcacgcctt 14220

taatcctagc actcaggagg cagaggcagg tggatttctg agttcaaggc cagcctggtc 14280

tacaaagtga gttccaggac agccagagct acacagagaa accctgtctc gaaaaaaaaa 14340

caaagaacaa aaaacacgag ggagagagaa agagaaagag aactcaagaa gctagacatc 14400

aataaggcaa atagcccaaa taaaaaatgg ggtacagagc tgaacagaga cttctcaaca 14460

ggaatctcaa atggctgaga aacacttaga acttcatcat ccttatccag cagggaaatg 14520

caaatcaaaa ccactctgag attccatctt taacacctgt caggatggct aagatcaaaa 14580

ctcaagtaac agctcatgct ggcgaggatg tggagaaagg ggagcacttg ggagcaaggg 14640

gagcactctt cggttgttgg tgagaatgca aatttgtaca gccactttgg agatcaatat 14700

ggtagtttct tagaaaattg gaacctccag acccagctat accacttctg gggatatacc 14760

caaaggatgt cccatcctac cacagggaca cttgctcaat tatgttcaca gcagctttat 14820

ttataataac cagaaactgg aaacaacctc gatgtccctt aactaaggaa tggataagaa 14880

a 14881

<210> 3
<211> 25
<212> DNA
<213> Artificial sequence

<220>
<223> Synthetic oligonucleotide

<400> 3
acacagaacc acacactcac cacac 25

<210> 4
<211> 18
<212> DNA
<213> Artificial sequence

<220>
<223> Synthetic oligonucleotide

<400> 4
gagaagagat gaggagga 18

<210> 5
<211> 18
<212> DNA
<213> Artificial sequence

<220>
<223> Synthetic oligonucleotide

<400> 5
ggctgagaag agatgagg 18

<210> 6
<211> 18

<212>  DNA
<213>  Artificial sequence

<220>
<223>  Synthetic oligonucleotide

<400>  6
gggctgagaa gagatgag                                                    18


<210>  7
<211>  18
<212>  DNA
<213>  Artificial sequence

<220>
<223>  Synthetic oligonucleotide

<400>  7
cactgacgag cacccggg                                                    18


<210>  8
<211>  18
<212>  DNA
<213>  Artificial sequence

<220>
<223>  Synthetic oligonucleotide

<400>  8
ccactgacga gcacccgg                                                    18


<210>  9
<211>  18
<212>  DNA
<213>  Artificial sequence

<220>
<223>  Synthetic oligonucleotide

<400>  9
tccactgacg agcacccg                                                    18


<210>  10
<211>  18
<212>  DNA
<213>  Artificial sequence

<220>
<223>  Synthetic oligonucleotide

<400>  10
aaactccact gacgagca                                                    18


<210>  11
<211>  18
<212>  DNA
<213>  Artificial sequence

<220>
<223>  Synthetic oligonucleotide

<400>  11
gaacaaactc cactgacg                                                    18


<210>  12
<211>  18
<212>  DNA
<213>  Artificial sequence

<220>
<223>  Synthetic oligonucleotide

<400>  12
agcagaacaa actccact                                                    18


<210>  13
<211>  18
<212>  DNA
<213>  Artificial sequence

<220>
<223>  Synthetic oligonucleotide

<400>  13
tcccagcaga acaaactc                                                    18


<210>  14
<211>  18
<212>  DNA
<213>  Artificial sequence

<220>
<223>  Synthetic oligonucleotide

<400>  14
aagctcccag cagaacaa                                                    18


<210>  15
<211>  18
<212>  DNA
<213>  Artificial sequence

<220>
<223>  Synthetic oligonucleotide

<400>  15
aacaaagctc ccagcaga                                                    18


<210>  16
<211>  18
<212>  DNA
<213>  Artificial sequence

<220>
<223>  Synthetic oligonucleotide

<400>  16
ccagaacaaa gctcccag                                                    18


<210>  17
<211>  18
<212>  DNA
<213>  Artificial sequence

<220>
<223>  Synthetic oligonucleotide

<400>  17
gcaaccagaa caaagctc                                                    18


<210>  18
<211>  18
<212>  DNA
<213>  Artificial sequence

<220>
<223>  Synthetic oligonucleotide

<400>  18
gaaggcaacc agaacaaa                                                    18


<210>  19
<211>  18
<212>  DNA
<213>  Artificial sequence

<220>
<223>  Synthetic oligonucleotide

<400>  19
gagagaaggc aaccagaa                                                    18


<210>  20
<211>  18
<212>  DNA
<213>  Artificial sequence

<220>
<223>  Synthetic oligonucleotide

<400>  20
gactgagaga aggcaacc                                                    18


<210>  21
<211>  18
<212>  DNA
<213>  Artificial sequence

<220>
<223>  Synthetic oligonucleotide

<400>  21
cactgactga gagaaggc                                                    18

<210>  22
<211>  18
<212>  DNA
<213>  Artificial sequence

<220>
<223>  Synthetic oligonucleotide

<400>  22
accccactga ctgagaga                                                    18


<210>  23
<211>  18
<212>  DNA
<213>  Artificial sequence

<220>
<223>  Synthetic oligonucleotide

<400>  23
cttaacccca ctgactga                                                    18


<210>  24
<211>  18
<212>  DNA
<213>  Artificial sequence

<220>
<223>  Synthetic oligonucleotide

<400>  24
caggcttaac cccactga                                                    18


<210>  25
<211>  18
<212>  DNA
<213>  Artificial sequence

<220>
<223>  Synthetic oligonucleotide

<400>  25
cacacaggct taacccca                                                    18


<210>  26
<211>  18
<212>  DNA
<213>  Artificial sequence

<220>
<223>  Synthetic oligonucleotide

<400>  26
tcaccacaca ggcttaac                                                    18


<210>  27
<211>  18

<212>  DNA
<213>  Artificial sequence

<220>
<223>  Synthetic oligonucleotide

<400>  27
gtactcacca cacaggct                                                    18


<210>  28
<211>  18
<212>  DNA
<213>  Artificial sequence

<220>
<223>  Synthetic oligonucleotide

<400>  28
cttcgtactc accacaca                                                    18


<210>  29
<211>  18
<212>  DNA
<213>  Artificial sequence

<220>
<223>  Synthetic oligonucleotide

<400>  29
ccagcttcgt actcacca                                                    18


<210>  30
<211>  18
<212>  DNA
<213>  Artificial sequence

<220>
<223>  Synthetic oligonucleotide

<400>  30
tctcccagct tcgtactc                                                    18


<210>  31
<211>  18
<212>  DNA
<213>  Artificial sequence

<220>
<223>  Synthetic oligonucleotide

<400>  31
gggatggaat ggagaagc                                                    18


<210>  32
<211>  18
<212>  DNA
<213>  Artificial sequence

<220>
<223>  Synthetic oligonucleotide

<400>  32
agctgggatg gaatggag                                                     18


<210>  33
<211>  18
<212>  DNA
<213>  Artificial sequence

<220>
<223>  Synthetic oligonucleotide

<400>  33
aaatagctgg gatggaat                                                     18


<210>  34
<211>  18
<212>  DNA
<213>  Artificial sequence

<220>
<223>  Synthetic oligonucleotide

<400>  34
caagaaatag ctgggatg                                                     18


<210>  35
<211>  18
<212>  DNA
<213>  Artificial sequence

<220>
<223>  Synthetic oligonucleotide

<400>  35
gcaacaagaa atagctgg                                                     18


<210>  36
<211>  18
<212>  DNA
<213>  Artificial sequence

<220>
<223>  Synthetic oligonucleotide

<400>  36
gtgagcaaca agaaatag                                                     18


<210>  37
<211>  18
<212>  DNA
<213>  Artificial sequence

<220>
<223>  Synthetic oligonucleotide

<400>  37
agacgtgagc aacaagaa                                                    18


<210>  38
<211>  18
<212>  DNA
<213>  Artificial sequence

<220>
<223>  Synthetic oligonucleotide

<400>  38
caggagacgt gagcaaca                                                    18


<210>  39
<211>  18
<212>  DNA
<213>  Artificial sequence

<220>
<223>  Synthetic oligonucleotide

<400>  39
ggttcaggag acgtgagc                                                    18


<210>  40
<211>  18
<212>  DNA
<213>  Artificial sequence

<220>
<223>  Synthetic oligonucleotide

<400>  40
gggttcagga gacgtgag                                                    18


<210>  41
<211>  18
<212>  DNA
<213>  Artificial sequence

<220>
<223>  Synthetic oligonucleotide

<400>  41
cccctccagg gtccaggg                                                    18


<210>  42
<211>  18
<212>  DNA
<213>  Artificial sequence

<220>
<223>  Synthetic oligonucleotide

<400>  42
tttcccctcc agggtcca                                                    18

<210>  43
<211>  18
<212>  DNA
<213>  Artificial sequence

<220>
<223>  Synthetic oligonucleotide

<400>  43
tcgttttccc ctccaggg                                                    18


<210>  44
<211>  18
<212>  DNA
<213>  Artificial sequence

<220>
<223>  Synthetic oligonucleotide

<400>  44
tgcctcgttt tcccctcc                                                    18


<210>  45
<211>  18
<212>  DNA
<213>  Artificial sequence

<220>
<223>  Synthetic oligonucleotide

<400>  45
tctctgcctc gttttccc                                                    18


<210>  46
<211>  18
<212>  DNA
<213>  Artificial sequence

<220>
<223>  Synthetic oligonucleotide

<400>  46
gcagtctctg cctcgttt                                                    18


<210>  47
<211>  18
<212>  DNA
<213>  Artificial sequence

<220>
<223>  Synthetic oligonucleotide

<400>  47
ggcagcagtc tctgcctc                                                    18


<210>  48
<211>  18

<212>  DNA
<213>  Artificial sequence

<220>
<223>  Synthetic oligonucleotide

<400>  48
gccgggcagc agtctctg                                                    18


<210>  49
<211>  18
<212>  DNA
<213>  Artificial sequence

<220>
<223>  Synthetic oligonucleotide

<400>  49
ggctgccggg cagcagtc                                                    18


<210>  50
<211>  18
<212>  DNA
<213>  Artificial sequence

<220>
<223>  Synthetic oligonucleotide

<400>  50
gagaggctgc cgggcagc                                                    18


<210>  51
<211>  18
<212>  DNA
<213>  Artificial sequence

<220>
<223>  Synthetic oligonucleotide

<400>  51
ctatgagagg ctgccggg                                                    18


<210>  52
<211>  18
<212>  DNA
<213>  Artificial sequence

<220>
<223>  Synthetic oligonucleotide

<400>  52
gtgcctatga gaggctgc                                                    18


<210>  53
<211>  18
<212>  DNA
<213>  Artificial sequence

<220>
<223>  Synthetic oligonucleotide

<400>  53
ctcggtgcct atgagagg                                                    18


<210>  54
<211>  18
<212>  DNA
<213>  Artificial sequence

<220>
<223>  Synthetic oligonucleotide

<400>  54
gggtctcggt gcctatga                                                    18


<210>  55
<211>  18
<212>  DNA
<213>  Artificial sequence

<220>
<223>  Synthetic oligonucleotide

<400>  55
cctggctttg actctggg                                                    18


<210>  56
<211>  18
<212>  DNA
<213>  Artificial sequence

<220>
<223>  Synthetic oligonucleotide

<400>  56
cctacctggc tttgactc                                                    18


<210>  57
<211>  18
<212>  DNA
<213>  Artificial sequence

<220>
<223>  Synthetic oligonucleotide

<400>  57
gcatcctacc tggctttg                                                    18


<210>  58
<211>  18
<212>  DNA
<213>  Artificial sequence

<220>
<223>  Synthetic oligonucleotide

<400>  58
ctttgcatcc tacctggc                                                    18


<210>  59
<211>  18
<212>  DNA
<213>  Artificial sequence

<220>
<223>  Synthetic oligonucleotide

<400>  59
gggcctttgc atcctacc                                                    18


<210>  60
<211>  18
<212>  DNA
<213>  Artificial sequence

<220>
<223>  Synthetic oligonucleotide

<400>  60
gggagggcct ttgcatcc                                                    18


<210>  61
<211>  18
<212>  DNA
<213>  Artificial sequence

<220>
<223>  Synthetic oligonucleotide

<400>  61
agctgatcat attctacc                                                    18


<210>  62
<211>  25
<212>  DNA
<213>  Artificial sequence

<220>
<223>  Synthetic oligonucleotide

<400>  62
agctgatcat attctacctg gtgct                                            25


<210>  63
<211>  18
<212>  DNA
<213>  Artificial sequence

<220>
<223>  Synthetic oligonucleotide

<400>  63
aaaggcaacc aggacgaa                                                    18

<210>  64
<211>  18
<212>  DNA
<213>  Artificial sequence

<220>
<223>  Synthetic oligonucleotide

<400>  64
aaatgctctg atgtggtt                                                    18


<210>  65
<211>  18
<212>  DNA
<213>  Artificial sequence

<220>
<223>  Synthetic oligonucleotide

<400>  65
acaaatccca ctgacgag                                                    18


<210>  66
<211>  18
<212>  DNA
<213>  Artificial sequence

<220>
<223>  Synthetic oligonucleotide

<400>  66
acaggctggt ccccacag                                                    18


<210>  67
<211>  18
<212>  DNA
<213>  Artificial sequence

<220>
<223>  Synthetic oligonucleotide

<400>  67
accacacagg ctggtccc                                                    18


<210>  68
<211>  18
<212>  DNA
<213>  Artificial sequence

<220>
<223>  Synthetic oligonucleotide

<400>  68
actctcagca tctcagcc                                                    18


<210>  69
<211>  18

<212>  DNA
<213>  Artificial sequence

<220>
<223>  Synthetic oligonucleotide

<400>  69
aggacgaagc ttccagca                                                    18


<210>  70
<211>  18
<212>  DNA
<213>  Artificial sequence

<220>
<223>  Synthetic oligonucleotide

<400>  70
caaccaggac gaagcttc                                                    18


<210>  71
<211>  18
<212>  DNA
<213>  Artificial sequence

<220>
<223>  Synthetic oligonucleotide

<400>  71
cactcaccac acaggctg                                                    18


<210>  72
<211>  18
<212>  DNA
<213>  Artificial sequence

<220>
<223>  Synthetic oligonucleotide

<400>  72
cagaaccaca cactcacc                                                    18


<210>  73
<211>  18
<212>  DNA
<213>  Artificial sequence

<220>
<223>  Synthetic oligonucleotide

<400>  73
cagaatgaga aaaggcaa                                                    18


<210>  74
<211>  18
<212>  DNA
<213>  Artificial sequence

<220>
<223>  Synthetic oligonucleotide

<400>  74
ccacacactc accacaca                                                    18


<210>  75
<211>  18
<212>  DNA
<213>  Artificial sequence

<220>
<223>  Synthetic oligonucleotide

<400>  75
ccatctgaca cagaacca                                                    18


<210>  76
<211>  18
<212>  DNA
<213>  Artificial sequence

<220>
<223>  Synthetic oligonucleotide

<400>  76
cccacaaatg ctctgatg                                                    18


<210>  77
<211>  18
<212>  DNA
<213>  Artificial sequence

<220>
<223>  Synthetic oligonucleotide

<400>  77
ccccacagaa tgagaaaa                                                    18


<210>  78
<211>  18
<212>  DNA
<213>  Artificial sequence

<220>
<223>  Synthetic oligonucleotide

<400>  78
ctctgatgtg gttcctcg                                                    18


<210>  79
<211>  18
<212>  DNA
<213>  Artificial sequence

<220>
<223>  Synthetic oligonucleotide

<400>  79
ctggtcccca cagaatga                                                    18


<210>  80
<211>  18
<212>  DNA
<213>  Artificial sequence

<220>
<223>  Synthetic oligonucleotide

<400>  80
gaagcttcca gcagcaca                                                    18


<210>  81
<211>  18
<212>  DNA
<213>  Artificial sequence

<220>
<223>  Synthetic oligonucleotide

<400>  81
gcagcacaaa tcccactg                                                    18


<210>  82
<211>  18
<212>  DNA
<213>  Artificial sequence

<220>
<223>  Synthetic oligonucleotide

<400>  82
gctccccatc tgacacag                                                    18


<210>  83
<211>  18
<212>  DNA
<213>  Artificial sequence

<220>
<223>  Synthetic oligonucleotide

<400>  83
ggaaggtcgg tctctact                                                    18


<210>  84
<211>  18
<212>  DNA
<213>  Artificial sequence

<220>
<223>  Synthetic oligonucleotide

<400>  84
ggtgagaagg gaagggag                                                    18

<210>  85
<211>  18
<212>  DNA
<213>  Artificial sequence

<220>
<223>  Synthetic oligonucleotide

<400>  85
gtcggtctct actctcag                                                    18


<210>  86
<211>  18
<212>  DNA
<213>  Artificial sequence

<220>
<223>  Synthetic oligonucleotide

<400>  86
tcccactgac gagaaccc                                                    18


<210>  87
<211>  18
<212>  DNA
<213>  Artificial sequence

<220>
<223>  Synthetic oligonucleotide

<400>  87
tctctactct cagcatct                                                    18


<210>  88
<211>  18
<212>  DNA
<213>  Artificial sequence

<220>
<223>  Synthetic oligonucleotide

<400>  88
tgacacagaa ccacacac                                                    18


<210>  89
<211>  18
<212>  DNA
<213>  Artificial sequence

<220>
<223>  Synthetic oligonucleotide

<400>  89
tgagaaaagg caaccagg                                                    18


<210>  90
<211>  18

<212>  DNA
<213>  Artificial sequence

<220>
<223>  Synthetic oligonucleotide

<400>  90
ttccagcagc acaaatcc                                                    18


<210>  91
<211>  18
<212>  DNA
<213>  Artificial sequence

<220>
<223>  Synthetic oligonucleotide

<400>  91
gggaaggtcc ccagggac                                                    18


<210>  92
<211>  18
<212>  DNA
<213>  Artificial sequence

<220>
<223>  Synthetic oligonucleotide

<400>  92
tggctgggaa ggtcccca                                                    18


<210>  93
<211>  18
<212>  DNA
<213>  Artificial sequence

<220>
<223>  Synthetic oligonucleotide

<400>  93
cctactggct gggaaggt                                                    18


<210>  94
<211>  18
<212>  DNA
<213>  Artificial sequence

<220>
<223>  Synthetic oligonucleotide

<400>  94
aaaaccctac tggctggg                                                    18


<210>  95
<211>  18
<212>  DNA
<213>  Artificial sequence

<220>
<223>  Synthetic oligonucleotide

<400>  95
gtcagaaaac cctactgg                                                    18


<210>  96
<211>  18
<212>  DNA
<213>  Artificial sequence

<220>
<223>  Synthetic oligonucleotide

<400>  96
gcagggtcag aaaaccct                                                    18


<210>  97
<211>  18
<212>  DNA
<213>  Artificial sequence

<220>
<223>  Synthetic oligonucleotide

<400>  97
tgaaggcagg gtcagaaa                                                    18


<210>  98
<211>  18
<212>  DNA
<213>  Artificial sequence

<220>
<223>  Synthetic oligonucleotide

<400>  98
taggatgaag gcagggtc                                                    18


<210>  99
<211>  18
<212>  DNA
<213>  Artificial sequence

<220>
<223>  Synthetic oligonucleotide

<400>  99
aggagtagga tgaaggca                                                    18


<210>  100
<211>  18
<212>  DNA
<213>  Artificial sequence

<220>
<223>  Synthetic oligonucleotide

<400>  100
tagctaggag taggatga                                                    18


<210>  101
<211>  18
<212>  DNA
<213>  Artificial sequence

<220>
<223>  Synthetic oligonucleotide

<400>  101
agaaatagct aggagtag                                                    18


<210>  102
<211>  18
<212>  DNA
<213>  Artificial sequence

<220>
<223>  Synthetic oligonucleotide

<400>  102
caacaagaaa tagctagg                                                    18


<210>  103
<211>  18
<212>  DNA
<213>  Artificial sequence

<220>
<223>  Synthetic oligonucleotide

<400>  103
gagatgtgag caacaaga                                                    18


<210>  104
<211>  18
<212>  DNA
<213>  Artificial sequence

<220>
<223>  Synthetic oligonucleotide

<400>  104
ctcaggagat gtgagcaa                                                    18


<210>  105
<211>  18
<212>  DNA
<213>  Artificial sequence

<220>
<223>  Synthetic oligonucleotide

<400>  105
tgggcctcag gagatgtg                                                    18

<210>  106
<211>  18
<212>  DNA
<213>  Artificial sequence

<220>
<223>  Synthetic oligonucleotide

<400>  106
ggtcctgggc ctcaggag                                                    18


<210>  107
<211>  18
<212>  DNA
<213>  Artificial sequence

<220>
<223>  Synthetic oligonucleotide

<400>  107
tccagggtcc tgggcctc                                                    18


<210>  108
<211>  18
<212>  DNA
<213>  Artificial sequence

<220>
<223>  Synthetic oligonucleotide

<400>  108
tcccctccag ggtcctgg                                                    18


<210>  109
<211>  18
<212>  DNA
<213>  Artificial sequence

<220>
<223>  Synthetic oligonucleotide

<400>  109
cttcttcccc tccagggt                                                    18


<210>  110
<211>  18
<212>  DNA
<213>  Artificial sequence

<220>
<223>  Synthetic oligonucleotide

<400>  110
tgcttcttct tcccctcc                                                    18


<210>  111
<211>  18

<212>  DNA
<213>  Artificial sequence

<220>
<223>  Synthetic oligonucleotide

<400>  111
ctctctgctt cttcttcc                                                    18


<210>  112
<211>  18
<212>  DNA
<213>  Artificial sequence

<220>
<223>  Synthetic oligonucleotide

<400>  112
ctgcgctctc tgcttctt                                                    18


<210>  113
<211>  18
<212>  DNA
<213>  Artificial sequence

<220>
<223>  Synthetic oligonucleotide

<400>  113
ccgggctgcg ctctctgc                                                    18


<210>  114
<211>  18
<212>  DNA
<213>  Artificial sequence

<220>
<223>  Synthetic oligonucleotide

<400>  114
ggctgccggg ctgcgctc                                                    18


<210>  115
<211>  18
<212>  DNA
<213>  Artificial sequence

<220>
<223>  Synthetic oligonucleotide

<400>  115
gggcctcggt tcttatga                                                    18


<210>  116
<211>  18
<212>  DNA
<213>  Artificial sequence

<220>
<223>  Synthetic oligonucleotide

<400>  116
cctacctggc ttcgactc                                                     18


<210>  117
<211>  18
<212>  DNA
<213>  Artificial sequence

<220>
<223>  Synthetic oligonucleotide

<400>  117
tgtctcctac ctggcttc                                                     18


<210>  118
<211>  18
<212>  DNA
<213>  Artificial sequence

<220>
<223>  Synthetic oligonucleotide

<400>  118
gtctgtgtct cctacctg                                                     18


<210>  119
<211>  18
<212>  DNA
<213>  Artificial sequence

<220>
<223>  Synthetic oligonucleotide

<400>  119
tgagggtctg tgtctcct                                                     18


<210>  120
<211>  18
<212>  DNA
<213>  Artificial sequence

<220>
<223>  Synthetic oligonucleotide

<400>  120
ctctctgagg gtctgtgt                                                     18


<210>  121
<211>  18
<212>  DNA
<213>  Artificial sequence

<220>
<223>  Synthetic oligonucleotide

<400>  121
gtgacctctc tgagggtc                                                    18


<210>  122
<211>  18
<212>  DNA
<213>  Artificial sequence

<220>
<223>  Synthetic oligonucleotide

<400>  122
agaaagtgac ctctctga                                                    18


<210>  123
<211>  18
<212>  DNA
<213>  Artificial sequence

<220>
<223>  Synthetic oligonucleotide

<400>  123
gagaaagaaa gtgacctc                                                    18


<210>  124
<211>  18
<212>  DNA
<213>  Artificial sequence

<220>
<223>  Synthetic oligonucleotide

<400>  124
ccagagagaa agaaagtg                                                    18


<210>  125
<211>  18
<212>  DNA
<213>  Artificial sequence

<220>
<223>  Synthetic oligonucleotide

<400>  125
caaacccaga gagaaaga                                                    18


<210>  126
<211>  18
<212>  DNA
<213>  Artificial sequence

<220>
<223>  Synthetic oligonucleotide

<400>  126
aaggccaaac ccagagag                                                    18

<210> 127
<211> 18
<212> DNA
<213> Artificial sequence

<220>
<223> Synthetic oligonucleotide

<400> 127
aggaaaaggc caaaccca 18

## Description

### SEQUENCE LISTING

The present application is being filed along with a Sequence Listing in electronic format. The Sequence Listing is provided as a file entitled CORE0120WOSEQ_ST25.txt, created November 25, 2014, which is 68 kb in size. The information in the electronic format of the sequence listing is incorporated herein by reference in its entirety.

### BACKGROUND

Juvenile neuronal ceroid lipofuscinosis (JNCL), also known as Batten Disease, is the most common of the NCL disorders, a group of childhood neurodegenerative diseases. JNCL occurs in approximately 1 in 25,000 births in the United States and Europe and has been reported in many other countries worldwide. Onset occurs between five and eight years of age and symptoms include progressive loss of motor function, seizures, vision loss, and loss of cognitive function, resulting in death before age 30. JNCL is an autosomal recessive disorder caused by mutations of the CLN3 gene. There are forty-nine known mutations of CLN3, but approximately 80% of JNCL cases result from a particular deletion of the CLN3 gene spanning exons 7 and 8 (*CLN3*Δ*78*)*.* The *CLN3*Δ*78* deletion causes a frameshift that results in a premature stop codon in exon 9. The truncated protein product of *CLN3*Δ*78* is 33% of the length of the wild type. The function of the CLN3 protein is not well understood, but it is implicated in many important processes, for example, membrane trafficking, phospholipid distribution, and response to oxidative stress. Currently, there are no treatments for any of the NCL disorders, and patient options are limited to remedial management of symptoms *(see* Bennett and Rakheja, Dev. Disabil. Res. Rev. 2013, 17, 254-259).

Antisense compounds have been used to modulate target nucleic acids. Antisense compounds comprising a variety of chemical modifications and motifs have been reported. In certain instances, such compounds are useful as research tools, diagnostic reagents, and as therapeutic agents. In certain instances antisense compounds have been shown to modulate protein expression by binding to a target messenger RNA (mRNA) encoding the protein. In certain instances, such binding of an antisense compound to its target mRNA results in cleavage of the mRNA. Antisense compounds that modulate processing of a pre-mRNA have also been reported. Such antisense compounds alter splicing, interfere with polyadenlyation or prevent formation of the 5'-cap of a pre-mRNA.

Certain antisense compounds have been described previously. See for example United States Patent No. 7,399,845 and published International Patent Application No. WO 2008/049085, which are hereby incorporated by reference herein in their entirety.

### SUMMARY

Many JNCL cases result from a particular deletion of the CLN3 gene spanning exons 7 and 8 (*CLN3*Δ*78*)*.* The *CLN3*Δ*78* deletion causes a frameshift that results in a premature stop codon in exon 9. The truncated protein product of *CLN3*Δ*78* is 33% of the length of the wild type. In certain embodiments, the truncated *CLN3*Δ*78* protein causes a variety of cellular defects, including lysosomal and transporter dysfunction. In certain embodiments, modified oligonucleotides targeted to CLN3 pre-mRNA are able to induce skipping of one or more exons and thereby prevent the frameshift that results in a premature stop codon in exon 9.

For example, in certain embodiments, modified oligonucleotides targeted to exon 6 CLN3 pre-mRNA may induce skipping on exon 6 and prevent recognition of the premature stop codon in exon 9, thereby producing a truncated CLN3 protein having restored functionality compared to the *CLN3*Δ*78* isoform. In certain embodiments, modified oligonucleotides targeted to exon 9 CLN3 pre-mRNA may induce skipping on exon 9 and prevent recognition of the premature stop codon in exon 9, thereby producing a truncated CLN3 protein having restored functionality compared to the *CLN3*Δ*78* isoform.

In certain embodiments, the present disclosure provides compounds comprising oligonucleotides. In certain embodiments, such oligonucleotides are complementary to a ceroid-lipofuscinosis, neuronal 3 ("CLN3") transcript. In certain such embodiments, oligonucleotides are complementary to a target region of the CLN3 transcript comprising exon 6. In certain such embodiments, oligonucleotides are complementary to a target region of the CLN3 transcript comprising an intron adjacent to exon 6. In certain such embodiments, oligonucleotides are complementary to a target region of the CLN3 transcript comprising an intron adjacent to exon 6 and downstream of exon 6. In certain such embodiments, oligonucleotides are complementary to a target region of the CLN3 transcript comprising an intron adjacent to exon 6 and upstream of exon 6.

In certain such embodiments, oligonucleotides are complementary to a target region of the CLN3 transcript comprising exon 9. In certain such embodiments, oligonucleotides are complementary to a target region of the CLN3 transcript comprising an intron adjacent to exon 9. In certain such embodiments, oligonucleotides are complementary to a target region of the CLN3 transcript comprising an intron adjacent to exon 9 and downstream of exon 9. In certain such embodiments, oligonucleotides are complementary to a target region of the CLN3 transcript comprising an intron adjacent to exon 9 and upstream of exon 9.

In certain embodiments, oligonucleotides promote skipping of exon 6. In certain embodiments, oligonucleotides promote skipping of exon 6 of a *CLN3*Δ*78* transcript. In certain embodiments, oligonucleotides promote skipping of exon 9. In certain embodiments, oligonucleotides promote skipping of exon 9 of a *CLN3*Δ*78* transcript.

In certain embodiments, oligonucleotides promote skipping of exons 6, 7, and 8. In certain embodiments, oligonucleotides promote skipping of exons 7, 8, and 9. In certain embodiments, oligonucleotides promote skipping of exon 6. In certain embodiments, oligonucleotides promote skipping of exon 9.

In certain embodiments, including, but not limited to any of the above numbered embodiments, the CLN3 transcript is in a human. In certain embodiments, including, but not limited to any of the above numbered embodiments, the CLN3 transcript is in a mouse.

The present disclosure provides the following non-limiting numbered embodiments:
Embodiment 1. A compound comprising a modified oligonucleotide consisting of 8 to 30 linked nucleosides and having a nucleobase sequence comprising a complementary region, wherein the complementary region comprises at least 8 contiguous nucleobases and is complementary to an equal-length portion of a target region of a CLN3 transcript.
Embodiment 2. The compound of embodiment 1, wherein the target region of the CLN3 transcript comprises at least a portion of exon 6 of the CLN3 transcript.
Embodiment 3. The compound of embodiment 1, wherein the target region of the CLN3 transcript comprises at least a portion of exon 9 of the CLN3 transcript.
Embodiment 4. The compound of embodiment 1, wherein the target region of the CLN3 transcript comprises at least a portion of intron 5 of the CLN3 transcript.
Embodiment 5. The compound of embodiment 1, wherein the target region of the CLN3 transcript comprises at least a portion of intron 6 of the CLN3 transcript.
Embodiment 6. The compound of embodiment 1, wherein the target region of the CLN3 transcript comprises at least a portion of intron 9 of the CLN3 transcript.
Embodiment 7. The compound of embodiment 1, wherein the target region of the CLN3 transcript comprises at least a portion of intron 10 of the CLN3 transcript.
Embodiment 8. The compound of any of embodiments 1to 7, wherein the complementary region of the modified oligonucleotide is 100% complementary to the target region.
Embodiment 9. The compound of any of embodiments 1 to 8, wherein the complementary region of the modified oligonucleotide comprises at least 10 contiguous nucleobases.
Embodiment 10. The compound of any of embodiments 1 to 8, wherein the complementary region of the modified oligonucleotide comprises at least 12 contiguous nucleobases.
Embodiment 11. The compound of any of embodiments 1 to 8, wherein the complementary region of the modified oligonucleotide comprises at least 14 contiguous nucleobases.
Embodiment 12. The compound of any of embodiments 1 to 8, wherein the complementary region of the modified oligonucleotide comprises at least 15 contiguous nucleobases.
Embodiment 13. The compound of any of embodiments 1 to 8, wherein the complementary region of the modified oligonucleotide comprises at least 16 contiguous nucleobases.
Embodiment 14. The compound of any of embodiments 1 to 8, wherein the complementary region of the modified oligonucleotide comprises at least 17 contiguous nucleobases.
Embodiment 15. The compound of any of embodiments 1 to 8, wherein the complementary region of the modified oligonucleotide comprises at least 18 contiguous nucleobases.
Embodiment 16. The compound of any of embodiments 1 to 8, wherein the complementary region of the modified oligonucleotide comprises at least 19 contiguous nucleobases.
Embodiment 17. The compound of any of embodiments 1 to 8, wherein the complementary region of the modified oligonucleotide comprises at least 20 contiguous nucleobases.
Embodiment 18. The compound of any of embodiments 1 to 17, wherein the nucleobase sequence of the oligonucleotide is at least 80% complementary to an equal-length region of the CLN3 transcript, as measured over the entire length of the oligonucleotide.
Embodiment 19. The compound of any of embodiments 1 to 17, wherein the nucleobase sequence of the oligonucleotide is at least 90% complementary to an equal-length region of the CLN3 transcript, as measured over the entire length of the oligonucleotide.
Embodiment 20. The compound of any of embodiments 1 to 17, wherein the nucleobase sequence of the oligonucleotide is 100% complementary to an equal-length region of the CLN3 transcript, as measured over the entire length of the oligonucleotide.
Embodiment 21. The compound of any of embodiments 1-2 or 8 to 20, wherein the target region is within nucleobase 5053 and nucleobase 5070 of SEQ ID NO.: 2.
Embodiment 22. The compound of any of embodiments 1-2 or 8 to 20, wherein the target region is within nucleobase 5082 and nucleobase 5119 of SEQ ID NO.: 2.
Embodiment 23. The compound of any of embodiments 1-2 or 8 to 20, wherein the target region is within nucleobase 5082 and nucleobase 5099 of SEQ ID NO.: 2.
Embodiment 24. The compound of any of embodiments 1-2 or 8 to 20, wherein the target region is within nucleobase 5086 and nucleobase 5103 of SEQ ID NO.: 2.
Embodiment 25. The compound of any of embodiments 1-2 or 8 to 20, wherein the target region is within nucleobase 5090 and nucleobase 5107 of SEQ ID NO.: 2.
Embodiment 26. The compound of any of embodiments 1-2 or 8 to 20, wherein the target region is within nucleobase 5094 and nucleobase 5111 of SEQ ID NO.: 2.
Embodiment 27. The compound of any of embodiments 1-2 or 8 to 20, wherein the target region is within nucleobase 5098 and nucleobase 5115 of SEQ ID NO.: 2.
Embodiment 28. The compound of any of embodiments 1-2 or 8 to 20, wherein the target region is within nucleobase 5102 and nucleobase 5119 of SEQ ID NO.: 2.
Embodiment 29. The compound of any of embodiments 1-2, 5, or 8 to 20, wherein the target region is within nucleobase 5126 and nucleobase 5143 of SEQ ID NO.: 2.
Embodiment 30. The compound of any of embodiments 1-2, 5 or 8 to 20, wherein the target region is within nucleobase 5134 and nucleobase 5155 of SEQ ID NO.: 2.
Embodiment 31. The compound of any of embodiments 1-2, 5, or 8 to 20, wherein the target region is within nucleobase 5134 and nucleobase 5151 of SEQ ID NO.: 2.
Embodiment 32. The compound of any of embodiments 1-2, 5 or 8 to 20, wherein the target region is within nucleobase 5138 and nucleobase 5155 of SEQ ID NO.: 2.
Embodiment 33. The compound of any of embodiments 1, 3, or 8 to 20, wherein the target region is within nucleobase 7366 and nucleobase 7411 of SEQ ID NO.: 2.
Embodiment 34. The compound of any of embodiments 1, 3, or 8 to 20, wherein the target region is within nucleobase 7366 and nucleobase 7383 of SEQ ID NO.: 2.
Embodiment 35. The compound of any of embodiments 1, 3, or 8 to 20, wherein the target region is within nucleobase 7370 and nucleobase 7387 of SEQ ID NO.: 2.
Embodiment 36. The compound of any of embodiments 1, 3, or 8 to 20, wherein the target region is within nucleobase 7371 and nucleobase 7388 of SEQ ID NO.: 2.
Embodiment 37. The compound of any of embodiments 1, 3, or 8 to 20, wherein the target region is within nucleobase 7387 and nucleobase 7404 of SEQ ID NO.: 2.
Embodiment 38. The compound of any of embodiments 1, 3, or 8 to 20, wherein the target region is within nucleobase 7394 and nucleobase 7411 of SEQ ID NO.: 2.
Embodiment 39. The compound of any of embodiments 1, 3, 6, or 8 to 20, wherein the target region is within nucleobase 7454 and nucleobase 7471 of SEQ ID NO.: 2.
Embodiment 40. The compound of any of embodiments 1, 3, 6, or 8 to 20, wherein the target region is within nucleobase 7462 and nucleobase 7483 of SEQ ID NO.: 2.
Embodiment 41. The compound of any of embodiments 1, 3, 6, or 8 to 20, wherein the target region is within nucleobase 7462 and nucleobase 7479 of SEQ ID NO.: 2.
Embodiment 42. The compound of any of embodiments 1, 3, 6, or 8 to 20, wherein the target region is within nucleobase 7466 and nucleobase 7483 of SEQ ID NO.: 2.
Embodiment 43. The compound of any of embodiments 1-42, wherein the nucleobase sequence of the antisense oligonucleotide comprises any one of SEQ ID NOs: 3 to 60.
Embodiment 44. The compound of any of embodiments 1-43, wherein the modified oligonucleotide comprises at least one modified nucleoside.
Embodiment 45. The compound of embodiment 44, wherein at least one modified nucleoside comprises a modified sugar moiety.
Embodiment 46. The compound of embodiment 45, wherein at least one modified sugar moiety is a 2'-substituted sugar moiety.
Embodiment 47. The compound of embodiment 46, wherein the 2'-substitutent of at least one 2'-substituted sugar moiety is selected from among: 2'-OMe, 2'-F, and 2'-MOE.
Embodiment 48. The compound of any of embodiments 44-47, wherein the 2'-substiuent of at least one 2'-substituted sugar moiety is a 2'-MOE.
Embodiment 49. The compound of any of embodiments 1-48, wherein at least one modified sugar moiety is a bicyclic sugar moiety.
Embodiment 50. The compound of embodiment 49, wherein at least one bicyclic sugar moiety is LNA or cEt.
Embodiment 51. The compound of any of embodiments 1-51, wherein at least one sugar moiety is a sugar surrogate.
Embodiment 52. The compound of embodiment 51, wherein at least one sugar surrogate is a morpholino.
Embodiment 53. The compound of embodiment 51, wherein at least one sugar surrogate is a modified morpholino.
Embodiment 54. The compound of any of embodiment 1-53, wherein the modified oligonucleotide comprises at least 5 modified nucleosides, each independently comprising a modified sugar moiety.
Embodiment 55. The compound of embodiment 54, wherein the modified oligonucleotide comprises at least 10 modified nucleosides, each independently comprising a modified sugar moiety.
Embodiment 56. The compound of embodiment 54, wherein the modified oligonucleotide comprises at least 15 modified nucleosides, each independently comprising a modified sugar moiety.
Embodiment 57. The compound of embodiment 54, wherein each nucleoside of the modified oligonucleotide is a modified nucleoside, each independently comprising a modified sugar moiety.
Embodiment 58. The compound of any of embodiments 1-57, wherein the modified oligonucleotide comprises at least two modified nucleosides comprising modified sugar moieties that are the same as one another.
Embodiment 59. The compound of any of embodiments 1-57, wherein the modified oligonucleotide comprises at least two modified nucleosides comprising modified sugar moieties that are different from one another.
Embodiment 60. The compound of any of embodiments 1-59, wherein the modified oligonucleotide comprises a modified region of at least 5 contiguous modified nucleosides.
Embodiment 61. The compound of any of embodiments 1 to 60, wherein the modified oligonucleotide comprises a modified region of at least 10 contiguous modified nucleosides.
Embodiment 62. The compound of any of embodiments 1 to 61, wherein the modified oligonucleotide comprises a modified region of at least 15 contiguous modified nucleosides.
Embodiment 63. The compound of any of embodiments 1 to 61, wherein the modified oligonucleotide comprises a modified region of at least 20 contiguous modified nucleosides.
Embodiment 64. The compound of any of embodiments 58 to 63, wherein each modified nucleoside of the modified region has a modified sugar moiety independently selected from among: 2'-F, 2'-OMe, 2'-MOE, cEt, LNA, morpholino, and modified morpholino.
Embodiment 65. The compound of any of embodiments 58 to 64, wherein the modified nucleosides of the modified region each comprise the same modification as one another.
Embodiment 66. The compound of embodiment 65, wherein the modified nucleosides of the modified region each comprise the same 2'-substituted sugar moiety.
Embodiment 67. The compound of embodiment 65, wherein the 2'-substituted sugar moiety of the modified nucleosides of the region of modified nucleosides is selected from 2'-F, 2'-OMe, and 2'-MOE.
Embodiment 68. The compound of embodiment 67, wherein the 2'-substituted sugar moiety of the modified nucleosides of the region of modified nucleosides is 2'-MOE.
Embodiment 69. The compound of embodiment 65, wherein the modified nucleosides of the region of modified nucleosides each comprise the same bicyclic sugar moiety.
Embodiment 70. The compound of embodiment 69, wherein the bicyclic sugar moiety of the modified nucleosides of the region of modified nucleosides is selected from LNA and cEt.
Embodiment 71. The compound of embodiment 65, wherein the modified nucleosides of the region of modified nucleosides each comprises a sugar surrogate.
Embodiment 72. The compound of embodiment 71, wherein the sugar surrogate of the modified nucleosides of the region of modified nucleosides is a morpholino.
Embodiment 73. The compound of embodiment 71, wherein the sugar surrogate of the modified nucleosides of the region of modified nucleosides is a modified morpholino.
Embodiment 74. The compound of any of embodiments 1 to 73, wherein the modified nucleotide comprises no more than 4 contiguous naturally occurring nucleosides.
Embodiment 75. The compound of any of embodiments 1 to 74, wherein each nucleoside of the modified oligonucleotide is a modified nucleoside.
Embodiment 76. The compound of embodiment 75 wherein each modified nucleoside comprises a modified sugar moiety.
Embodiment 77. The compound of embodiment 76, wherein the modified nucleosides of the modified oligonucleotide comprise the same modification as one another.
Embodiment 78. The compound of embodiment 77, wherein the modified nucleosides of the modified oligonucleotide each comprise the same 2'-substituted sugar moiety.
Embodiment 79. The compound of embodiment 78, wherein the 2'-substituted sugar moiety of the modified oligonucleotide is selected from 2'-F, 2'-OMe, and 2'-MOE.
Embodiment 80. The compound of embodiment 78, wherein the 2'-substituted sugar moiety of the modified oligonucleotide is 2'-MOE.
Embodiment 81. The compound of embodiment 77, wherein the modified nucleosides of the modified oligonucleotide each comprise the same bicyclic sugar moiety.
Embodiment 82. The compound of embodiment 81, wherein the bicyclic sugar moiety of the modified oligonucleotide is selected from LNA and cEt.
Embodiment 83. The compound of embodiment 77, wherein the modified nucleosides of the modified oligonucleotide each comprises a sugar surrogate.
Embodiment 84. The compound of embodiment 83, wherein the sugar surrogate of the modified oligonucleotide is a morpholino.
Embodiment 85. The compound of embodiment 83, wherein the sugar surrogate of the modified oligonucleotide is a modified morpholino.
Embodiment 86. The compound of any of embodiments 1 to 85, wherein the modified oligonucleotide comprises at least one modified internucleoside linkage.
Embodiment 87. The compound of embodiment 86, wherein each internucleoside linkage is a modified internucleoside linkage.
Embodiment 88. The compound of embodiment 86 or 87, comprising at least one phosphorothioate internucleoside linkage.
Embodiment 89. The compound of embodiment 86, wherein each internucleoside linkage is a modified internucleoside linkage and wherein each internucleoside linkage comprises the same modification.
Embodiment 90. The compound of embodiment 89, wherein each internucleoside linkage is a phosphorothioate internucleoside linkage.
Embodiment 91. The compound of any of embodiments 1 to 90, comprising at least one conjugate.
Embodiment 92. The compound of any of embodiments 1 to 91 consisting of the modified oligonucleotide.
Embodiment 93. The compound of any of embodiments 1 to 92, wherein the compound modulates splicing of the CLN3 transcript.
Embodiment 94. The compound of any of embodiments 1 to 93, having a nucleobase sequence comprising any of the sequences as set forth in SEQ ID NOs. 3 to 60.
Embodiment 95. The compound of any of embodiments 1, 2, 4 to 5, or 8 to 93, having a nucleobase sequence comprising any of the sequences as set forth in SEQ ID NOs. 8, 16, 17, 18, 19, 20, 21, 27, 29, or 30.
Embodiment 96. The compound of any of embodiments 1, 3, or 6 to 93, having a nucleobase sequence comprising any of the sequences as set forth in SEQ ID NOs. 38, 39, 40, 41, 43, 55, 57, or 58.
Embodiment 97. The compound of any of embodiments 1, 2, or 8 to 93, having a nucleobase sequence comprising SEQ ID NO. 20.
Embodiment 98. A pharmaceutical composition comprising a compound according to any of embodiments 1-97 and a pharmaceutically acceptable carrier or diluent.
Embodiment 99. The pharmaceutical composition of embodiment 98, wherein the pharmaceutically acceptable carrier or diluent is sterile saline.
Embodiment 100. A method of modulating splicing of a CLN3 transcript in a cell comprising contacting the cell with a compound according to any of embodiments 1-99.
Embodiment 101. The method of embodiment 100, wherein the cell is in vitro.
Embodiment 102. The method of embodiment 100, wherein the cell is in an animal.
Embodiment 103. The method of any of embodiments 100 to 102, wherein the amount of CLN3 mRNA without exon 6 is increased.
Embodiment 104. The method of any of embodiments 100 to 102, wherein the amount of CLN3 mRNA without exon 9 is increased.
Embodiment 105. The method of any of embodiments 100 to 102, wherein the amount of CLN3 mRNA with exon 10 is increased.
Embodiment 106. The method of any of embodiments 100 to 105, wherein the CLN3 transcript is transcribed from a *CLN3*Δ*78* gene.
Embodiment 107. A method of modulating the expression of CLN3 in a cell, comprising contacting the cell with a compound according to any of embodiments 1-99.
Embodiment 108. The method of embodiment 84, wherein the cell is in vitro.
Embodiment 109. The method of embodiment 84, wherein the cell is in an animal.
Embodiment 110. A method comprising administering the compound according to any of embodiments 1-97 or the pharmaceutical composition of embodiments 98 or 99 to an animal.
Embodiment 111. The method of embodiment 108, wherein the administration is intracerebroventricular.
Embodiment 112. The method of embodiment 108, wherein the administration is into the central nervous sysem.
Embodiment 113. The method of any of embodiments 108-110, wherein the animal has one or more symptoms associated with Batten Disease.
Embodiment 114. The method of any of embodiments 108-110, wherein the administration results in amelioration of at least one symptom of Batten Disease.
Embodiment 115. The method of any of embodiments 108 to 112, wherein the animal is a mouse.
Embodiment 116. The method of any of embodiments 108 to 112, wherein the animal is a human.
Embodiment 117. A method of preventing or slowing one or more symptoms Batten Disease, comprising administering the compound according to any of embodiments 1-97 or the pharmaceutical composition of embodiments 98 or 99 to an animal in need thereof.
Embodiment 118. Use of the compound according to any of embodiments 1-97 or the pharmaceutical composition of embodiments 98 or 99 for the preparation of a medicament for use in the treatment of Batten Disease.
Embodiment 119. Use of the compound according to any of embodiments 1-97 or the pharmaceutical composition of embodiments 98 or 99 for the preparation of a medicament for use in the amelioration of one or more symptoms Batten Disease.
Embodiment 120. A compound comprising a modified oligonucleotide consisting of 8 to 30 linked nucleosides and having a nucleobase sequence comprising a complementary region, wherein the complementary region comprises at least 8 contiguous nucleobases and is complementary to an equal-length portion of a target region of a CLN3 transcript.
Embodiment 121. The compound of embodiment 120, wherein the CLN3 transcript comprises the nucleobase sequence of SEQ ID NO. 1.
Embodiment 122. The compound of embodiment 120 or 121, wherein the complementary region of the modified oligonucleotide is 100% complementary to the target region.
Embodiment 123. The compound of any of embodiments 120 to 122, wherein the complementary region of the modified oligonucleotide comprises at least 10 contiguous nucleobases.
Embodiment 124. The compound of any of embodiments 120 to 122, wherein the complementary region of the modified oligonucleotide comprises at least 12 contiguous nucleobases.
Embodiment 125. The compound of any of embodiments 120 to 122, wherein the complementary region of the modified oligonucleotide comprises at least 14 contiguous nucleobases.
Embodiment 126. The compound of any of embodiments 120 to 122, wherein the complementary region of the modified oligonucleotide comprises at least 15 contiguous nucleobases.
Embodiment 127. The compound of any of embodiments 120 to 122, wherein the complementary region of the modified oligonucleotide comprises at least 16 contiguous nucleobases.
Embodiment 128. The compound of any of embodiments 120 to 122, wherein the complementary region of the modified oligonucleotide comprises at least 17 contiguous nucleobases.
Embodiment 129. The compound of any of embodiments 120 to 122, wherein the complementary region of the modified oligonucleotide comprises at least 18 contiguous nucleobases.
Embodiment 130. The compound of any of embodiments 120 to 129, wherein the nucleobase sequence of the modified oligonucleotide is at least 80% complementary to an equal-length region of the CLN3 transcript, as measured over the entire length of the oligonucleotide.
Embodiment 131. The compound of any of embodiments 120 to 129, wherein the nucleobase sequence of the modified oligonucleotide is at least 90% complementary to an equal-length region of the CLN3 transcript, as measured over the entire length of the oligonucleotide.
Embodiment 132. The compound of any of embodiments 120 to 129, wherein the nucleobase sequence of the modified oligonucleotide is 100% complementary to an equal-length region of the CLN3 transcript, as measured over the entire length of the oligonucleotide.
Embodiment 133. The compound of any of embodiments 120-132, wherein the target region is within nucleobase 5704-5721, 5709-5726, 5714-5731, 5734-5751, 5764-5781, 5769-5786, 5774-5791, 5779-5796, 5784-5801, 5789-5806, 5794-5811, 5799-5816, 5804-5821, 5809-5826, 5814-5831, 5819-5836, 5824-5841, 5829-5846, 5834-5851, 5839-5856, 5844-5861, 5849-5866, 5854-5871, 5859-5876, 5879-5896, 5884-5901, or 5889-5906 of SEQ ID NO.: 1.
Embodiment 134. The compound of any of embodiments 120-133, wherein the target region is within nucleobase 5784- 5801 of SEQ ID NO.: 1.
Embodiment 135. The compound of any of embodiments 120-132, wherein the target region is within nucleobase 5804-5821 of SEQ ID NO.: 1.
Embodiment 136. The compound of any of embodiments 120-132, wherein the target region is within nucleobase 9122-9139, 9127-9144, 9132-9149, 9137-9154, 9142-9159, 9147-9164, 9152-9169, 9157-9174, 9162-9179, 9167-9184, 9172-9189, 9177-9194, 9182-9199, 9187-9204, 9192-9209, 9197-9214, 9202-9219, 9207-9224, 9212-9229, 9217-9234, 9222-9239, 9227-9244, 9232-9249, 9237-9254, 9242-9259, 9262-9279, 9282-9299, 9287-9304, 9292-9309, 9297-9314, 9302-9319, 9307-9324, 9312-9329, 9317-9334, 9322-9339, 9327-9344, 9332-9349, or 9337-9354 of SEQ ID NO.: 1.
Embodiment 137. The compound of any of embodiments 120-132, wherein the modified oligonucleotide has a nucleobase sequence comprising any of the sequences as set forth in SEQ ID NOs: 63, 64, 65, 66, 67, 68, 69, 70, 71, 72, 73, 74, 75, 76, 77, 78, 79, 80, 81, 82, 83, 84, 85, 86, 87, 88, 89, or 90.
Embodiment 138. The compound of any of embodiments 120-132, wherein the modified oligonucleotide has a nucleobase sequence consisting of the nucleobase sequence of any one of SEQ ID NOs: 63, 64, 65, 66, 67, 68, 69, 70, 71, 72, 73, 74, 75, 76, 77, 78, 79, 80, 81, 82, 83, 84, 85, 86, 87, 88, 89, or 90.
Embodiment 139. The compound of any of embodiments 120-132, wherein the modified oligonucleotide has a nucleobase sequence comprising the nucleobase sequence of SEQ ID NO. 72.
Embodiment 140. The compound of embodiment 138, wherein the modified oligonucleotide has a nucleobase sequence consisting of the nucleobase sequence of SEQ ID NO. 72.
Embodiment 141. The compound of any of embodiments 120-132, wherein the modified oligonucleotide has a nucleobase sequence comprising the nucleobase sequence of SEQ ID NO. 73.
Embodiment 142. The compound of embodiment 138, wherein the modified oligonucleotide has a nucleobase sequence consisting of the nucleobase sequence of SEQ ID NO. 73.
Embodiment 143. The compound of any of embodiments 120-132, wherein the modified oligonucleotide has a nucleobase sequence comprising any of the sequences as set forth in SEQ ID NOs: 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, or 127.
Embodiment 144. The compound of any of embodiments 120-132, wherein the modified oligonucleotide has a nucleobase sequence consisting of the nucleobase sequence of any one of SEQ ID NOs: 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, or 127.
Embodiment 145. The compound of any of embodiments 120-132, wherein the modified oligonucleotide has a nucleobase sequence comprising the nucleobase sequence of SEQ ID NO. 112.
Embodiment 146. The compound of embodiment 144, wherein the modified oligonucleotide has a nucleobase sequence consisting of the nucleobase sequence of SEQ ID NO. 112.
Embodiment 147. The compound of any of embodiments 120-146, wherein the modified oligonucleotide comprises at least one modified nucleoside.
Embodiment 148. The compound of any of embodiments 120-146, wherein each nucleoside of the modified oligonucleotide is a modified nucleoside selected from among: 2'-OMe, 2'-F, and 2'-MOE or a sugar surrogate.
Embodiment 149. The compound of embodiment 148, wherein the modified nucleoside is 2'-MOE.
Embodiment 150. The compound of embodiment 148, wherein the modified nucleoside is a morpholino.
Embodiment 151. The compound of embodiment 147, wherein at least one modified nucleoside comprises a modified sugar moiety.
Embodiment 152. The compound of embodiment 151, wherein at least one modified sugar moiety is a 2'-substituted sugar moiety.
Embodiment 153. The compound of embodiment 152, wherein the 2'-substitutent of at least one 2'-substituted sugar moiety is selected from among: 2'-OMe, 2'-F, and 2'-MOE.
Embodiment 154. The compound of any of embodiments 152-153, wherein the 2'-substiuent of at least one 2'-substituted sugar moiety is a 2'-MOE.
Embodiment 155. The compound of any of embodiments 120-154, wherein at least one modified sugar moiety is a bicyclic sugar moiety.
Embodiment 156. The compound of embodiment 155, wherein at least one bicyclic sugar moiety is LNA or cEt.
Embodiment 157. The compound of any of embodiments 120-1563, wherein at least one sugar moiety is a sugar surrogate.
Embodiment 158. The compound of embodiment 157, wherein at least one sugar surrogate is a morpholino.
Embodiment 159. The compound of embodiment 158, wherein at least one sugar surrogate is a modified morpholino.
Embodiment 160. The compound of any of embodiments 120-159, wherein the modified oligonucleotide comprises at least 5 modified nucleosides, each independently comprising a modified sugar moiety.
Embodiment 161. The compound of any of embodiments 120-159, wherein the modified oligonucleotide comprises at least 10 modified nucleosides, each independently comprising a modified sugar moiety.
Embodiment 162. The compound of any of embodiments 120-159, wherein the modified oligonucleotide comprises at least 15 modified nucleosides, each independently comprising a modified sugar moiety.
Embodiment 163. The compound of any of embodiments 120-159, wherein each nucleoside of the modified oligonucleotide is a modified nucleoside, each independently comprising a modified sugar moiety.
Embodiment 164. The compound of any of embodiments 120-163, wherein the modified oligonucleotide comprises at least two modified nucleosides comprising modified sugar moieties that are the same as one another.
Embodiment 165. The compound of any of embodiments 120-163, wherein the modified oligonucleotide comprises at least two modified nucleosides comprising modified sugar moieties that are different from one another.
Embodiment 166. The compound of any of embodiments 120-165, wherein the modified oligonucleotide comprises a modified region of at least 5 contiguous modified nucleosides.
Embodiment 167. The compound of any of embodiments 120-165, wherein the modified oligonucleotide comprises a modified region of at least 10 contiguous modified nucleosides.
Embodiment 168. The compound of any of embodiments 120-165, wherein the modified oligonucleotide comprises a modified region of at least 15 contiguous modified nucleosides.
Embodiment 169. The compound of any of embodiments 120-165, wherein the modified oligonucleotide comprises a modified region of at least 16 contiguous modified nucleosides.
Embodiment 170. The compound of any of embodiments 120-165, wherein the modified oligonucleotide comprises a modified region of at least 17 contiguous modified nucleosides.
Embodiment 171. The compound of any of embodiments 120-165, wherein the modified oligonucleotide comprises a modified region of at least 18 contiguous modified nucleosides.
Embodiment 172. The compound of any of embodiments 120-165, wherein the modified oligonucleotide comprises a modified region of at least 20 contiguous modified nucleosides.
Embodiment 173. The compound of any of embodiments 166 to 1 72, wherein each modified nucleoside of the modified region has a modified sugar moiety independently selected from among: 2'-F, 2'-OMe, 2'-MOE, cEt, LNA, morpholino, and modified morpholino.
Embodiment 174. The compound of any of embodiments 166 to 173, wherein the modified nucleosides of the modified region each comprise the same modification as one another.
Embodiment 175. The compound of embodiment 174, wherein the modified nucleosides of the modified region each comprise the same 2'-substituted sugar moiety.
Embodiment 176. The compound of embodiment 174, wherein the 2'-substituted sugar moiety of the modified nucleosides of the region of modified nucleosides is selected from 2'-F, 2'-OMe, and 2'-MOE.
Embodiment 177. The compound of embodiment 174, wherein the 2'-substituted sugar moiety of the modified nucleosides of the region of modified nucleosides is 2'-MOE.
Embodiment 178. The compound of embodiment 174, wherein the modified nucleosides of the region of modified nucleosides each comprise the same bicyclic sugar moiety.
Embodiment 179. The compound of embodiment 178, wherein the bicyclic sugar moiety of the modified nucleosides of the region of modified nucleosides is selected from LNA and cEt.
Embodiment 180. The compound of embodiment 174, wherein the modified nucleosides of the region of modified nucleosides each comprises a sugar surrogate.
Embodiment 181. The compound of embodiment 180, wherein the sugar surrogate of the modified nucleosides of the region of modified nucleosides is a morpholino.
Embodiment 182. The compound of embodiment 180, wherein the sugar surrogate of the modified nucleosides of the region of modified nucleosides is a modified morpholino.
Embodiment 183. The compound of any of embodiments 120 to 182, wherein the modified nucleotide comprises no more than 4 contiguous naturally occurring nucleosides.
Embodiment 184. The compound of any of embodiments 120 to 183, wherein each nucleoside of the modified oligonucleotide is a modified nucleoside.
Embodiment 185. The compound of embodiment 184 wherein each modified nucleoside comprises a modified sugar moiety.
Embodiment 186. The compound of embodiment 185, wherein the modified nucleosides of the modified oligonucleotide comprise the same modification as one another.
Embodiment 187. The compound of embodiment 186, wherein the modified nucleosides of the modified oligonucleotide each comprise the same 2'-substituted sugar moiety.
Embodiment 188. The compound of embodiment 187, wherein the 2'-substituted sugar moiety of the modified oligonucleotide is selected from 2'-F, 2'-OMe, and 2'-MOE.
Embodiment 189. The compound of embodiment 187, wherein the 2'-substituted sugar moiety of the modified oligonucleotide is 2'-MOE.
Embodiment 190. The compound of embodiment 186, wherein the modified nucleosides of the modified oligonucleotide each comprise the same bicyclic sugar moiety.
Embodiment 191. The compound of embodiment 190, wherein the bicyclic sugar moiety of the modified oligonucleotide is selected from LNA and cEt.
Embodiment 192. The compound of embodiment 186, wherein the modified nucleosides of the modified oligonucleotide each comprises a sugar surrogate.
Embodiment 193. The compound of embodiment 192, wherein the sugar surrogate of the modified oligonucleotide is a morpholino.
Embodiment 194. The compound of embodiment 192, wherein the sugar surrogate of the modified oligonucleotide is a modified morpholino.
Embodiment 195. The compound of any of embodiments 120 to 194, wherein the modified oligonucleotide comprises at least one modified internucleoside linkage.
Embodiment 196. The compound of embodiment 195, wherein each internucleoside linkage is a modified internucleoside linkage.
Embodiment 197. The compound of embodiment 195 or 196, comprising at least one phosphorothioate internucleoside linkage.
Embodiment 198. The compound of embodiment 196, wherein each internucleoside linkage is a modified internucleoside linkage and wherein each internucleoside linkage comprises the same modification.
Embodiment 199. The compound of embodiment 198, wherein each internucleoside linkage is a phosphorothioate internucleoside linkage.
Embodiment 200. The compound of any of embodiments 120 to 200, comprising at least one conjugate.
Embodiment 201. The compound of any of embodiments 120 to 200, consisting of the modified oligonucleotide.
Embodiment 202. The compound of any of embodiments 120 to 201, wherein the compound modulates splicing of the CLN3 transcript.
Embodiment 203. A pharmaceutical composition comprising a compound according to any of embodiments 120-202 and a pharmaceutically acceptable carrier or diluent.
Embodiment 204. The pharmaceutical composition of embodiment 202, wherein the pharmaceutically acceptable carrier or diluent is sterile saline.
Embodiment 205. A method of modulating splicing of a CLN3 transcript in a cell comprising contacting the cell with a compound according to any of embodiments 120-204.
Embodiment 206. The method of embodiment 205, wherein the cell is in vitro.
Embodiment 207. The method of embodiment 205, wherein the cell is in an animal.
Embodiment 208. The method of any of embodiments 205 to 207, wherein the amount of CLN3 mRNA without exon 6 is increased.
Embodiment 209. The method of any of embodiments 205 to 207, wherein the amount of CLN3 mRNA without exon 9 is increased.
Embodiment 210. The method of any of embodiments 205 to 209, wherein the amount of CLN3 mRNA with exon 10 is increased.
Embodiment 211. The method of any of embodiments 205 to 210, wherein the CLN3 transcript is transcribed from a *CLN3*Δ*78* gene.
Embodiment 212. A method of modulating the expression of CLN3 in a cell, comprising contacting the cell with a compound according to any of embodiments 120-204.
Embodiment 213. The method of embodiment 212, wherein the cell is in vitro.
Embodiment 214. The method of embodiment 212, wherein the cell is in an animal.
Embodiment 215. A method comprising administering the compound of any of embodiments 120-204 to an animal.
Embodiment 216. The method of embodiment 215, wherein the administration is intracerebroventricular.
Embodiment 217. The method of embodiment 215, wherein the administration is into the central nervous sysem.
Embodiment 218. The method of any of embodiments 215 to 217, wherein the animal has one or more symptoms associated with Batten Disease.
Embodiment 219. The method of any of embodiments 215 to 217, wherein the administration results in amelioration of at least one symptom of Batten Disease.
Embodiment 220. The method of any of embodiments 215 to 219, wherein the animal is a mouse.
Embodiment 221. The method of any of embodiments 215 to 219, wherein the animal is a human.
Embodiment 222. A method of preventing or slowing one or more symptoms Batten Disease, comprising administering the compound according to any of embodiments 120-204 to an animal in need thereof.
Embodiment 223. The method of embodiment 222, wherein the animal is a human.
Embodiment 224. Use of the compound according to any of embodiments 120-204 for the preparation of a medicament for use in the treatment of Batten Disease.
Embodiment 225. Use of the compound according to any of embodiments 120-204 for the preparation of a medicament for use in the amelioration of one or more symptoms Batten Disease.

### BRIEF DESCRIPTION OF THE FIGURES

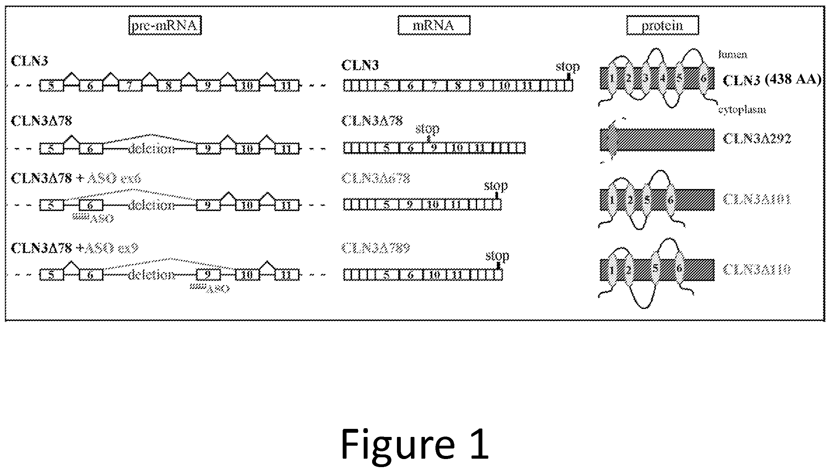

Figure 1 is a schematic of the human WT CLN3 and mutant *CLN3*Δ*78* RNA and proteins. The splice products, CLN3Δ678 and CLN3Δ789, are depicted as well. Exons are depicted as boxes, introns are depicted as lines. In certain embodiments, ASOs are used to alter the splicing of *CLN3*Δ*78* pre-mRNA.
Figure 2 shows splice products produced following treatment of WT, heterozygous, and homozygous mutant CLN3Δ78 fibroblasts with an ASO targeted to exon 6 of CLN3 and analysis by RT-PCR.
Figure 3 shows splice products produced following treatment of homozygous mutant CLN3Δ78 fibroblasts with varying concentrations of an ASO targeted to exon 6 of CLN3 and analysis by RT-PCR.
Figure 4 shows a western blot illustrating protein products produced following treatment of WT, heterozygous, and homozygous mutant CLN3Δ78 fibroblasts with an ASO targeted to exon 6 and a control ASO.
Figure 5 shows the RT-PCR results of a screen of ASOs targeted to exon 6 of mouse CLN3.
Figure 6 shows the RT-PCR results of a screen of ASOs targeted to exon 9 of mouse CLN3.
Figure 7 shows splice products produced following treatment of homozygous mutant CLN3Δ78 Batten mice with an ASO targeted to exon 6 of CLN3 (616709) or a control ASO and analysis by RT-PCR.
Figure 8 shows the RT-PCR results of a screen of ASOs targeted to exon 6 of human CLN3.
Figure 9 shows the RT-PCR results of a screen of ASOs targeted to exon 9 of human CLN3.
Figure 10 shows splice products produced following treatment of homozygous mutant CLN3Δ78 fibroblasts with ASOs targeted to exon 6 or exon 9 of human CLN3.

### DETAILED DESCRIPTION

Unless specific definitions are provided, the nomenclature used in connection with, and the procedures and techniques of, analytical chemistry, synthetic organic chemistry, and medicinal and pharmaceutical chemistry described herein are those well known and commonly used in the art. Standard techniques may be used for chemical synthesis, and chemical analysis. Certain such techniques and procedures may be found for example in "Carbohydrate Modifications in Antisense Research" Edited by Sangvi and Cook, American Chemical Society , Washington D.C., 1994; "Remington's Pharmaceutical Sciences," Mack Publishing Co., Easton, Pa., 21st edition, 2005; and "Antisense Drug Technology, Principles, Strategies, and Applications" Edited by Stanley T. Crooke, CRC Press, Boca Raton, Florida; and Sambrook et al., "Molecular Cloning, A laboratory Manual," 2nd Edition, Cold Spring Harbor Laboratory Press, 1989, which are hereby incorporated by reference for any purpose. Where permitted, all patents, applications, published applications and other publications and other data referred to throughout in the disclosure are incorporated by reference herein in their entirety.

Unless otherwise indicated, the following terms have the following meanings:
As used herein, "nucleoside" means a compound comprising a nucleobase moiety and a sugar moiety. Nucleosides include, but are not limited to, naturally occurring nucleosides (as found in DNA and RNA) and modified nucleosides. Nucleosides may be linked to a phosphate moiety.

As used herein, "chemical modification" means a chemical difference in a compound when compared to a naturally occurring counterpart. In reference to an oligonucleotide, chemical modification does not include differences only in nucleobase sequence. Chemical modifications of oligonucleotides include nucleoside modifications (including sugar moiety modifications and nucleobase modifications) and internucleoside linkage modifications.

As used herein, "furanosyl" means a structure comprising a 5-membered ring comprising four carbon atoms and one oxygen atom.

As used herein, "naturally occurring sugar moiety" means a ribofuranosyl as found in naturally occurring RNA or a deoxyribofuranosyl as found in naturally occurring DNA.

As used herein, "sugar moiety" means a naturally occurring sugar moiety or a modified sugar moiety of a nucleoside.

As used herein, "modified sugar moiety" means a substituted sugar moiety, a bicyclic or tricyclic sugar moiety, or a sugar surrogate.

As used herein, "substituted sugar moiety" means a furanosyl comprising at least one substituent group that differs from that of a naturally occurring sugar moiety. Substituted sugar moieties include, but are not limited to furanosyls comprising substituents at the 2'-position, the 3'-position, the 5'-position and/or the 4'-position.

As used herein, "2'-substituted sugar moiety" means a furanosyl comprising a substituent at the 2'-position other than H or OH. Unless otherwise indicated, a 2'-substituted sugar moiety is not a bicyclic sugar moiety (i.e., the 2'-substituent of a 2'-substituted sugar moiety does not form a bridge to another atom of the furanosyl ring.

As used herein, "MOE" means -OCH₂CH₂OCH₃.

As used herein, "bicyclic sugar moiety" means a modified sugar moiety comprising a 4 to 7 membered ring (including but not limited to a furanosyl) comprising a bridge connecting two atoms of the 4 to 7 membered ring to form a second ring, resulting in a bicyclic structure. In certain embodiments, the 4 to 7 membered ring is a sugar ring. In certain embodiments the 4 to 7 membered ring is a furanosyl. In certain such embodiments, the bridge connects the 2'-carbon and the 4'-carbon of the furanosyl.

As used herein the term "sugar surrogate" means a structure that does not comprise a furanosyl and that is capable of replacing the naturally occurring sugar moiety of a nucleoside, such that the resulting nucleoside is capable of (1) incorporation into an oligonucleotide and (2) hybridization to a complementary nucleoside. Such structures include rings comprising a different number of atoms than furanosyl (e.g., 4, 6, or 7-membered rings); replacement of the oxygen of a furanosyl with a non-oxygen atom (e.g., carbon, sulfur, or nitrogen); or both a change in the number of atoms and a replacement of the oxygen. Such structures may also comprise substitutions corresponding to those described for substituted sugar moieties (e.g., 6-membered carbocyclic bicyclic sugar surrogates optionally comprising additional substituents). Sugar surrogates also include more complex sugar replacements (e.g., the non-ring systems of peptide nucleic acid). Sugar surrogates include without limitation morpholino, modified morpholinos, cyclohexenyls and cyclohexitols.

As used herein, "nucleotide" means a nucleoside further comprising a phosphate linking group. As used herein, "linked nucleosides" may or may not be linked by phosphate linkages and thus includes, but is not limited to "linked nucleotides." As used herein, "linked nucleosides" are nucleosides that are connected in a continuous sequence (i.e. no additional nucleosides are present between those that are linked).

As used herein, "nucleobase" means a group of atoms that can be linked to a sugar moiety to create a nucleoside that is capable of incorporation into an oligonucleotide, and wherein the group of atoms is capable of bonding with a complementary naturally occurring nucleobase of another oligonucleotide or nucleic acid. Nucleobases may be naturally occurring or may be modified.

As used herein, "heterocyclic base" or "heterocyclic nucleobase" means a nucleobase comprising a heterocyclic structure.

As used herein the terms, "unmodified nucleobase" or "naturally occurring nucleobase" means the naturally occurring heterocyclic nucleobases of RNA or DNA: the purine bases adenine (A) and guanine (G), and the pyrimidine bases thymine (T), cytosine (C) (including 5-methyl C), and uracil (U).

As used herein, "modified nucleobase" means any nucleobase that is not a naturally occurring nucleobase.

As used herein, "modified nucleoside" means a nucleoside comprising at least one chemical modification compared to naturally occurring RNA or DNA nucleosides. Modified nucleosides comprise a modified sugar moiety and/or a modified nucleobase.

As used herein, "bicyclic nucleoside" or "BNA" means a nucleoside comprising a bicyclic sugar moiety.

As used herein, "constrained ethyl nucleoside" or "cEt" means a nucleoside comprising a bicyclic sugar moiety comprising a 4'-CH(CH₃)-O-2'bridge.

As used herein, "locked nucleic acid nucleoside" or "LNA" means a nucleoside comprising a bicyclic sugar moiety comprising a 4'-CH₂-O-2'bridge.

As used herein, "2'-substituted nucleoside" means a nucleoside comprising a substituent at the 2'-position other than H or OH. Unless otherwise indicated, a 2'-substituted nucleoside is not a bicyclic nucleoside.

As used herein, "2'-deoxynucleoside" means a nucleoside comprising 2'-H furanosyl sugar moiety, as found in naturally occurring deoxyribonucleosides (DNA). In certain embodiments, a 2'-deoxynucleoside may comprise a modified nucleobase or may comprise an RNA nucleobase (e.g., uracil).

As used herein, "oligonucleotide" means a compound comprising a plurality of linked nucleosides. In certain embodiments, an oligonucleotide comprises one or more unmodified ribonucleosides (RNA) and/or unmodified deoxyribonucleosides (DNA) and/or one or more modified nucleosides.

As used herein "oligonucleoside" means an oligonucleotide in which none of the internucleoside linkages contains a phosphorus atom. As used herein, oligonucleotides include oligonucleosides.

As used herein, "modified oligonucleotide" means an oligonucleotide comprising at least one modified nucleoside and/or at least one modified internucleoside linkage.

As used herein "internucleoside linkage" means a covalent linkage between adjacent nucleosides in an oligonucleotide.

As used herein "naturally occurring internucleoside linkage" means a 3' to 5' phosphodiester linkage.

As used herein, "modified internucleoside linkage" means any internucleoside linkage other than a naturally occurring internucleoside linkage.

As used herein, "oligomeric compound" means a polymeric structure comprising two or more substructures. In certain embodiments, an oligomeric compound comprises an oligonucleotide. In certain embodiments, an oligomeric compound comprises one or more conjugate groups and/or terminal groups. In certain embodiments, an oligomeric compound consists of an oligonucleotide.

As used herein, "terminal group" means one or more atom attached to either, or both, the 3' end or the 5' end of an oligonucleotide. In certain embodiments a terminal group is a conjugate group. In certain embodiments, a terminal group comprises one or more terminal group nucleosides.

As used herein, "conjugate" means an atom or group of atoms bound to an oligonucleotide or oligomeric compound. In general, conjugate groups modify one or more properties of the compound to which they are attached, including, but not limited to pharmacodynamic, pharmacokinetic, binding, absorption, cellular distribution, cellular uptake, charge and/or clearance properties.

As used herein, "conjugate linking group" means any atom or group of atoms used to attach a conjugate to an oligonucleotide or oligomeric compound.

As used herein, "antisense compound" means a compound comprising or consisting of an oligonucleotide at least a portion of which is complementary to a target nucleic acid to which it is capable of hybridizing, resulting in at least one antisense activity.

As used herein, "antisense activity" means any detectable and/or measurable change attributable to the hybridization of an antisense compound to its target nucleic acid.

As used herein, "detecting" or "measuring" means that a test or assay for detecting or measuring is performed. Such detection and/or measuring may result in a value of zero. Thus, if a test for detection or measuring results in a finding of no activity (activity of zero), the step of detecting or measuring the activity has nevertheless been performed.

As used herein, "detectable and/or measureable activity" means a statistically significant activity that is not zero.

As used herein, "essentially unchanged" means little or no change in a particular parameter, particularly relative to another parameter which changes much more. In certain embodiments, a parameter is essentially unchanged when it changes less than 5%. In certain embodiments, a parameter is essentially unchanged if it changes less than two-fold while another parameter changes at least ten-fold. For example, in certain embodiments, an antisense activity is a change in the amount of a target nucleic acid. In certain such embodiments, the amount of a non-target nucleic acid is essentially unchanged if it changes much less than the target nucleic acid does, but the change need not be zero.

As used herein, "expression" means the process by which a gene ultimately results in a protein. Expression includes, but is not limited to, transcription, post-transcriptional modification (e.g., splicing, polyadenlyation, addition of 5'-cap), and translation.

As used herein, "target nucleic acid" means a nucleic acid molecule to which an antisense compound hybridizes.

As used herein, "mRNA" means an RNA molecule that encodes a protein.

As used herein, "pre-mRNA" means an RNA transcript that has not been fully processed into mRNA. Pre-RNA includes one or more intron.

As used herein, "transcript" means an RNA molecule transcribed from DNA. Transcripts include, but are not limitied to mRNA, pre-mRNA, and partially processed RNA.

As used herein, "CLN3" means ceroid-lipofuscinosis, neuronal 3.

As used herein, "CLN3 transcript" means a transcript transcribed from a CLN3 gene. In certain embodiments, a CLN3 transcript comprises SEQ ID NO: 1: the complement of GENBANK accession number NT_010393.16 truncated from nucleotides 28427600 to 28444620. In certain embodiments, a CLN3 transcript comprises SEQ ID NO: 2: the complement of GENBANK accession number NT_039433.8, truncated from nucleotides 44319075 to 44333955.

As used herein, "CLN3 gene" means a gene that encodes a ceroid-lipofuscinosis, neuronal 3 protein and any ceroid-lipofuscinosis, neuronal 3 protein isoforms. In certain embodiments, a CLN3 gene is represented by GENBANK accession number NT_010393.16 truncated from nucleotides 28427600 to 28444620, or a variant thereof. In certain embodiments, a CLN3 gene is at least 95% identical to GENBANK accession number NT_010393.16 truncated from nucleotides 28427600 to 28444620. In certain embodiments, a CLN3 gene is at least 90% identical to GENBANK accession number NT_010393.16 truncated from nucleotides 28427600 to 28444620. In certain embodiments, a CLN3 gene is represented by GENBANK accession number NT_039433.8, truncated from nucleotides 44319075 to 44333955, or a variant thereof. In certain embodiments, a CLN3 gene is at least 95% identical to GENBANK accession number NT_039433.8, truncated from nucleotides 44319075 to 44333955. In certain embodiments, a CLN3 gene is at least 90% identical to GENBANK accession number NT_039433.8, truncated from nucleotides 44319075 to 44333955. In certain embodiments, a CLN3 gene encodes a wild-type CLN3 protein. In certain embodiments, a CLN3 gene encodes a *CLN3*Δ*78* protein.

As used herein, "*CLN3*Δ*78*" means a CLN3 gene having a deletion spanning all or part of exons 7 and 8. In certain embodiments, the *CLN3*Δ*78* deletion causes a frameshift that result in a premature stop codon in exon 9. In certain embodiments, the truncated protein product of *CLN3*Δ*78* is 33% of the length of the wild type.

As used herein, "targeting" or "targeted to" means the association of an antisense compound to a particular target nucleic acid molecule or a particular region of a target nucleic acid molecule. An antisense compound targets a target nucleic acid if it is sufficiently complementary to the target nucleic acid to allow hybridization under physiological conditions.

As used herein, "nucleobase complementarity" or "complementarity" when in reference to nucleobases means a nucleobase that is capable of base pairing with another nucleobase. For example, in DNA, adenine (A) is complementary to thymine (T). For example, in RNA, adenine (A) is complementary to uracil (U). In certain embodiments, complementary nucleobase means a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair. Nucleobases comprising certain modifications may maintain the ability to pair with a counterpart nucleobase and thus, are still capable of nucleobase complementarity.

As used herein, "non-complementary" in reference to nucleobases means a pair of nucleobases that do not form hydrogen bonds with one another.

As used herein, "complementary" in reference to oligomeric compounds (e.g., linked nucleosides, oligonucleotides, or nucleic acids) means the capacity of such oligomeric compounds or regions thereof to hybridize to another oligomeric compound or region thereof through nucleobase complementarity under stringent conditions. Complementary oligomeric compounds need not have nucleobase complementarity at each nucleoside. Rather, some mismatches are tolerated. In certain embodiments, complementary oligomeric compounds or regions are complementary at 70% of the nucleobases (70% complementary). In certain embodiments, complementary oligomeric compounds or regions are 80% complementary. In certain embodiments, complementary oligomeric compounds or regions are 90% complementary. In certain embodiments, complementary oligomeric compounds or regions are 95% complementary. In certain embodiments, complementary oligomeric compounds or regions are 100% complementary.

As used herein, "hybridization" means the pairing of complementary oligomeric compounds (e.g., an antisense compound and its target nucleic acid). While not limited to a particular mechanism, the most common mechanism of pairing involves hydrogen bonding, which may be Watson-Crick, Hoogsteen or reversed Hoogsteen hydrogen bonding, between complementary nucleobases.

As used herein, "specifically hybridizes" means the ability of an oligomeric compound to hybridize to one nucleic acid site with greater affinity than it hybridizes to another nucleic acid site. In certain embodiments, an antisense oligonucleotide specifically hybridizes to more than one target site.

As used herein, "percent complementarity" means the percentage of nucleobases of an oligomeric compound that are complementary to an equal-length portion of a target nucleic acid. Percent complementarity is calculated by dividing the number of nucleobases of the oligomeric compound that are complementary to nucleobases at corresponding positions in the target nucleic acid by the total length of the oligomeric compound.

As used herein, "percent identity" means the number of nucleobases in a first nucleic acid that are the same type (independent of chemical modification) as nucleobases at corresponding positions in a second nucleic acid, divided by the total number of nucleobases in the first nucleic acid.

As used herein, "modulation" means a change of amount or quality of a molecule, function, or activity when compared to the amount or quality of a molecule, function, or activity prior to modulation. For example, modulation includes the change, either an increase (stimulation or induction) or a decrease (inhibition or reduction) in gene expression. As a further example, modulation of expression can include a change in splice site selection of pre-mRNA processing, resulting in a change in the absolute or relative amount of a particular splice-variant compared to the amount in the absence of modulation.

As used herein, "motif" means a pattern of chemical modifications in an oligomeric compound or a region thereof. Motifs may be defined by modifications at certain nucleosides and/or at certain linking groups of an oligomeric compound.

As used herein, "nucleoside motif" means a pattern of nucleoside modifications in an oligomeric compound or a region thereof. The linkages of such an oligomeric compound may be modified or unmodified. Unless otherwise indicated, motifs herein describing only nucleosides are intended to be nucleoside motifs. Thus, in such instances, the linkages are not limited.

As used herein, "sugar motif" means a pattern of sugar modifications in an oligomeric compound or a region thereof.

As used herein, "linkage motif" means a pattern of linkage modifications in an oligomeric compound or region thereof. The nucleosides of such an oligomeric compound may be modified or unmodified. Unless otherwise indicated, motifs herein describing only linkages are intended to be linkage motifs. Thus, in such instances, the nucleosides are not limited.

As used herein, "nucleobase modification motif" means a pattern of modifications to nucleobases along an oligonucleotide. Unless otherwise indicated, a nucleobase modification motif is independent of the nucleobase sequence.

As used herein, "sequence motif" means a pattern of nucleobases arranged along an oligonucleotide or portion thereof. Unless otherwise indicated, a sequence motif is independent of chemical modifications and thus may have any combination of chemical modifications, including no chemical modifications.

As used herein, "type of modification" in reference to a nucleoside or a nucleoside of a "type" means the chemical modification of a nucleoside and includes modified and unmodified nucleosides. Accordingly, unless otherwise indicated, a "nucleoside having a modification of a first type" may be an unmodified nucleoside.

As used herein, "differently modified" mean chemical modifications or chemical substituents that are different from one another, including absence of modifications. Thus, for example, a MOE nucleoside and an unmodified DNA nucleoside are "differently modified," even though the DNA nucleoside is unmodified. Likewise, DNA and RNA are "differently modified," even though both are naturally-occurring unmodified nucleosides. Nucleosides that are the same but for comprising different nucleobases are not differently modified. For example, a nucleoside comprising a 2'-OMe modified sugar and an unmodified adenine nucleobase and a nucleoside comprising a 2'-OMe modified sugar and an unmodified thymine nucleobase are not differently modified.

As used herein, "the same type of modifications" refers to modifications that are the same as one another, including absence of modifications. Thus, for example, two unmodified DNA nucleoside have "the same type of modification," even though the DNA nucleoside is unmodified. Such nucleosides having the same type modification may comprise different nucleobases.

As used herein, "pharmaceutically acceptable carrier or diluent" means any substance suitable for use in administering to an animal. In certain embodiments, a pharmaceutically acceptable carrier or diluent is sterile saline. In certain embodiments, such sterile saline is pharmaceutical grade saline.

As used herein, "substituent" and "substituent group," means an atom or group that replaces the atom or group of a named parent compound. For example a substituent of a modified nucleoside is any atom or group that differs from the atom or group found in a naturally occurring nucleoside (e.g., a modified 2'-substuent is any atom or group at the 2'-position of a nucleoside other than H or OH). Substituent groups can be protected or unprotected. In certain embodiments, compounds of the present invention have substituents at one or at more than one position of the parent compound. Substituents may also be further substituted with other substituent groups and may be attached directly or via a linking group such as an alkyl or hydrocarbyl group to a parent compound.

Likewise, as used herein, "substituent" in reference to a chemical functional group means an atom or group of atoms differs from the atom or a group of atoms normally present in the named functional group. In certain embodiments, a substituent replaces a hydrogen atom of the functional group (e.g., in certain embodiments, the substituent of a substituted methyl group is an atom or group other than hydrogen which replaces one of the hydrogen atoms of an unsubstituted methyl group). Unless otherwise indicated, groups amenable for use as substituents include without limitation, halogen, hydroxyl, alkyl, alkenyl, alkynyl, acyl (-C(O)Rₐₐ), carboxyl (-C(O)O-Rₐₐ), aliphatic groups, alicyclic groups, alkoxy, substituted oxy (-O-Rₐₐ), aryl, aralkyl, heterocyclic radical, heteroaryl, heteroarylalkyl, amino (-N(R_{bb})(R_{cc})), imino(=NR_{bb}), amido (-C(O)N(R_{bb})(R_{cc}) or -N(R_{bb})C(O)Rₐₐ), azido (-N₃), nitro (-NO₂), cyano (-CN), carbamido (-OC(O)N(R_{bb})(R_{cc}) or -N(R_{bb})C(O)ORₐₐ), ureido (-N(R_{bb})C(O)N(R_{bb})(R_{cc})), thioureido (-N(R_{bb})C(S)N(R_{bb})-(R_{cc})), guanidinyl (-N(R_{bb})C(=NR_{bb})N(R_{bb})(R_{cc})), amidinyl (-C(=NR_{bb})N(R_{bb})(R_{cc}) or -N(R_{bb})C(=NR_{bb})(Rₐₐ)), thiol (-SR_{bb}), sulfinyl (-S(O)R_{bb}), sulfonyl (-S(O)₂R_{bb}) and sulfonamidyl (-S(O)₂N(R_{bb})(R_{cc}) or -N(R_{bb})S-(O₂R_{bb}). Wherein each Rₐₐ, R_{bb} and R_{cc} is, independently, H, an optionally linked chemical functional group or a further substituent group with a preferred list including without limitation, alkyl, alkenyl, alkynyl, aliphatic, alkoxy, acyl, aryl, aralkyl, heteroaryl, alicyclic, heterocyclic and heteroarylalkyl. Selected substituents within the compounds described herein are present to a recursive degree.

As used herein, "alkyl," as used herein, means a saturated straight or branched hydrocarbon radical containing up to twenty four carbon atoms. Examples of alkyl groups include without limitation, methyl, ethyl, propyl, butyl, isopropyl, n-hexyl, octyl, decyl, dodecyl and the like. Alkyl groups typically include from 1 to about 24 carbon atoms, more typically from 1 to about 12 carbon atoms (C₁-C₁₂ alkyl) with from 1 to about 6 carbon atoms being more preferred.

As used herein, "alkenyl," means a straight or branched hydrocarbon chain radical containing up to twenty four carbon atoms and having at least one carbon-carbon double bond. Examples of alkenyl groups include without limitation, ethenyl, propenyl, butenyl, 1-methyl-2-buten-1-yl, dienes such as 1,3-butadiene and the like. Alkenyl groups typically include from 2 to about 24 carbon atoms, more typically from 2 to about 12 carbon atoms with from 2 to about 6 carbon atoms being more preferred. Alkenyl groups as used herein may optionally include one or more further substituent groups.

As used herein, "alkynyl," means a straight or branched hydrocarbon radical containing up to twenty four carbon atoms and having at least one carbon-carbon triple bond. Examples of alkynyl groups include, without limitation, ethynyl, 1-propynyl, 1 -butynyl, and the like. Alkynyl groups typically include from 2 to about 24 carbon atoms, more typically from 2 to about 12 carbon atoms with from 2 to about 6 carbon atoms being more preferred. Alkynyl groups as used herein may optionally include one or more further substituent groups.

As used herein, "acyl," means a radical formed by removal of a hydroxyl group from an organic acid and has the general Formula -C(O)-X where X is typically aliphatic, alicyclic or aromatic. Examples include aliphatic carbonyls, aromatic carbonyls, aliphatic sulfonyls, aromatic sulfinyls, aliphatic sulfinyls, aromatic phosphates, aliphatic phosphates and the like. Acyl groups as used herein may optionally include further substituent groups.

As used herein, "alicyclic" means a cyclic ring system wherein the ring is aliphatic. The ring system can comprise one or more rings wherein at least one ring is aliphatic. Preferred alicyclics include rings having from about 5 to about 9 carbon atoms in the ring. Alicyclic as used herein may optionally include further substituent groups.

As used herein, "aliphatic" means a straight or branched hydrocarbon radical containing up to twenty four carbon atoms wherein the saturation between any two carbon atoms is a single, double or triple bond. An aliphatic group preferably contains from 1 to about 24 carbon atoms, more typically from 1 to about 12 carbon atoms with from 1 to about 6 carbon atoms being more preferred. The straight or branched chain of an aliphatic group may be interrupted with one or more heteroatoms that include nitrogen, oxygen, sulfur and phosphorus. Such aliphatic groups interrupted by heteroatoms include without limitation, polyalkoxys, such as polyalkylene glycols, polyamines, and polyimines. Aliphatic groups as used herein may optionally include further substituent groups.

As used herein, "alkoxy" means a radical formed between an alkyl group and an oxygen atom wherein the oxygen atom is used to attach the alkoxy group to a parent molecule. Examples of alkoxy groups include without limitation, methoxy, ethoxy, propoxy, isopropoxy, n-butoxy, sec-butoxy, tert-butoxy, n-pentoxy, neopentoxy, n-hexoxy and the like. Alkoxy groups as used herein may optionally include further substituent groups.

As used herein, "aminoalkyl" means an amino substituted C₁-C₁₂ alkyl radical. The alkyl portion of the radical forms a covalent bond with a parent molecule. The amino group can be located at any position and the aminoalkyl group can be substituted with a further substituent group at the alkyl and/or amino portions.

As used herein, "aralkyl" and "arylalkyl" mean an aromatic group that is covalently linked to a C₁-C₁₂ alkyl radical. The alkyl radical portion of the resulting aralkyl (or arylalkyl) group forms a covalent bond with a parent molecule. Examples include without limitation, benzyl, phenethyl and the like. Aralkyl groups as used herein may optionally include further substituent groups attached to the alkyl, the aryl or both groups that form the radical group.

As used herein, "aryl" and "aromatic" mean a mono- or polycyclic carbocyclic ring system radicals having one or more aromatic rings. Examples of aryl groups include without limitation, phenyl, naphthyl, tetrahydronaphthyl, indanyl, idenyl and the like. Preferred aryl ring systems have from about 5 to about 20 carbon atoms in one or more rings. Aryl groups as used herein may optionally include further substituent groups.

As used herein, "halo" and "halogen," mean an atom selected from fluorine, chlorine, bromine and iodine.

As used herein, "heteroaryl," and "heteroaromatic," mean a radical comprising a mono- or polycyclic aromatic ring, ring system or fused ring system wherein at least one of the rings is aromatic and includes one or more heteroatoms. Heteroaryl is also meant to include fused ring systems including systems where one or more of the fused rings contain no heteroatoms. Heteroaryl groups typically include one ring atom selected from sulfur, nitrogen or oxygen. Examples of heteroaryl groups include without limitation, pyridinyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, benzimidazolyl, benzooxazolyl, quinoxalinyl and the like. Heteroaryl radicals can be attached to a parent molecule directly or through a linking moiety such as an aliphatic group or hetero atom. Heteroaryl groups as used herein may optionally include further substituent groups.

### Oligomeric Compounds

In certain embodiments, the present invention provides oligomeric compounds. In certain embodiments, such oligomeric compounds comprise oligonucleotides optionally comprising one or more conjugate and/or terminal groups. In certain embodiments, an oligomeric compound consists of an oligonucleotide. In certain embodiments, oligonucleotides comprise one or more chemical modifications. Such chemical modifications include modifications one or more nucleoside (including modifications to the sugar moiety and/or the nucleobase) and/or modifications to one or more internucleoside linkage.

### Certain Sugar Moieties

In certain embodiments, oligomeric compounds of the invention comprise one or more modifed nucleosides comprising a modifed sugar moiety. Such oligomeric compounds comprising one or more sugar-modified nucleosides may have desirable properties, such as enhanced nuclease stability or increased binding affinity with a target nucleic acid relative to oligomeric compounds comprising only nucleosides comprising naturally occurring sugar moieties. In certain embodiments, modified sugar moieties are substitued sugar moieties. In certain embodiments, modified sugar moieties are bicyclic or tricyclic sugar moieties. In certain embodiments, modified sugar moieties are sugar surrogates. Such sugar surogates may comprise one or more substitutions corresponding to those of substituted sugar moieties.

In certain embodiments, modified sugar moieties are substituted sugar moieties comprising one or more substituent, including but not limited to substituents at the 2' and/or 5' positions. Examples of sugar substituents suitable for the 2'-position, include, but are not limited to: 2'-F, 2'-OCH₃ ("OMe" or "O-methyl"), and 2'-O(CH₂)₂OCH₃ ("MOE"). In certain embodiments, sugar substituents at the 2' position is selected from allyl, amino, azido, thio, O-allyl, O-C₁-C₁₀ alkyl, O-C₁-C₁₀ substituted alkyl; O- C₁-C₁₀ alkoxy; O- C₁-C₁₀ substituted alkoxy, OCF₃, O(CH₂)₂SCH₃, O(CH₂)₂-O-N(Rm)(Rn), and O-CH₂-C(=O)-N(Rm)(Rn), where each Rm and Rn is, independently, H or substituted or unsubstituted C₁-C₁₀ alkyl. Examples of sugar substituents at the 5'-position, include, but are not limited to:, 5'-methyl (R or S); 5'-vinyl, and 5'-methoxy. In certain embodiments, substituted sugars comprise more than one non-bridging sugar substituent, for example, 2'-F-5'-methyl sugar moieties (*see*,e.g., PCT International Application WO 2008/101157, for additional 5', 2'-bis substituted sugar moieties and nucleosides).

Nucleosides comprising 2'-substituted sugar moieties are referred to as 2'-substituted nucleosides. In certain embodiments, a 2'- substituted nucleoside comprises a 2'-substituent group selected from halo, allyl, amino, azido, O- C₁-C₁₀ alkoxy; O- C₁-C₁₀ substituted alkoxy, SH, CN, OCN, CF₃, OCF₃, O-alkyl, S-alkyl, N(Rₘ)-alkyl; O- alkenyl, S- alkenyl, or N(Rₘ)-alkenyl; O- alkynyl, S- alkynyl, N(Rₘ)-alkynyl; O-alkylenyl-O-alkyl, alkynyl, alkaryl, aralkyl, O-alkaryl, O-aralkyl, O(CH₂)₂SCH₃, O-(CH₂)₂-O-N(Rₘ)(Rₙ) or O-CH₂-C(=O)-N(Rₘ)(Rₙ), where each Rₘ and Rₙ is, independently, H, an amino protecting group or substituted or unsubstituted C₁-C₁₀ alkyl. These 2'-substituent groups can be further substituted with one or more substituent groups independently selected from hydroxyl, amino, alkoxy, carboxy, benzyl, phenyl, nitro (NO₂), thiol, thioalkoxy (S-alkyl), halogen, alkyl, aryl, alkenyl and alkynyl.

In certain embodiments, a 2'- substituted nucleoside comprises a 2'-substituent group selected from F, NH₂, N₃, OCF₃, O-CH₃, O(CH₂)₃NH₂, CH₂-CH=CH₂, O-CH₂-CH=CH₂, OCH₂CH₂OCH₃, O(CH₂)₂SCH₃, O-(CH₂)₂-O-N(Rₘ)(Rₙ), O(CH₂)₂O(CH₂)₂N(CH₃)₂, and N-substituted acetamide (O-CH₂-C(=O)-N(Rₘ)(Rₙ) where each Rₘ and Rₙ is, independently, H, an amino protecting group or substituted or unsubstituted C₁-C₁₀ alkyl.

In certain embodiments, a 2'- substituted nucleoside comprises a sugar moiety comprising a 2'-substituent group selected from F, OCF₃, O-CH₃, OCH₂CH₂OCH₃, O(CH₂)₂SCH₃, O-(CH₂)₂-ON(CH₃)₂, -O(CH₂)₂O(CH₂)₂N(CH₃)₂, and O-CH₂-C(=O)-N(H)CH₃.

In certain embodiments, a 2'- substituted nucleoside comprises a sugar moiety comprising a 2'-substituent group selected from F, O-CH₃, and OCH₂CH₂OCH₃.

Certain modifed sugar moieties comprise a bridging sugar substituent that forms a second ring resulting in a bicyclic sugar moiety. In certain such embodiments, the bicyclic sugar moiety comprises a bridge between the 4' and the 2' furanose ring atoms. Examples of such 4' to 2' sugar substituents, include, but are not limited to: -[C(Rₐ)(R_{b})]ₙ-, -[C(Rₐ)(R_{b})]ₙ-O-, -C(RₐR_{b})-N(R)-O- or, -C(RₐR_{b})-O-N(R)-; 4'-CH₂-2', 4'-(CH₂)₂-2', 4'-(CH₂)₃-2',. 4'-(CH₂)-O-2' (LNA); 4'-(CH₂)-S-2'; 4'-(CH₂)₂-O-2' (ENA); 4'-CH(CH₃)-O-2' (cEt) and 4'-CH(CH₂OCH₃)-O-2',and analogs thereof (*see, e.g.,* U.S. Patent 7,399,845, issued on July 15, 2008); 4'-C(CH₃)(CH₃)-O-2'and analogs thereof, (*see, e.g.,* WO2009/006478, published January 8, 2009); 4'-CH₂-N(OCH₃)-2' and analogs thereof (*see*, *e.g.,* WO2008/150729, published December 11, 2008); 4'-CH₂-ON(CH₃)-2' *(see, e.g.,* US2004/0171570, published September 2, 2004); 4'-CH₂-O-N(R)-2', and 4'-CH₂-N(R)-O-2'-, wherein each R is, independently, H, a protecting group, or C₁-C₁₂ alkyl; 4'-CH₂-N(R)-O-2', wherein R is H, C₁-C₁₂ alkyl, or a protecting group *(see,* U.S. Patent 7,427,672, issued on September 23, 2008); 4'-CH₂-C(H)(CH₃)-2' *(see, e.g.,* Chattopadhyaya, et al., J. Org. Chem.,2009, 74, 118-134); and 4'-CH₂-C(=CH₂)-2' and analogs thereof *(see,* published PCT International Application WO 2008/154401, published on December 8, 2008).

In certain embodiments, such 4' to 2' bridges independently comprise from 1 to 4 linked groups independently selected from -[C(Rₐ)(R_{b})]ₙ-, -C(Rₐ)=C(R_{b})-, -C(Rₐ)=N-, -C(=NRₐ)-, -C(=O)-, -C(=S)-, -O-, - Si(Rₐ)₂-, -S(=O)ₓ-, and -N(Rₐ)-;
wherein:
x is 0, 1, or 2;
n is 1, 2, 3, or 4;
each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl, or a protecting group.

Nucleosides comprising bicyclic sugar moieties are referred to as bicyclic nucleosides or BNAs. Bicyclic nucleosides include, but are not limited to, (A) α-L-Methyleneoxy (4'-CH₂-O-2') BNA, (B) β-D-Methyleneoxy (4'-CH₂-O-2') BNA (also referred to as locked nucleic acid or LNA), (C) Ethyleneoxy (4'-(CH₂)₂-O-2') BNA, (D) Aminooxy (4'-CH₂-O-N(R)-2') BNA, (E) Oxyamino (4'-CH₂-N(R)-O-2') BNA, (F) Methyl(methyleneoxy) (4'-CH(CH₃)-O-2') BNA (also referred to as constrained ethyl or cEt), (G) methylene-thio (4'-CH₂-S-2') BNA, (H) methylene-amino (4'-CH2-N(R)-2') BNA, (I) methyl carbocyclic (4'-CH₂-CH(CH₃)-2') BNA, and (J) propylene carbocyclic (4'-(CH₂)₃-2') BNA as depicted below. 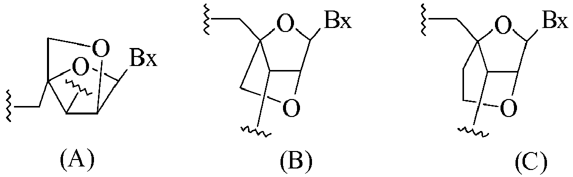 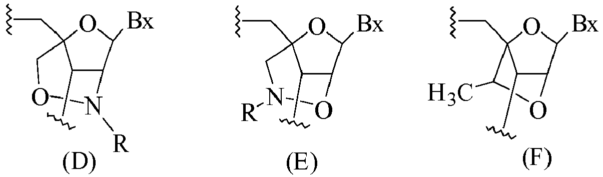 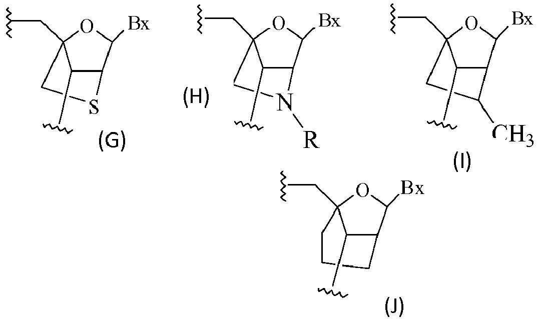 wherein Bx is a nucleobase moiety and R is, independently, H, a protecting group, or C₁-C₁₂ alkyl.

Additional bicyclic sugar moieties are known in the art, for example: Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; Singh et al., J. Org. Chem., 1998, 63, 10035-10039; Srivastava et al., J. Am. Chem. Soc., 129(26) 8362-8379 (Jul. 4, 2007); Elayadi et al., Curr. Opinion Invens. Drugs, 2001, 2, 558-561; Braasch et al., Chem. Biol., 2001, 8, 1-7; Orum et al., Curr. Opinion Mol. Ther., 2001, 3, 239-243; U.S. Patent Nos. 7,053,207, 6,268,490, 6,770,748, 6,794,499, 7,034,133, 6,525,191, 6,670,461, and 7,399,845; WO 2004/106356, WO 1994/14226, WO 2005/021570, and WO 2007/134181; U.S. Patent Publication Nos. US2004/0171570, US2007/0287831, and US2008/0039618; U.S. Patent Serial Nos. 12/129,154, 60/989,574, 61/026,995, 61/026,998, 61/056,564, 61/086,231, 61/097,787, and 61/099,844; and PCT International Applications Nos. PCT/US2008/064591, PCT/US2008/066154, and PCT/US2008/068922.

In certain embodiments, bicyclic sugar moieties and nucleosides incorporating such bicyclic sugar moieties are further defined by isomeric configuration. For example, a nucleoside comprising a 4'-2' methylene-oxy bridge, may be in the α-L configuration or in the β-D configuration. Previously, α-L-methyleneoxy (4'-CH₂-O-2') bicyclic nucleosides have been incorporated into antisense oligonucleotides that showed antisense activity (Frieden et al., Nucleic Acids Research, 2003, 21, 6365-6372).

In certain embodiments, substituted sugar moieties comprise one or more non-bridging sugar substituent and one or more bridging sugar substituent (e.g., 5'-substituted and 4'-2' bridged sugars), *(see,* PCT International Application WO 2007/134181, published on 11/22/07, wherein LNA is substituted with, for example, a 5'-methyl or a 5'-vinyl group).

In certain embodiments, modified sugar moieties are sugar surrogates. In certain such embodiments, the oxygen atom of the naturally occuring sugar is substituted, e.g., with a sulfur, carbon or nitrogen atom. In certain such embodiments, such modified sugar moiety also comprises bridging and/or non-bridging substituents as described above. For example, certain sugar surogates comprise a 4'-sulfur atom and a substitution at the 2'-position (*see,*e.g., published U.S. Patent Application US2005/0130923, published on June 16, 2005) and/or the 5' position. By way of additional example, carbocyclic bicyclic nucleosides having a 4'-2' bridge have been described *(see, e.g.,* Freier et al., Nucleic Acids Research, 1997, 25(22), 4429-4443 and Albaek et al., J. Org. Chem., 2006, 71, 7731-7740).

In certain embodiments, sugar surrogates comprise rings having other than 5-atoms. For example, in certain embodiments, a sugar surrogate comprises a six-membered tetrahydropyran. Such tetrahydropyrans may be further modified or substituted. Nucleosides comprising such modified tetrahydropyrans include, but are not limited to, hexitol nucleic acid (HNA), anitol nucleic acid (ANA), manitol nucleic acid (MNA) *(see* Leumann, CJ. Bioorg. & Med. Chem. (2002) 10:841-854), fluoro HNA (F-HNA), and those compounds having Formula VII: 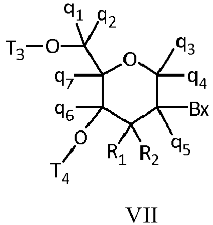 wherein independently for each of said at least one tetrahydropyran nucleoside analog of Formula VII:
Bx is a nucleobase moiety;
T₃ and T₄ are each, independently, an internucleoside linking group linking the tetrahydropyran nucleoside analog to the antisense compound or one of T₃ and T₄ is an internucleoside linking group linking the tetrahydropyran nucleoside analog to the antisense compound and the other of T₃ and T₄ is H, a hydroxyl protecting group, a linked conjugate group, or a 5' or 3'-terminal group;
q₁, q₂, q₃, q₄, q₅, q₆ and q₇ are each, independently, H, C₁-C₆ alkyl, substituted C₁-C₆ alkyl, C₂-C₆ alkenyl, substituted C₂-C₆ alkenyl, C₂-C₆ alkynyl, or substituted C₂-C₆ alkynyl; and
each of R₁ and R₂ is independently selected from among: hydrogen, halogen, substituted or unsubstituted alkoxy, NJ₁J₂, SJ₁, N₃, OC(=X)J₁, OC(=X)NJ₁J₂, NJ₃C(=X)NJ₁J₂, and CN, wherein X is O, S or NJ₁, and each J₁, J₂, and J₃ is, independently, H or C₁-C₆ alkyl.

In certain embodiments, the modified THP nucleosides of Formula VII are provided wherein q₁, q₂, q₃, q₄, q₅, q₆ and q₇ are each H. In certain embodiments, at least one of q₁, q₂, q₃, q₄, q₅, q₆ and q₇ is other than H. In certain embodiments, at least one of q₁, q₂, q₃, q₄, q₅, q₆ and q₇ is methyl. In certain embodiments, THP nucleosides of Formula VII are provided wherein one of R₁ and R₂ is F. In certain embodiments, R₁ is fluoro and R₂ is H, R₁ is methoxy and R₂ is H, and R₁ is methoxyethoxy and R₂ is H.

Many other bicyclic and tricyclic sugar and sugar surrogate ring systems are known in the art that can be used to modify nucleosides *(see, e.g.,* review article: Leumann, J. C, Bioorganic & Medicinal Chemistry, 2002, 10, 841-854).

In certain embodiments, sugar surrogates comprise rings having more than 5 atoms and more than one heteroatom. For example nucleosides comprising morpholino sugar moieties and their use in oligomeric compounds has been reported (see for example: Braasch et al., Biochemistry, 2002, 41, 4503-4510; and U.S. Patents 5,698,685; 5,166,315; 5,185,444; and 5,034,506). As used here, the term "morpholino" means a sugar surrogate having the following structure: 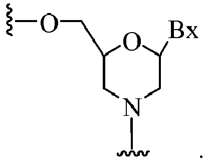 In certain embodiments, morpholinos may be modified, for example by adding or altering various substituent groups from the above morpholino structure. Such sugar surrogates are refered to herein as "modifed morpholinos."

Combinations of modifications are also provided without limitation, such as 2'-F-5'-methyl substituted nucleosides (see PCT International Application WO 2008/101157 Published on 8/21/08 for other disclosed 5', 2'-bis substituted nucleosides) and replacement of the ribosyl ring oxygen atom with S and further substitution at the 2'-position (see published U.S. Patent Application US2005-0130923, published on June 16, 2005) or alternatively 5'-substitution of a bicyclic nucleic acid (see PCT International Application WO 2007/134181, published on 11/22/07 wherein a 4'-CH₂-O-2' bicyclic nucleoside is further substituted at the 5' position with a 5'-methyl or a 5'-vinyl group). The synthesis and preparation of carbocyclic bicyclic nucleosides along with their oligomerization and biochemical studies have also been described (*see*, *e.g.,* Srivastava et al., J. Am. Chem. Soc. 2007, 129(26), 8362-8379).

### Certain Nucleobases

In certain embodiments, nucleosides of the present invention comprise one or more unmodified nucleobases. In certain embodiments, nucleosides of the present invention comprise one or more modifed nucleobases.

In certain embodiments, modified nucleobases are selected from: universal bases, hydrophobic bases, promiscuous bases, size-expanded bases, and fluorinated bases as defined herein. 5-substituted pyrimidines, 6-azapyrimidines and N-2, N-6 and O-6 substituted purines, including 2-aminopropyladenine, 5-propynyluracil; 5-propynylcytosine; 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine, 3-deazaguanine and 3-deazaadenine, universal bases, hydrophobic bases, promiscuous bases, size-expanded bases, and fluorinated bases as defined herein. Further modified nucleobases include tricyclic pyrimidines such as phenoxazine cytidine([5,4-b] [1,4]benzoxazin-2(3H)-one), phenothiazine cytidine (1H-pyrimido[5,4-b][1,4]benzothiazin-2(3H)-one), G-clamps such as a substituted phenoxazine cytidine (e.g. 9-(2-aminoethoxy)-H-pyrimido[5,4-b][1,4]benzoxazin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in United States Patent No. 3,687,808, those disclosed in The Concise Encyclopedia Of Polymer Science And Engineering, Kroschwitz, J.I., Ed., John Wiley & Sons, 1990, 858-859; those disclosed by Englisch et al., Angewandte Chemie, International Edition, 1991, 30, 613; and those disclosed by Sanghvi, Y.S., Chapter 15, Antisense Research and Applications, Crooke, S.T. and Lebleu, B., Eds., CRC Press, 1993, 273-288.

Representative United States patents that teach the preparation of certain of the above noted modified nucleobases as well as other modified nucleobases include without limitation, U.S. 3,687,808; 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121; 5,596,091; 5,614,617; 5,645,985; 5,681,941; 5,750,692; 5,763,588; 5,830,653 and 6,005,096, certain of which are commonly owned with the instant application, and each of which is herein incorporated by reference in its entirety.

### Certain Internucleoside Linkages

In certain embodiments, the present invention provides oligomeric compounds comprising linked nucleosides. In such embodiments, nucleosides may be linked together using any internucleoside linkage. The two main classes of internucleoside linking groups are defined by the presence or absence of a phosphorus atom. Representative phosphorus containing internucleoside linkages include, but are not limited to, phosphodiesters (P=O), phosphotriesters, methylphosphonates, phosphoramidate, and phosphorothioates (P=S). Representative non-phosphorus containing internucleoside linking groups include, but are not limited to, methylenemethylimino (-CH₂-N(CH₃)-O-CH₂-), thiodiester (-O-C(O)-S-), thionocarbamate (-O-C(O)(NH)-S-); siloxane (-O-Si(H)₂-O-); and N,N'-dimethylhydrazine (-CH₂-N(CH₃)-N(CH₃)-). Modified linkages, compared to natural phosphodiester linkages, can be used to alter, typically increase, nuclease resistance of the oligomeric compound. In certain embodiments, internucleoside linkages having a chiral atom can be prepared as a racemic mixture, or as separate enantiomers. Representative chiral linkages include, but are not limited to, alkylphosphonates and phosphorothioates. Methods of preparation of phosphorous-containing and non-phosphorous-containing internucleoside linkages are well known to those skilled in the art.

The oligonucleotides described herein contain one or more asymmetric centers and thus give rise to enantiomers, diastereomers, and other stereoisomeric configurations that may be defined, in terms of absolute stereochemistry, as (R) or (S), α or β such as for sugar anomers, or as (D) or (L) such as for amino acids etc. Included in the antisense compounds provided herein are all such possible isomers, as well as their racemic and optically pure forms.

Neutral internucleoside linkages include without limitation, phosphotriesters, methylphosphonates, MMI (3'-CH₂-N(CH₃)-O-5'), amide-3 (3'-CH₂-C(=O)-N(H)-5'), amide-4 (3'-CH₂-N(H)-C(=O)-5'), formacetal (3'-O-CH₂-O-5'), and thioformacetal (3'-S-CH₂-O-5'). Further neutral internucleoside linkages include nonionic linkages comprising siloxane (dialkylsiloxane), carboxylate ester, carboxamide, sulfide, sulfonate ester and amides (See for example: Carbohydrate Modifications in Antisense Research; Y.S. Sanghvi and P.D. Cook, Eds., ACS Symposium Series 580; Chapters 3 and 4, 40-65). Further neutral internucleoside linkages include nonionic linkages comprising mixed N, O, S and CH₂ component parts.

### Certain Motifs

In certain embodiments, the present invention provides oligomeric compounds comprising oligonucleotides. In certain embodiments, such oligonucleotides comprise one or more chemical modification. In certain embodiments, chemically modified oligonucleotides comprise one or more modified nucleosides. In certain embodiments, chemically modified oligonucleotides comprise one or more modified nucleosides comprising modified sugars. In certain embodiments, chemically modified oligonucleotides comprise one or more modified nucleosides comprising one or more modified nucleobases. In certain embodiments, chemically modified oligonucleotides comprise one or more modified internucleoside linkages. In certain embodiments, the chemically modifications (sugar modifications, nucleobase modifications, and/or linkage modifications) define a pattern or motif. In certain embodiments, the patterns of chemical modifications of sugar moieties, internucleoside linkages, and nucleobases are each independent of one another. Thus, an oligonucleotide may be described by its sugar modification motif, internucleoside linkage motif and/or nucleobase modification motif (as used herein, nucleobase modification motif describes the chemical modifications to the nucleobases independent of the sequence of nucleobases).

### Certain sugar motifs

In certain embodiments, oligonucleotides comprise one or more type of modified sugar moieties and/or naturally occurring sugar moieties arranged along an oligonucleotide or region thereof in a defined pattern or sugar modification motif. Such motifs may include any of the sugar modifications discussed herein and/or other known sugar modifications.

In certain embodiments, the oligonucleotides comprise or consist of a region having a gapmer sugar modification motif, which comprises two external regions or "wings" and an internal region or "gap." The three regions of a gapmer motif (the 5'-wing, the gap, and the 3'-wing) form a contiguous sequence of nucleosides wherein at least some of the sugar moieties of the nucleosides of each of the wings differ from at least some of the sugar moieties of the nucleosides of the gap. Specifically, at least the sugar moieties of the nucleosides of each wing that are closest to the gap (the 3'-most nucleoside of the 5'-wing and the 5'-most nucleoside of the 3'-wing) differ from the sugar moiety of the neighboring gap nucleosides, thus defining the boundary between the wings and the gap. In certain embodiments, the sugar moieties within the gap are the same as one another. In certain embodiments, the gap includes one or more nucleoside having a sugar moiety that differs from the sugar moiety of one or more other nucleosides of the gap. In certain embodiments, the sugar modification motifs of the two wings are the same as one another (symmetric gapmer). In certain embodiments, the sugar modification motifs of the 5'-wing differs from the sugar modification motif of the 3'-wing (asymmetric gapmer). In certain embodiments, oligonucleotides comprise 2'-MOE modified nucleosides in the wings and 2'-F modified nucleosides in the gap.

In certain embodiments, oligonucleotides are fully modified. In certain such embodiments, oligonucleotides are uniformly modified. In certain embodiments, oligonucleotides are uniform 2'-MOE. In certain embodiments, oligonucleotides are uniform 2'-F. In certain embodiments, oligonucleotides are uniform morpholino. In certain embodiments, oligonucleotides are uniform BNA. In certain embodiments, oligonucleotides are uniform LNA. In certain embodiments, oligonucleotides are uniform cEt.

In certain embodiments, oligonucleotides comprise a uniformly modified region and additional nucleosides that are unmodified or differently modified. In certain embodiments, the uniformly modified region is at least 5, 10, 15, or 20 nucleosides in length. In certain embodiments, the uniform region is a 2'-MOE region. In certain embodiments, the uniform region is a 2'-F region. In certain embodiments, the uniform region is a morpholino region. In certain embodiments, the uniform region is a BNA region. In certain embodiments, the uniform region is a LNA region. In certain embodiments, the uniform region is a cEt region.

In certain embodiments, the oligonucleotide does not comprise more than 4 contiguous unmodified 2'-deoxynucleosides. In certain circumstances, antisesense oligonucleotides comprising more than 4 contiguous 2'-deoxynucleosides activate RNase H, resulting in cleavage of the target RNA. In certain embodiments, such cleavage is avoided by not having more than 4 contiguous 2'-deoxynucleosides, for example, where alteration of splicing and not cleavage of a target RNA is desired.

### Certain Internucleoside Linkage Motifs

In certain embodiments, oligonucleotides comprise modified internucleoside linkages arranged along the oligonucleotide or region thereof in a defined pattern or modified internucleoside linkage motif. In certain embodiments, internucleoside linkages are arranged in a gapped motif, as described above for sugar modification motif. In such embodiments, the internucleoside linkages in each of two wing regions are different from the internucleoside linkages in the gap region. In certain embodiments the internucleoside linkages in the wings are phosphodiester and the internucleoside linkages in the gap are phosphorothioate. The sugar modification motif is independently selected, so such oligonucleotides having a gapped internucleoside linkage motif may or may not have a gapped sugar modification motif and if it does have a gapped sugar motif, the wing and gap lengths may or may not be the same.

In certain embodiments, oligonucleotides comprise a region having an alternating internucleoside linkage motif. In certain embodiments, oligonucleotides of the present invention comprise a region of uniformly modified internucleoside linkages. In certain such embodiments, the oligonucleotide comprises a region that is uniformly linked by phosphorothioate internucleoside linkages. In certain embodiments, the oligonucleotide is uniformly linked by phosphorothioate. In certain embodiments, each internucleoside linkage of the oligonucleotide is selected from phosphodiester and phosphorothioate. In certain embodiments, each internucleoside linkage of the oligonucleotide is selected from phosphodiester and phosphorothioate and at least one internucleoside linkage is phosphorothioate.

In certain embodiments, the oligonucleotide comprises at least 6 phosphorothioate internucleoside linkages. In certain embodiments, the oligonucleotide comprises at least 8 phosphorothioate internucleoside linkages. In certain embodiments, the oligonucleotide comprises at least 10 phosphorothioate internucleoside linkages. In certain embodiments, the oligonucleotide comprises at least one block of at least 6 consecutive phosphorothioate internucleoside linkages. In certain embodiments, the oligonucleotide comprises at least one block of at least 8 consecutive phosphorothioate internucleoside linkages. In certain embodiments, the oligonucleotide comprises at least one block of at least 10 consecutive phosphorothioate internucleoside linkages. In certain embodiments, the oligonucleotide comprises at least block of at least one 12 consecutive phosphorothioate internucleoside linkages. In certain such embodiments, at least one such block is located at the 3' end of the oligonucleotide. In certain such embodiments, at least one such block is located within 3 nucleosides of the 3' end of the oligonucleotide.

### Certain Nucleobase Modification Motifs

In certain embodiments, oligonucleotides comprise chemical modifications to nucleobases arranged along the oligonucleotide or region thereof in a defined pattern or nucleobases modification motif. In certain such embodiments, nucleobase modifications are arranged in a gapped motif. In certain embodiments, nucleobase modifications are arranged in an alternating motif. In certain embodiments, each nucleobase is modified. In certain embodiments, none of the nucleobases is chemically modified.

In certain embodiments, oligonucleotides comprise a block of modified nucleobases. In certain such embodiments, the block is at the 3'-end of the oligonucleotide. In certain embodiments the block is within 3 nucleotides of the 3'-end of the oligonucleotide. In certain such embodiments, the block is at the 5'-end of the oligonucleotide. In certain embodiments the block is within 3 nucleotides of the 5'-end of the oligonucleotide.

In certain embodiments, nucleobase modifications are a function of the natural base at a particular position of an oligonucleotide. For example, in certain embodiments each purine or each pyrimidine in an oligonucleotide is modified. In certain embodiments, each adenine is modified. In certain embodiments, each guanine is modified. In certain embodiments, each thymine is modified. In certain embodiments, each cytosine is modified. In certain embodiments, each uracil is modified.

In certain embodiments, some, all, or none of the cytosine moieties in an oligonucleotide are 5-methyl cytosine moieties. Herein, 5-methyl cytosine is not a "modified nucleobase." Accordingly, unless otherwise indicated, unmodified nucleobases include both cytosine residues having a 5-methyl and those lacking a 5 methyl. In certain embodiments, the methylation state of all or some cytosine nucleobases is specified.

### Certain Overall Lengths

In certain embodiments, the present invention provides oligomeric compounds including oligonucleotides of any of a variety of ranges of lengths. In certain embodiments, the invention provides oligomeric compounds or oligonucleotides consisting of X to Y linked nucleosides, where X represents the fewest number of nucleosides in the range and Y represents the largest number of nucleosides in the range. In certain such embodiments, X and Y are each independently selected from 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50; provided that X≤Y. For example, in certain embodiments, the invention provides oligomeric compounds which comprise oligonucleotides consisting of 8 to 9, 8 to 10, 8 to 11, 8 to 12, 8 to 13, 8 to 14, 8 to 15, 8 to 16, 8 to 17, 8 to 18, 8 to 19, 8 to 20, 8 to 21, 8 to 22, 8 to 23, 8 to 24, 8 to 25, 8 to 26, 8 to 27, 8 to 28, 8 to 29, 8 to 30, 9 to 10, 9 to 11, 9 to 12, 9 to 13, 9 to 14, 9 to 15, 9 to 16, 9 to 17, 9 to 18, 9 to 19, 9 to 20, 9 to 21, 9 to 22, 9 to 23, 9 to 24, 9 to 25, 9 to 26, 9 to 27, 9 to 28, 9 to 29, 9 to 30, 10 to 11, 10 to 12, 10 to 13, 10 to 14, 10 to 15, 10 to 16, 10 to 17, 10 to 18, 10 to 19, 10 to 20, 10 to 21, 10 to 22, 10 to 23, 10 to 24, 10 to 25, 10 to 26, 10 to 27, 10 to 28, 10 to 29, 10 to 30, 11 to 12, 11 to 13, 11 to 14, 11 to 15, 11 to 16, 11 to 17, 11 to 18, 11 to 19, 11 to 20, 11 to 21, 11 to 22, 11 to 23, 11 to 24, 11 to 25, 11 to 26, 11 to 27, 11 to 28, 11 to 29, 11 to 30, 12 to 13, 12 to 14, 12 to 15, 12 to 16, 12 to 17, 12 to 18, 12 to 19, 12 to 20, 12 to 21, 12 to 22, 12 to 23, 12 to 24, 12 to 25, 12 to 26, 12 to 27, 12 to 28, 12 to 29, 12 to 30, 13 to 14, 13 to 15, 13 to 16, 13 to 17, 13 to 18, 13 to 19, 13 to 20, 13 to 21, 13 to 22, 13 to 23, 13 to 24, 13 to 25, 13 to 26, 13 to 27, 13 to 28, 13 to 29, 13 to 30, 14 to 15, 14 to 16, 14 to 17, 14 to 18, 14 to 19, 14 to 20, 14 to 21, 14 to 22, 14 to 23, 14 to 24, 14 to 25, 14 to 26, 14 to 27, 14 to 28, 14 to 29, 14 to 30, 15 to 16, 15 to 17, 15 to 18, 15 to 19, 15 to 20, 15 to 21, 15 to 22, 15 to 23, 15 to 24, 15 to 25, 15 to 26, 15 to 27, 15 to 28, 15 to 29, 15 to 30, 16 to 17, 16 to 18, 16 to 19, 16 to 20, 16 to 21, 16 to 22, 16 to 23, 16 to 24, 16 to 25, 16 to 26, 16 to 27, 16 to 28, 16 to 29, 16 to 30, 17 to 18, 17 to 19, 17 to 20, 17 to 21, 17 to 22, 17 to 23, 17 to 24, 17 to 25, 17 to 26, 17 to 27, 17 to 28, 17 to 29, 17 to 30, 18 to 19, 18 to 20, 18 to 21, 18 to 22, 18 to 23, 18 to 24, 18 to 25, 18 to 26, 18 to 27, 18 to 28, 18 to 29, 18 to 30, 19 to 20, 19 to 21, 19 to 22, 19 to 23, 19 to 24, 19 to 25, 19 to 26, 19 to 29, 19 to 28, 19 to 29, 19 to 30, 20 to 21, 20 to 22, 20 to 23, 20 to 24, 20 to 25, 20 to 26, 20 to 27, 20 to 28, 20 to 29, 20 to 30, 21 to 22, 21 to 23, 21 to 24, 21 to 25, 21 to 26, 21 to 27, 21 to 28, 21 to 29, 21 to 30, 22 to 23, 22 to 24, 22 to 25, 22 to 26, 22 to 27, 22 to 28, 22 to 29, 22 to 30, 23 to 24, 23 to 25, 23 to 26, 23 to 27, 23 to 28, 23 to 29, 23 to 30, 24 to 25, 24 to 26, 24 to 27, 24 to 28, 24 to 29, 24 to 30, 25 to 26, 25 to 27, 25 to 28, 25 to 29, 25 to 30, 26 to 27, 26 to 28, 26 to 29, 26 to 30, 27 to 28, 27 to 29, 27 to 30, 28 to 29, 28 to 30, or 29 to 30 linked nucleosides. In embodiments where the number of nucleosides of an oligomeric compound or oligonucleotide is limited, whether to a range or to a specific number, the oligomeric compound or oligonucleotide may, nonetheless further comprise additional other substituents. For example, an oligonucleotide comprising 8-30 nucleosides excludes oligonucleotides having 31 nucleosides, but, unless otherwise indicated, such an oligonucleotide may further comprise, for example one or more conjugates, terminal groups, or other substituents. In certain embodiments, a gapmer oligonucleotide has any of the above lengths.

One of skill in the art will appreciate that certain lengths may not be possible for certain motifs. For example: a gapmer having a 5'-wing region consisting of four nucleotides, a gap consisting of at least six nucleotides, and a 3'-wing region consisting of three nucleotides cannot have an overall length less than 13 nucleotides. Thus, one would understand that the lower length limit is 13 and that the limit of 10 in "10-20" has no effect in that embodiment.

Further, where an oligonucleotide is described by an overall length range and by regions having specified lengths, and where the sum of specified lengths of the regions is less than the upper limit of the overall length range, the oligonucleotide may have additional nucleosides, beyond those of the specified regions, provided that the total number of nucleosides does not exceed the upper limit of the overall length range. For example, an oligonucleotide consisting of 20-25 linked nucleosides comprising a 5'-wing consisting of 5 linked nucleosides; a 3'-wing consisting of 5 linked nucleosides and a central gap consisting of 10 linked nucleosides (5+5+10=20) may have up to 5 nucleosides that are not part of the 5'-wing, the 3'-wing, or the gap (before reaching the overall length limitation of 25). Such additional nucleosides may be 5' of the 5'-wing and/or 3' of the 3' wing.

### Certain Oligonucleotides

In certain embodiments, oligonucleotides of the present invention are characterized by their sugar motif, internucleoside linkage motif, nucleobase modification motif and overall length. In certain embodiments, such parameters are each independent of one another. Thus, each internucleoside linkage of an oligonucleotide having a gapmer sugar motif may be modified or unmodified and may or may not follow the gapmer modification pattern of the sugar modifications. Thus, the internucleoside linkages within the wing regions of a sugar-gapmer may be the same or different from one another and may be the same or different from the internucleoside linkages of the gap region. Likewise, such sugar-gapmer oligonucleotides may comprise one or more modified nucleobase independent of the gapmer pattern of the sugar modifications. Herein if a description of an oligonucleotide or oligomeric compound is silent with respect to one or more parameter, such parameter is not limited. Thus, an oligomeric compound described only as having a gapmer sugar motif without further description may have any length, internucleoside linkage motif, and nucleobase modification motif. Unless otherwise indicated, all chemical modifications are independent of nucleobase sequence.

### Certain Conjugate Groups

In certain embodiments, oligomeric compounds are modified by attachment of one or more conjugate groups. In general, conjugate groups modify one or more properties of the attached oligomeric compound including but not limited to pharmacodynamics, pharmacokinetics, stability, binding, absorption, cellular distribution, cellular uptake, charge and clearance. Conjugate groups are routinely used in the chemical arts and are linked directly or via an optional conjugate linking moiety or conjugate linking group to a parent compound such as an oligomeric compound, such as an oligonucleotide. Conjugate groups includes without limitation, intercalators, reporter molecules, polyamines, polyamides, polyethylene glycols, thioethers, polyethers, cholesterols, thiocholesterols, cholic acid moieties, folate, lipids, phospholipids, biotin, phenazine, phenanthridine, anthraquinone, adamantane, acridine, fluoresceins, rhodamines, coumarins and dyes. Certain conjugate groups have been described previously, for example: cholesterol moiety (Letsinger et al., Proc. Natl. Acad. Sci. USA, 1989, 86, 6553-6556), cholic acid (Manoharan et al., Bioorg. Med. Chem. Let., 1994, 4, 1053-1060), a thioether, e.g., hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, e.g., do-decan-diol or undecyl residues (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethyl-ammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyl-oxycholesterol moiety (Crooke et al., J. Pharmacol. Exp. Ther., 1996, 277, 923-937).

In certain embodiments, a conjugate group comprises an active drug substance, for example, aspirin, warfarin, phenylbutazone, ibuprofen, suprofen, fen-bufen, ketoprofen, (*S*)-(+)-pranoprofen, carprofen, dansylsarcosine, 2,3,5-triiodobenzoic acid, flufenamic acid, folinic acid, a benzothiadiazide, chlorothiazide, a diazepine, indo-methicin, a barbiturate, a cephalosporin, a sulfa drug, an antidiabetic, an antibacterial or an antibiotic.

In certain embodiments, conjugate groups are directly attached to oligonucleotides in oligomeric compounds. In certain embodiments, conjugate groups are attached to oligonucleotides by a conjugate linking group. In certain such embodiments, conjugate linking groups, including, but not limited to, bifunctional linking moieties such as those known in the art are amenable to the compounds provided herein. Conjugate linking groups are useful for attachment of conjugate groups, such as chemical stabilizing groups, functional groups, reporter groups and other groups to selective sites in a parent compound such as for example an oligomeric compound. In general a bifunctional linking moiety comprises a hydrocarbyl moiety having two functional groups. One of the functional groups is selected to bind to a parent molecule or compound of interest and the other is selected to bind essentially any selected group such as chemical functional group or a conjugate group. In some embodiments, the conjugate linker comprises a chain structure or an oligomer of repeating units such as ethylene glycol or amino acid units. Examples of functional groups that are routinely used in a bifunctional linking moiety include, but are not limited to, electrophiles for reacting with nucleophilic groups and nucleophiles for reacting with electrophilic groups. In some embodiments, bifunctional linking moieties include amino, hydroxyl, carboxylic acid, thiol, unsaturations (e.g., double or triple bonds), and the like.

Some nonlimiting examples of conjugate linking moieties include pyrrolidine, 8-amino-3,6-dioxaoctanoic acid (ADO), succinimidyl 4-(N-maleimidomethyl) cyclohexane-1-carboxylate (SMCC) and 6-aminohexanoic acid (AHEX or AHA). Other linking groups include, but are not limited to, substituted C₁-C₁₀ alkyl, substituted or unsubstituted C₂-C₁₀ alkenyl or substituted or unsubstituted C₂-C₁₀ alkynyl, wherein a nonlimiting list of preferred substituent groups includes hydroxyl, amino, alkoxy, carboxy, benzyl, phenyl, nitro, thiol, thioalkoxy, halogen, alkyl, aryl, alkenyl and alkynyl.

Conjugate groups may be attached to either or both ends of an oligonucleotide (terminal conjugate groups) and/or at any internal position.

In certain embodiments, conjugate groups are at the 3'-end of an oligonucleotide of an oligomeric compound. In certain embodiments, conjugate groups are near the 3'-end. In certain embodiments, conjugates are attached at the 3'end of an oligomeric compound, but before one or more terminal group nucleosides. In certain embodiments, conjugate groups are placed within a terminal group.
In certain embodiments, the present invention provides oligomeric compounds. In certain embodiments, oligomeric compounds comprise an oligonucleotide. In certain embodiments, an oligomeric compound comprises an oligonucleotide and one or more conjugate and/or terminal groups. Such conjugate and/or terminal groups may be added to oligonucleotides having any of the chemical motifs discussed above. Thus, for example, an oligomeric compound comprising an oligonucleotide having region of alternating nucleosides may comprise a terminal group.

### Antisense Compounds

In certain embodiments, oligomeric compounds of the present invention are antisense compounds. Such antisense compounds are capable of hybridizing to a target nucleic acid, resulting in at least one antisense activity. In certain embodiments, antisense compounds specifically hybridize to one or more target nucleic acid. In certain embodiments, a specifically hybridizing antisense compound has a nucleobase sequence comprising a region having sufficient complementarity to a target nucleic acid to allow hybridization and result in antisense activity and insufficient complementarity to any non-target so as to avoid non-specific hybridization to any non-target nucleic acid sequences under conditions in which specific hybridization is desired (e.g., under physiological conditions for *in vivo* or therapeutic uses, and under conditions in which assays are performed in the case of *in vitro* assays).

In certain embodiments, the present invention provides antisense compounds comprising oligonucleotides that are fully complementary to the target nucleic acid over the entire length of the oligonucleotide. In certain embodiments, oligonucleotides are 99% complementary to the target nucleic acid. In certain embodiments, oligonucleotides are 95% complementary to the target nucleic acid. In certain embodiments, such oligonucleotides are 90% complementary to the target nucleic acid.

In certain embodiments, such oligonucleotides are 85% complementary to the target nucleic acid. In certain embodiments, such oligonucleotides are 80% complementary to the target nucleic acid. In certain embodiments, an antisense compound comprises a region that is fully complementary to a target nucleic acid and is at least 80% complementary to the target nucleic acid over the entire length of the oligonucleotide. In certain such embodiments, the region of full complementarity is from 6 to 14 nucleobases in length.

In certain embodiments antisense compounds and antisense oligonucleotides comprise single-strand compounds. In certain embodiments antisense compounds and antisense oligonucleotides comprise double-strand compounds.

### Certain Pathways and Mechanisms Associated With Neuronal ceroid lipofuscinoses

Neuronal ceroid lipofuscinoses (NCL) is the general name for a family of neurodegenerative disorders that result from excessive accumulation of lipopigments, e.g. lipofuscin, in the body's tissues. Juvenile neuronal ceroid lipofuscinosis (JNCL), also known as Batten Disease, is the most common of the NCL disorders. In certain embodiments, JNCL onset occurs between five and eight years of age and symptoms include progressive loss of motor function, seizures, vision loss, and loss of cognitive function, resulting in death before age 30.

JNCL is an autosomal recessive disorder caused by mutations of the CLN3 gene. Approximately 80% of JNCL cases result from a particular deletion of the CLN3 gene spanning exons 7 and 8 (*CLN3*Δ*78*)*.* In certain embodiments, the *CLN3*Δ*78* deletion causes a frameshift that results in a premature stop codon in exon 9. In certain embodiments, the truncated protein product of *CLN3*Δ*78* is 33% of the length of the wild type CLN3 protein. In certain embodiments, the truncated protein product of *CLN3*Δ*78* is non-functional. In certain embodiments, the truncated protein product of *CLN3*Δ*78* is partially functional.

In certain embodiments, antisense oligonucleotides complementary to the mutant *CLN3*Δ*78* transcript eliminate the frameshift that results in a premature stop codon in exon 9. In certain embodiments, for example, antisense oligonucleotides complementary to the mutant *CLN3*Δ*78* transcript produce *CLN3*Δ*78* mRNA having exons downstream of exon 9. For example, in certain embodiments, antisense oligonucleotides complementary to the mutant *CLN3*Δ*78* transcript produce *CLN3*Δ*78* mRNA having exons 10 and/or 11. In certain embodiments, for example, antisense oligonucleotides complementary to the mutant *CLN3*Δ*78* transcript produce a partially deleted, but still functional *CLN3*Δ*78* protein.

In certain embodiments, antisense oligonucleotides complementary to exon 6 of a *CLN3*Δ*78* transcript induces skipping of exon 6 and eliminates the frameshift in *CLN3*Δ*78* pre-mRNA that results in a premature stop codon in exon 9. In such embodiments, oligonucleotides complementary to exon 6 of a *CLN3*Δ*78* transcript produce *CLN3*Δ*78* mRNA without exon 6, but having the remaining exons (e.g. exons 1, 2, 3, 4, 5, 9, 10, 11...). In certain embodiments, *CLN3*Δ*78* mRNA having the remaining exons is translated into a *CLN3*Δ*78* protein that is partially functional. In certain embodiments, *CLN3*Δ*78* mRNA having the remaining exons is translated into a *CLN3*Δ*78* protein that functions in a similar manner as wild type CLN3 protein. In certain embodiments, *CLN3*Δ*78* mRNA having the remaining exons is translated into a *CLN3*Δ*78* protein that functions better than the truncated protein product of *CLN3*Δ*78* containing the premature stop codon. In certain embodiments, *CLN3*Δ*78* mRNA having the remaining exons is translated into a *CLN3*Δ*78* protein that does not produce the excessive accumulation of lipopigments in the body tissue.

In certain embodiments, antisense oligonucleotides complementary to exon 9 of a *CLN3*Δ*78* transcript induce skipping of exon 9 and eliminate the frameshift in *CLN3*Δ*78* pre-mRNA that results in a premature stop codon in exon 9. In such embodiments, oligonucleotides complementary to exon 9 of a *CLN3*Δ*78* transcript produce *CLN3*Δ*78* mRNA without exon 9, but having the remaining exons (e.g. exons 1, 2, 3, 4, 5, 6, 10, 11...). In certain embodiments, *CLN3*Δ*78* mRNA having the remaining exons, e.g. exons 1, 2, 3, 4, 5, 6, 10, 11..., is translated into a *CLN3*Δ*78* protein that is partially functional. In certain embodiments, *CLN3*Δ*78* mRNA missing exons 7, 8, and 9 is translated into a *CLN3*Δ*78* protein that functions in a similar manner as wild type CLN3 protein. In certain embodiments, *CLN3*Δ*78* mRNA missing exons 7, 8, and 9 is translated into a *CLN3*Δ*78* protein that functions better than the truncated protein product of *CLN*Δ*78* containing the premature stop codon. In certain embodiments, *CLN3*Δ*78* mRNA missing exons 7, 8, and 9 is translated into a *CLN3*Δ*78* protein that does not produce the excessive accumulation of lipopigments in the body tissue.

### Certain Target Nucleic Acids and Mechanisms

In certain embodiments, antisense compounds comprise or consist of an oligonucleotide comprising a region that is complementary to a target nucleic acid. In certain embodiments, the target nucleic acid is an endogenous RNA molecule. In certain embodiments, the target nucleic acid is a pre-mRNA. In certain embodiments, the target nucleic acid is a CLN3 transcript. In certain embodiments, the target RNA is a CLN3 pre-mRNA.

In certain embodiments, an antisense compound is complementary to a region of CLN3 pre-mRNA. In certain embodiments, an antisense compound is complementary to a region of CLN3 pre-mRNA comprising an intron-exon splice junction. In certain embodiments, an antisense compound is complementary to a region of CLN3 pre-mRNA comprising the intron-exon splice junction adjacent to exon 6. In certain embodiments, an antisense compound is complementary within a region of CLN3 pre-mRNA consisting of exon 6. In certain embodiments, an antisense compound is complementary within a region of CLN3 pre-mRNA comprising an exonic splicing silencer within an exon 6. In certain embodiments, an antisense compound is complementary to a region of CLN3 pre-mRNA comprising the intron-exon splice junction adjacent to exon 9. In certain embodiments, an antisense compound is complementary within a region of CLN3 pre-mRNA consisting of exon 9. In certain embodiments, an antisense compound is complementary within a region of CLN3 pre-mRNA comprising an exonic splicing silencer within an exon 9.

In certain embodiments, an antisense compound comprises a modified oligonucleotide consisting of 8 to 30 linked nucleosides and having a nucleobase sequence comprising a complementary region comprising at least 8 contiguous nucleobases complementary to a target region of equal length of a CLN3 transcript. In certain embodiments, the target region is within nucleobase 5082 and nucleobase 5119 of SEQ ID NO.: 2. In certain embodiments, the target region is within nucleobase 5082 and nucleobase 5099 of SEQ ID NO.: 2. In certain embodiments, the target region is within nucleobase 5053 and nucleobase 5070 of SEQ ID NO.: 2. In certain embodiments, the target region is within nucleobase 5086 and nucleobase 5103 of SEQ ID NO.: 2. In certain embodiments, the target region is within nucleobase 5090 and nucleobase 5107 of SEQ ID NO.: 2. In certain embodiments, the target region is within nucleobase 5094 and nucleobase 5111 of SEQ ID NO.: 2. In certain embodiments, the target region is within nucleobase 5098 and nucleobase 5115 of SEQ ID NO.: 2. In certain embodiments, the target region is within nucleobase 5102 and nucleobase 5119 of SEQ ID NO.: 2. In certain embodiments, the target region is within nucleobase 5126 and nucleobase 5143 of SEQ ID NO.: 2. In certain embodiments, the target region is within nucleobase 5134 and nucleobase 5155 of SEQ ID NO.: 2. In certain embodiments, the target region is within nucleobase 5134 and nucleobase 5151 of SEQ ID NO.: 2. In certain embodiments, the target region is within nucleobase 5138 and nucleobase 5155 of SEQ ID NO.: 2.

In certain embodiments, an antisense compound comprises a modified oligonucleotide consisting of 8 to 30 linked nucleosides and having a nucleobase sequence comprising a complementary region comprising at least 8 contiguous nucleobases complementary to a target region of equal length of a CLN3 transcript. In certain embodiments, the target region is within nucleobase 7366 and nucleobase 7411 of SEQ ID NO.: 2. In certain embodiments, the target region is within nucleobase 7366 and nucleobase 7383 of SEQ ID NO.: 2. In certain embodiments, the target region is within nucleobase 7370 and nucleobase 7387 of SEQ ID NO.: 2. In certain embodiments, the target region is within nucleobase 7371 and nucleobase 7388 of SEQ ID NO.: 2. In certain embodiments, the target region is within nucleobase 7387 and nucleobase 7404 of SEQ ID NO.: 2. In certain embodiments, the target region is within nucleobase 7394 and nucleobase 7411 of SEQ ID NO.: 2. In certain embodiments, the target region is within nucleobase 7454 and nucleobase 7471 of SEQ ID NO.: 2. In certain embodiments, the target region is within nucleobase 7462 and nucleobase 7483 of SEQ ID NO.: 2. In certain embodiments, the target region is within nucleobase 7462 and nucleobase 7479 of SEQ ID NO.: 2. In certain embodiments, the target region is within nucleobase 7466 and nucleobase 7483 of SEQ ID NO.: 2.

In certain embodiments, an antisense compound has a nucleobase sequence comprising SEQ ID NO. 3. In certain embodiments, an antisense compound has a nucleobase sequence comprising SEQ ID NO. 4. In certain embodiments, an antisense compound has a nucleobase sequence comprising SEQ ID NO. 5. In certain embodiments, an antisense compound has a nucleobase sequence comprising SEQ ID NO. 6. In certain embodiments, an antisense compound has a nucleobase sequence comprising SEQ ID NO. 7. In certain embodiments, an antisense compound has a nucleobase sequence comprising SEQ ID NO. 8. In certain embodiments, an antisense compound has a nucleobase sequence comprising SEQ ID NO. 9. In certain embodiments, an antisense compound has a nucleobase sequence comprising SEQ ID NO. 10. In certain embodiments, an antisense compound has a nucleobase sequence comprising SEQ ID NO. 11. In certain embodiments, an antisense compound has a nucleobase sequence comprising SEQ ID NO. 12. In certain embodiments, an antisense compound has a nucleobase sequence comprising SEQ ID NO. 13. In certain embodiments, an antisense compound has a nucleobase sequence comprising SEQ ID NO. 14. In certain embodiments, an antisense compound has a nucleobase sequence comprising SEQ ID NO. 15. In certain embodiments, an antisense compound has a nucleobase sequence comprising SEQ ID NO. 16. In certain embodiments, an antisense compound has a nucleobase sequence comprising SEQ ID NO. 17.

In certain embodiments, an antisense compound has a nucleobase sequence comprising SEQ ID NO. 18. In certain embodiments, an antisense compound has a nucleobase sequence comprising SEQ ID NO. 19. In certain embodiments, an antisense compound has a nucleobase sequence comprising SEQ ID NO. 20. In certain embodiments, an antisense compound has a nucleobase sequence comprising SEQ ID NO. 21. In certain embodiments, an antisense compound has a nucleobase sequence comprising SEQ ID NO. 22. In certain embodiments, an antisense compound has a nucleobase sequence comprising SEQ ID NO. 23. In certain embodiments, an antisense compound has a nucleobase sequence comprising SEQ ID NO. 24. In certain embodiments, an antisense compound has a nucleobase sequence comprising SEQ ID NO. 25. In certain embodiments, an antisense compound has a nucleobase sequence comprising SEQ ID NO. 26. In certain embodiments, an antisense compound has a nucleobase sequence comprising SEQ ID NO. 27. In certain embodiments, an antisense compound has a nucleobase sequence comprising SEQ ID NO. 28. In certain embodiments, an antisense compound has a nucleobase sequence comprising SEQ ID NO. 29. In certain embodiments, an antisense compound has a nucleobase sequence comprising SEQ ID NO. 30. In certain embodiments, an antisense compound has a nucleobase sequence comprising SEQ ID NO. 31. In certain embodiments, an antisense compound has a nucleobase sequence comprising SEQ ID NO. 32.

In certain embodiments, an antisense compound has a nucleobase sequence comprising SEQ ID NO. 33. In certain embodiments, an antisense compound has a nucleobase sequence comprising SEQ ID NO.3 4. In certain embodiments, an antisense compound has a nucleobase sequence comprising SEQ ID NO. 35. In certain embodiments, an antisense compound has a nucleobase sequence comprising SEQ ID NO. 36. In certain embodiments, an antisense compound has a nucleobase sequence comprising SEQ ID NO. 37. In certain embodiments, an antisense compound has a nucleobase sequence comprising SEQ ID NO. 38. In certain embodiments, an antisense compound has a nucleobase sequence comprising SEQ ID NO. 39. In certain embodiments, an antisense compound has a nucleobase sequence comprising SEQ ID NO. 40. In certain embodiments, an antisense compound has a nucleobase sequence comprising SEQ ID NO. 41. In certain embodiments, an antisense compound has a nucleobase sequence comprising SEQ ID NO. 42. In certain embodiments, an antisense compound has a nucleobase sequence comprising SEQ ID NO. 43. In certain embodiments, an antisense compound has a nucleobase sequence comprising SEQ ID NO. 44. In certain embodiments, an antisense compound has a nucleobase sequence comprising SEQ ID NO. 45. In certain embodiments, an antisense compound has a nucleobase sequence comprising SEQ ID NO. 46. In certain embodiments, an antisense compound has a nucleobase sequence comprising SEQ ID NO. 47.

In certain embodiments, an antisense compound has a nucleobase sequence comprising SEQ ID NO. 48. In certain embodiments, an antisense compound has a nucleobase sequence comprising SEQ ID NO. 49. In certain embodiments, an antisense compound has a nucleobase sequence comprising SEQ ID NO. 50. In certain embodiments, an antisense compound has a nucleobase sequence comprising SEQ ID NO. 51. In certain embodiments, an antisense compound has a nucleobase sequence comprising SEQ ID NO. 52. In certain embodiments, an antisense compound has a nucleobase sequence comprising SEQ ID NO. 53. In certain embodiments, an antisense compound has a nucleobase sequence comprising SEQ ID NO. 54. In certain embodiments, an antisense compound has a nucleobase sequence comprising SEQ ID NO. 55. In certain embodiments, an antisense compound has a nucleobase sequence comprising SEQ ID NO. 56. In certain embodiments, an antisense compound has a nucleobase sequence comprising SEQ ID NO. 57. In certain embodiments, an antisense compound has a nucleobase sequence comprising SEQ ID NO. 58. In certain embodiments, an antisense compound has a nucleobase sequence comprising SEQ ID NO. 59. In certain embodiments, an antisense compound has a nucleobase sequence comprising SEQ ID NO. 60.

In certain embodiments, an antisense compound has a nucleobase sequence consisting of SEQ ID NO. 3. In certain embodiments, an antisense compound has a nucleobase sequence consisting of SEQ ID NO. 4. In certain embodiments, an antisense compound has a nucleobase sequence consisting of SEQ ID NO. 5. In certain embodiments, an antisense compound has a nucleobase sequence consisting of SEQ ID NO. 6. In certain embodiments, an antisense compound has a nucleobase sequence consisting of SEQ ID NO. 7. In certain embodiments, an antisense compound has a nucleobase sequence consisting of SEQ ID NO. 8. In certain embodiments, an antisense compound has a nucleobase sequence consisting of SEQ ID NO. 9. In certain embodiments, an antisense compound has a nucleobase sequence consisting of SEQ ID NO. 10. In certain embodiments, an antisense compound has a nucleobase sequence consisting of SEQ ID NO. 11. In certain embodiments, an antisense compound has a nucleobase sequence consisting of SEQ ID NO. 12. In certain embodiments, an antisense compound has a nucleobase sequence consisting of SEQ ID NO. 13. In certain embodiments, an antisense compound has a nucleobase sequence consisting of SEQ ID NO. 14. In certain embodiments, an antisense compound has a nucleobase sequence consisting of SEQ ID NO. 15. In certain embodiments, an antisense compound has a nucleobase sequence consisting of SEQ ID NO. 16. In certain embodiments, an antisense compound has a nucleobase sequence consisting of SEQ ID NO. 17.

In certain embodiments, an antisense compound has a nucleobase sequence consisting of SEQ ID NO. 18. In certain embodiments, an antisense compound has a nucleobase sequence consisting of SEQ ID NO. 19. In certain embodiments, an antisense compound has a nucleobase sequence consisting of SEQ ID NO. 20. In certain embodiments, an antisense compound has a nucleobase sequence consisting of SEQ ID NO. 21. In certain embodiments, an antisense compound has a nucleobase sequence consisting of SEQ ID NO. 22. In certain embodiments, an antisense compound has a nucleobase sequence consisting of SEQ ID NO. 23. In certain embodiments, an antisense compound has a nucleobase sequence consisting of SEQ ID NO. 24. In certain embodiments, an antisense compound has a nucleobase sequence consisting of SEQ ID NO. 25. In certain embodiments, an antisense compound has a nucleobase sequence consisting of SEQ ID NO. 26. In certain embodiments, an antisense compound has a nucleobase sequence consisting of SEQ ID NO. 27. In certain embodiments, an antisense compound has a nucleobase sequence consisting of SEQ ID NO. 28. In certain embodiments, an antisense compound has a nucleobase sequence consisting of SEQ ID NO. 29. In certain embodiments, an antisense compound has a nucleobase sequence consisting of SEQ ID NO. 30. In certain embodiments, an antisense compound has a nucleobase sequence consisting of SEQ ID NO. 31. In certain embodiments, an antisense compound has a nucleobase sequence consisting of SEQ ID NO. 32.

In certain embodiments, an antisense compound has a nucleobase sequence consisting of SEQ ID NO. 33. In certain embodiments, an antisense compound has a nucleobase sequence consisting of SEQ ID NO.3 4. In certain embodiments, an antisense compound has a nucleobase sequence consisting of SEQ ID NO. 35. In certain embodiments, an antisense compound has a nucleobase sequence consisting of SEQ ID NO. 36. In certain embodiments, an antisense compound has a nucleobase sequence consisting of SEQ ID NO. 37. In certain embodiments, an antisense compound has a nucleobase sequence consisting of SEQ ID NO. 38. In certain embodiments, an antisense compound has a nucleobase sequence consisting of SEQ ID NO. 39. In certain embodiments, an antisense compound has a nucleobase sequence consisting of SEQ ID NO. 40. In certain embodiments, an antisense compound has a nucleobase sequence consisting of SEQ ID NO. 41. In certain embodiments, an antisense compound has a nucleobase sequence consisting of SEQ ID NO. 42. In certain embodiments, an antisense compound has a nucleobase sequence consisting of SEQ ID NO. 43. In certain embodiments, an antisense compound has a nucleobase sequence consisting of SEQ ID NO. 44. In certain embodiments, an antisense compound has a nucleobase sequence consisting of SEQ ID NO. 45. In certain embodiments, an antisense compound has a nucleobase sequence consisting of SEQ ID NO. 46. In certain embodiments, an antisense compound has a nucleobase sequence consisting of SEQ ID NO. 47.

In certain embodiments, an antisense compound has a nucleobase sequence consisting of SEQ ID NO. 48. In certain embodiments, an antisense compound has a nucleobase sequence consisting of SEQ ID NO. 49. In certain embodiments, an antisense compound has a nucleobase sequence consisting of SEQ ID NO. 50. In certain embodiments, an antisense compound has a nucleobase sequence consisting of SEQ ID NO. 51. In certain embodiments, an antisense compound has a nucleobase sequence consisting of SEQ ID NO. 52. In certain embodiments, an antisense compound has a nucleobase sequence consisting of SEQ ID NO. 53. In certain embodiments, an antisense compound has a nucleobase sequence consisting of SEQ ID NO. 54. In certain embodiments, an antisense compound has a nucleobase sequence consisting of SEQ ID NO. 55. In certain embodiments, an antisense compound has a nucleobase sequence consisting of SEQ ID NO. 56. In certain embodiments, an antisense compound has a nucleobase sequence consisting of SEQ ID NO. 57. In certain embodiments, an antisense compound has a nucleobase sequence consisting of SEQ ID NO. 58. In certain embodiments, an antisense compound has a nucleobase sequence consisting of SEQ ID NO. 59. In certain embodiments, an antisense compound has a nucleobase sequence consisting of SEQ ID NO. 60.

In certain embodiments, an antisense oligonucleotide modulates splicing of a pre-mRNA. In certain embodiments, an antisense oligonucleotide modulates splicing a CLN3 pre-mRNA. In certain embodiments, an antisense oligonucleotide increases the amount of CLN3 mRNA. In certain embodiments, an antisense oligonucleotide increases the exclusion of exon 6 in CLN3 mRNA. In certain embodiments, an antisense oligonucleotide decreases the inclusion of exon 6 in CLN3 mRNA. In certain embodiments, an antisense oligonucleotide increases the exclusion of exon 9 in CLN3 mRNA. In certain embodiments, an antisense oligonucleotide decreases the inclusion of exon 9 in CLN3 mRNA.

In certain embodiments, an antisense oligonucleotide comprises ISIS NO. 616709 (SEQ ID NO: 20). In certain embodiments, an antisense oligonucleotide consists of ISIS NO. 616709.

In certain embodiments, an antisense oligonucleotide promotes skipping of exon 6. In certain embodiments, an antisense oligonucleotide promotes skipping of exon 6 of a *CLN3*Δ*78* transcript. In certain embodiments, an antisense oligonucleotide promotes skipping of exon 9. In certain embodiments, an antisense oligonucleotide promotes skipping of exon 9 of a *CLN3*Δ*78* transcript.

In certain embodiments, an antisense oligonucleotide comprises the nucleobase sequence and modification motif of ISIS NO. 688555. In certain embodiments, an antisense oligonucleotide consists of the nucleobase sequence and modification motif of ISIS NO. 688555. In certain embodiments, an antisense oligonucleotide comprises the nucleobase sequence and modification motif of ISIS NO. 688559. In certain embodiments, an antisense oligonucleotide consists of the nucleobase sequence and modification motif of ISIS NO. 688559. In certain embodiments, an antisense oligonucleotide comprises the nucleobase sequence and modification motif of ISIS NO. 688595. In certain embodiments, an antisense oligonucleotide consists of the nucleobase sequence and modification motif of ISIS NO. 688595.

In certain embodiments, an antisense compound has a nucleobase sequence consisting of SEQ ID NO. 63. In certain embodiments, an antisense compound has a nucleobase sequence consisting of SEQ ID NO. 64. In certain embodiments, an antisense compound has a nucleobase sequence consisting of SEQ ID NO. 65. In certain embodiments, an antisense compound has a nucleobase sequence consisting of SEQ ID NO. 66. In certain embodiments, an antisense compound has a nucleobase sequence consisting of SEQ ID NO. 67. In certain embodiments, an antisense compound has a nucleobase sequence consisting of SEQ ID NO. 68. In certain embodiments, an antisense compound has a nucleobase sequence consisting of SEQ ID NO. 69. In certain embodiments, an antisense compound has a nucleobase sequence consisting of SEQ ID NO. 70. In certain embodiments, an antisense compound has a nucleobase sequence consisting of SEQ ID NO. 71. In certain embodiments, an antisense compound has a nucleobase sequence consisting of SEQ ID NO. 72. In certain embodiments, an antisense compound has a nucleobase sequence consisting of SEQ ID NO. 73. In certain embodiments, an antisense compound has a nucleobase sequence consisting of SEQ ID NO. 74. In certain embodiments, an antisense compound has a nucleobase sequence consisting of SEQ ID NO. 75. In certain embodiments, an antisense compound has a nucleobase sequence consisting of SEQ ID NO. 76. In certain embodiments, an antisense compound has a nucleobase sequence consisting of SEQ ID NO. 77. In certain embodiments, an antisense compound has a nucleobase sequence consisting of SEQ ID NO. 78. In certain embodiments, an antisense compound has a nucleobase sequence consisting of SEQ ID NO. 79. In certain embodiments, an antisense compound has a nucleobase sequence consisting of SEQ ID NO. 80. In certain embodiments, an antisense compound has a nucleobase sequence consisting of SEQ ID NO. 81. In certain embodiments, an antisense compound has a nucleobase sequence consisting of SEQ ID NO. 82. In certain embodiments, an antisense compound has a nucleobase sequence consisting of SEQ ID NO. 83 In certain embodiments, an antisense compound has a nucleobase sequence consisting of SEQ ID NO. 84. In certain embodiments, an antisense compound has a nucleobase sequence consisting of SEQ ID NO. 85. In certain embodiments, an antisense compound has a nucleobase sequence consisting of SEQ ID NO. 86. In certain embodiments, an antisense compound has a nucleobase sequence consisting of SEQ ID NO. 87. In certain embodiments, an antisense compound has a nucleobase sequence consisting of SEQ ID NO. 88. In certain embodiments, an antisense compound has a nucleobase sequence consisting of SEQ ID NO. 89. In certain embodiments, an antisense compound has a nucleobase sequence consisting of SEQ ID NO. 90.

In certain embodiments, an antisense compound has a nucleobase sequence consisting of SEQ ID NO. 91. In certain embodiments, an antisense compound has a nucleobase sequence consisting of SEQ ID NO. 92. In certain embodiments, an antisense compound has a nucleobase sequence consisting of SEQ ID NO. 93. In certain embodiments, an antisense compound has a nucleobase sequence consisting of SEQ ID NO. 94. In certain embodiments, an antisense compound has a nucleobase sequence consisting of SEQ ID NO. 95. In certain embodiments, an antisense compound has a nucleobase sequence consisting of SEQ ID NO. 96. In certain embodiments, an antisense compound has a nucleobase sequence consisting of SEQ ID NO. 97. In certain embodiments, an antisense compound has a nucleobase sequence consisting of SEQ ID NO. 98. In certain embodiments, an antisense compound has a nucleobase sequence consisting of SEQ ID NO. 99. In certain embodiments, an antisense compound has a nucleobase sequence consisting of SEQ ID NO. 100. In certain embodiments, an antisense compound has a nucleobase sequence consisting of SEQ ID NO. 101.

In certain embodiments, an antisense compound has a nucleobase sequence consisting of SEQ ID NO. 102. In certain embodiments, an antisense compound has a nucleobase sequence consisting of SEQ ID NO. 103. In certain embodiments, an antisense compound has a nucleobase sequence consisting of SEQ ID NO. 104. In certain embodiments, an antisense compound has a nucleobase sequence consisting of SEQ ID NO. 105. In certain embodiments, an antisense compound has a nucleobase sequence consisting of SEQ ID NO. 106. In certain embodiments, an antisense compound has a nucleobase sequence consisting of SEQ ID NO. 107. In certain embodiments, an antisense compound has a nucleobase sequence consisting of SEQ ID NO. 108. In certain embodiments, an antisense compound has a nucleobase sequence consisting of SEQ ID NO. 109. In certain embodiments, an antisense compound has a nucleobase sequence consisting of SEQ ID NO. 110.

In certain embodiments, an antisense compound has a nucleobase sequence consisting of SEQ ID NO. 111. In certain embodiments, an antisense compound has a nucleobase sequence consisting of SEQ ID NO. 112. In certain embodiments, an antisense compound has a nucleobase sequence consisting of SEQ ID NO. 113. In certain embodiments, an antisense compound has a nucleobase sequence consisting of SEQ ID NO. 114. In certain embodiments, an antisense compound has a nucleobase sequence consisting of SEQ ID NO. 115. In certain embodiments, an antisense compound has a nucleobase sequence consisting of SEQ ID NO. 116. In certain embodiments, an antisense compound has a nucleobase sequence consisting of SEQ ID NO. 117. In certain embodiments, an antisense compound has a nucleobase sequence consisting of SEQ ID NO. 118. In certain embodiments, an antisense compound has a nucleobase sequence consisting of SEQ ID NO. 119. In certain embodiments, an antisense compound has a nucleobase sequence consisting of SEQ ID NO. 120. In certain embodiments, an antisense compound has a nucleobase sequence consisting of SEQ ID NO. 121. In certain embodiments, an antisense compound has a nucleobase sequence consisting of SEQ ID NO. 122. In certain embodiments, an antisense compound has a nucleobase sequence consisting of SEQ ID NO. 123. In certain embodiments, an antisense compound has a nucleobase sequence consisting of SEQ ID NO. 124. In certain embodiments, an antisense compound has a nucleobase sequence consisting of SEQ ID NO. 125. In certain embodiments, an antisense compound has a nucleobase sequence consisting of SEQ ID NO. 126. In certain embodiments, an antisense compound has a nucleobase sequence consisting of SEQ ID NO. 127.

### Certain Pharmaceutical Compositions

In certain embodiments, the present invention provides pharmaceutical compositions comprising one or more antisense compound. In certain embodiments, such pharmaceutical composition comprises a suitable pharmaceutically acceptable diluent or carrier. In certain embodiments, a pharmaceutical composition comprises a sterile saline solution and one or more antisense compound. In certain embodiments, such pharmaceutical composition consists of a sterile saline solution and one or more antisense compound. In certain embodiments, the sterile saline is pharmaceutical grade saline. In certain embodiments, a pharmaceutical composition comprises one or more antisense compound and sterile water. In certain embodiments, a pharmaceutical composition consists of one or more antisense compound and sterile water. In certain embodiments, the sterile saline is pharmaceutical grade water. In certain embodiments, a pharmaceutical composition comprises one or more antisense compound and phosphate-buffered saline (PBS). In certain embodiments, a pharmaceutical composition consists of one or more antisense compound and sterile phosphate-buffered saline (PBS). In certain embodiments, the sterile saline is pharmaceutical grade PBS.

In certain embodiments, antisense compounds may be admixed with pharmaceutically acceptable active and/or inert substances for the preparation of pharmaceutical compositions or formulations. Compositions and methods for the formulation of pharmaceutical compositions depend on a number of criteria, including, but not limited to, route of administration, extent of disease, or dose to be administered. Pharmaceutical compositions comprising antisense compounds encompass any pharmaceutically acceptable salts, esters, or salts of such esters. In certain embodiments, pharmaceutical compositions comprising antisense compounds comprise one or more oligonucleotide which, upon administration to an animal, including a human, is capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts.

A prodrug can include the incorporation of additional nucleosides at one or both ends of an oligomeric compound which are cleaved by endogenous nucleases within the body, to form the active antisense oligomeric compound.

Lipid moieties have been used in nucleic acid therapies in a variety of methods. In certain such methods, the nucleic acid is introduced into preformed liposomes or lipoplexes made of mixtures of cationic lipids and neutral lipids. In certain methods, DNA complexes with mono- or poly-cationic lipids are formed without the presence of a neutral lipid. In certain embodiments, a lipid moiety is selected to increase distribution of a pharmaceutical agent to a particular cell or tissue. In certain embodiments, a lipid moiety is selected to increase distribution of a pharmaceutical agent to fat tissue. In certain embodiments, a lipid moiety is selected to increase distribution of a pharmaceutical agent to muscle tissue.

In certain embodiments, pharmaceutical compositions provided herein comprise one or more modified oligonucleotides and one or more excipients. In certain such embodiments, excipients are selected from water, salt solutions, alcohol, polyethylene glycols, gelatin, lactose, amylase, magnesium stearate, talc, silicic acid, viscous paraffin, hydroxymethylcellulose and polyvinylpyrrolidone.

In certain embodiments, a pharmaceutical composition provided herein comprises a delivery system. Examples of delivery systems include, but are not limited to, liposomes and emulsions. Certain delivery systems are useful for preparing certain pharmaceutical compositions including those comprising hydrophobic compounds. In certain embodiments, certain organic solvents such as dimethylsulfoxide are used.

In certain embodiments, a pharmaceutical composition provided herein comprises one or more tissue-specific delivery molecules designed to deliver the one or more pharmaceutical agents of the present invention to specific tissues or cell types. For example, in certain embodiments, pharmaceutical compositions include liposomes coated with a tissue-specific antibody.

In certain embodiments, a pharmaceutical composition provided herein comprises a co-solvent system. Certain of such co-solvent systems comprise, for example, benzyl alcohol, a nonpolar surfactant, a water-miscible organic polymer, and an aqueous phase. In certain embodiments, such co-solvent systems are used for hydrophobic compounds. A non-limiting example of such a co-solvent system is the VPD co-solvent system, which is a solution of absolute ethanol comprising 3% w/v benzyl alcohol, 8% w/v of the nonpolar surfactant Polysorbate 80™ and 65% w/v polyethylene glycol 300. The proportions of such co-solvent systems may be varied considerably without significantly altering their solubility and toxicity characteristics. Furthermore, the identity of co-solvent components may be varied: for example, other surfactants may be used instead of Polysorbate 80™; the fraction size of polyethylene glycol may be varied; other biocompatible polymers may replace polyethylene glycol, e.g., polyvinyl pyrrolidone; and other sugars or polysaccharides may substitute for dextrose.

In certain embodiments, a pharmaceutical composition provided herein is prepared for oral administration. In certain embodiments, pharmaceutical compositions are prepared for buccal administration.

In certain embodiments, a pharmaceutical composition is prepared for administration by injection (e.g., intravenous, subcutaneous, intramuscular, etc.). In certain of such embodiments, a pharmaceutical composition comprises a carrier and is formulated in aqueous solution, such as water or physiologically compatible buffers such as Hanks's solution, Ringer's solution, or physiological saline buffer. In certain embodiments, other ingredients are included (e.g., ingredients that aid in solubility or serve as preservatives). In certain embodiments, injectable suspensions are prepared using appropriate liquid carriers, suspending agents and the like. Certain pharmaceutical compositions for injection are presented in unit dosage form, e.g., in ampoules or in multi-dose containers. Certain pharmaceutical compositions for injection are suspensions, solutions or emulsions in oily or aqueous vehicles, and may contain formulatory agents such as suspending, stabilizing and/or dispersing agents. Certain solvents suitable for use in pharmaceutical compositions for injection include, but are not limited to, lipophilic solvents and fatty oils, such as sesame oil, synthetic fatty acid esters, such as ethyl oleate or triglycerides, and liposomes. Aqueous injection suspensions may contain substances that increase the viscosity of the suspension, such as sodium carboxymethyl cellulose, sorbitol, or dextran. Optionally, such suspensions may also contain suitable stabilizers or agents that increase the solubility of the pharmaceutical agents to allow for the preparation of highly concentrated solutions.

In certain embodiments, a pharmaceutical composition is prepared for transmucosal administration. In certain of such embodiments penetrants appropriate to the barrier to be permeated are used in the formulation. Such penetrants are generally known in the art.

In certain embodiments, a pharmaceutical composition provided herein comprises an oligonucleotide in a therapeutically effective amount. In certain embodiments, the therapeutically effective amount is sufficient to prevent, alleviate or ameliorate symptoms of a disease or to prolong the survival of the subject being treated. Determination of a therapeutically effective amount is well within the capability of those skilled in the art.

In certain embodiments, one or more modified oligonucleotide provided herein is formulated as a prodrug. In certain embodiments, upon *in vivo* administration, a prodrug is chemically converted to the biologically, pharmaceutically or therapeutically more active form of an oligonucleotide. In certain embodiments, prodrugs are useful because they are easier to administer than the corresponding active form. For example, in certain instances, a prodrug may be more bioavailable (e.g., through oral administration) than is the corresponding active form. In certain instances, a prodrug may have improved solubility compared to the corresponding active form. In certain embodiments, prodrugs are less water soluble than the corresponding active form. In certain instances, such prodrugs possess superior transmittal across cell membranes, where water solubility is detrimental to mobility. In certain embodiments, a prodrug is an ester. In certain such embodiments, the ester is metabolically hydrolyzed to carboxylic acid upon administration. In certain instances the carboxylic acid containing compound is the corresponding active form. In certain embodiments, a prodrug comprises a short peptide (polyaminoacid) bound to an acid group. In certain of such embodiments, the peptide is cleaved upon administration to form the corresponding active form.

In certain embodiments, the present invention provides compositions and methods for reducing the amount or activity of a target nucleic acid in a cell. In certain embodiments, the cell is in an animal. In certain embodiments, the animal is a mammal. In certain embodiments, the animal is a rodent. In certain embodiments, the animal is a primate. In certain embodiments, the animal is a non-human primate. In certain embodiments, the animal is a human.

In certain embodiments, the present invention provides methods of administering a pharmaceutical composition comprising an oligomeric compound of the present invention to an animal. Suitable administration routes include, but are not limited to, oral, rectal, transmucosal, intestinal, enteral, topical, suppository, through inhalation, intrathecal, intracerebroventricular, intraperitoneal, intranasal, intratumoral, and parenteral (e.g., intravenous, intramuscular, intramedullary, and subcutaneous). In certain embodiments, pharmaceutical intrathecals are administered to achieve local rather than systemic exposures. For example, pharmaceutical compositions may be injected directly in the area of desired effect (e.g., into the ears).

In certain embodiments, a pharmaceutical composition is administered to an animal having at least one symptom associated with Batten Disease. In certain embodiments, such administration results in amelioration of at least one symptom. In certain embodiments, administration of a pharmaceutical composition to an animal results in an increase in functional CLN3 protein in a cell. In certain embodiments, the administration of certain antisense oligonucleotides delays the onset of Batten Disease. In certain embodiments, the administration of certain antisense oligonucleotides prevents the onset of Batten Disease. In certain embodiments, the administration of certain antisense oligonucleotides rescues cellular phenotype.

### Nonlimiting disclosure and incorporation by reference

While certain compounds, compositions and methods described herein have been described with specificity in accordance with certain embodiments, the following examples serve only to illustrate the compounds described herein and are not intended to limit the same. Each of the references, GenBank accession numbers, and the like recited in the present application is incorporated herein by reference in its entirety.

Although the sequence listing accompanying this filing identifies each sequence as either "RNA" or "DNA" as required, in reality, those sequences may be modified with any combination of chemical modifications. One of skill in the art will readily appreciate that such designation as "RNA" or "DNA" to describe modified oligonucleotides is, in certain instances, arbitrary. For example, an oligonucleotide comprising a nucleoside comprising a 2'-OH sugar moiety and a thymine base could be described as a DNA having a modified sugar (2'-OH for the natural 2'-H of DNA) or as an RNA having a modified base (thymine (methylated uracil) for natural uracil of RNA).

Accordingly, nucleic acid sequences provided herein, including, but not limited to those in the sequence listing, are intended to encompass nucleic acids containing any combination of natural or modified RNA and/or DNA, including, but not limited to such nucleic acids having modified nucleobases. By way of further example and without limitation, an oligomeric compound having the nucleobase sequence "ATCGATCG" encompasses any oligomeric compounds having such nucleobase sequence, whether modified or unmodified, including, but not limited to, such compounds comprising RNA bases, such as those having sequence "AUCGAUCG" and those having some DNA bases and some RNA bases such as "AUCGATCG" and oligomeric compounds having other modified or naturally occurring bases, such as "AT^{me}CGAUCG," wherein ^{me}C indicates a cytosine base comprising a methyl group at the 5-position.

### Examples

The following examples illustrate certain embodiments of the present invention and are not limiting. Moreover, where specific embodiments are provided, the inventors have contemplated generic application of those specific embodiments. For example, disclosure of an oligonucleotide having a particular motif provides reasonable support for additional oligonucleotides having the same or similar motif. And, for example, where a particular high-affinity modification appears at a particular position, other high-affinity modifications at the same position are considered suitable, unless otherwise indicated.

### Example 1: Antisense Oligonucleotide Synthesis

The preparation of nucleoside phosphoramidites is performed following procedures that are extensively illustrated in the art such as but not limited to US Patent 6,426,220 and published PCT application WO 02/36743.

### Oligonucleotide and oligonucleoside synthesis

The oligomeric compounds used in accordance with this invention may be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives.

Oligonucleotides: Unsubstituted and substituted phosphodiester (P=O) oligonucleotides are synthesized on an automated DNA synthesizer (Applied Biosystems model 394) using standard phosphoramidite chemistry with oxidation by iodine.

Phosphorothioates (P=S) are synthesized similar to phosphodiester oligonucleotides with the following exceptions: thiation was effected by utilizing a 10% w/v solution of 3,H-1,2-benzodithiole-3-one 1,1-dioxide in acetonitrile for the oxidation of the phosphite linkages. The thiation reaction step time was increased to 180 sec and preceded by the normal capping step. After cleavage from the CPG column and deblocking in concentrated ammonium hydroxide at 55°C (12-16 hr), the oligonucleotides were recovered by precipitating with >3 volumes of ethanol from a 1 M NH₄OAc solution. Phosphinate oligonucleotides are prepared as described in U.S. Patent 5,508,270.

Alkyl phosphonate oligonucleotides are prepared as described in U.S. Patent 4,469,863.

3'-Deoxy-3'-methylene phosphonate oligonucleotides are prepared as described in U.S. Patents 5,610,289 or 5,625,050.

Phosphoramidite oligonucleotides are prepared as described in U.S. Patent, 5,256,775 or U.S. Patent 5,366,878.

Alkylphosphonothioate oligonucleotides are prepared as described in published PCT applications PCT/US94/00902 and PCT/US93/06976 (published as WO 94/17093 and WO 94/02499, respectively).

3'-Deoxy-3'-amino phosphoramidate oligonucleotides are prepared as described in U.S. Patent 5,476,925.

Phosphotriester oligonucleotides are prepared as described in U.S. Patent 5,023,243.

Borano phosphate oligonucleotides are prepared as described in U.S. Patents 5,130,302 and 5,177,198.

Oligonucleosides: Methylenemethylimino linked oligonucleosides, also identified as MMI linked oligonucleosides, methylenedimethylhydrazo linked oligonucleosides, also identified as MDH linked oligonucleosides, and methylenecarbonylamino linked oligonucleosides, also identified as amide-3 linked oligonucleosides, and methyleneaminocarbonyl linked oligonucleosides, also identified as amide-4 linked oligonucleosides, as well as mixed backbone oligomeric compounds having, for instance, alternating MMI and P=O or P=S linkages are prepared as described in U.S. Patents 5,378,825, 5,386,023, 5,489,677, 5,602,240 and 5,610,289.

Formacetal and thioformacetal linked oligonucleosides are prepared as described in U.S. Patents 5,264,562 and 5,264,564.

Ethylene oxide linked oligonucleosides are prepared as described in U.S. Patent 5,223,618.

### Oligonucleotide Isolation

After cleavage from the controlled pore glass solid support and deblocking in concentrated ammonium hydroxide at 55°C for 12-16 hours, the oligonucleotides or oligonucleosides are recovered by precipitation out of 1 M NH₄OAC with >3 volumes of ethanol. Synthesized oligonucleotides were analyzed by electrospray mass spectroscopy (molecular weight determination) and by capillary gel electrophoresis and judged to be at least 70% full length material. The relative amounts of phosphorothioate and phosphodiester linkages obtained in the synthesis was determined by the ratio of correct molecular weight relative to the -16 amu product (+/-32 +/-48). For some studies oligonucleotides were purified by HPLC, as described by Chiang et al., J. Biol. Chem. 1991, 266, 18162-18171. Results obtained with HPLC-purified material were similar to those obtained with non-HPLC purified material.

### Oligonucleotide Synthesis - 96 Well Plate Format

Oligonucleotides can be synthesized via solid phase P(III) phosphoramidite chemistry on an automated synthesizer capable of assembling 96 sequences simultaneously in a 96-well format. Phosphodiester internucleotide linkages are afforded by oxidation with aqueous iodine. Phosphorothioate internucleotide linkages are generated by sulfurization utilizing 3,H-1,2 benzodithiole-3-one 1,1 dioxide (Beaucage Reagent) in anhydrous acetonitrile. Standard base-protected beta-cyanoethyl-diiso-propyl phosphoramidites are purchased from commercial vendors (e.g. PE-Applied Biosystems, Foster City, CA, or Pharmacia, Piscataway, NJ). Non-standard nucleosides are synthesized as per standard or patented methods. They are utilized as base protected beta-cyanoethyldiisopropyl phosphoramidites.

Oligonucleotides are cleaved from support and deprotected with concentrated NH₄OH at elevated temperature (55-60°C) for 12-16 hours and the released product then dried *in vacuo.* The dried product is then re-suspended in sterile water to afford a master plate from which all analytical and test plate samples are then diluted utilizing robotic pipettors.

### Oligonucleotide Analysis using 96-Well Plate Format

The concentration of oligonucleotide in each well is assessed by dilution of samples and UV absorption spectroscopy. The full-length integrity of the individual products is evaluated by capillary electrophoresis (CE) in either the 96-well format (Beckman P/ACE™ MDQ) or, for individually prepared samples, on a commercial CE apparatus (e.g., Beckman P/ACE™ 5000, ABI 270). Base and backbone composition is confirmed by mass analysis of the oligomeric compounds utilizing electrospray-mass spectroscopy. All assay test plates are diluted from the master plate using single and multi-channel robotic pipettors. Plates are judged to be acceptable if at least 85% of the oligomeric compounds on the plate are at least 85% full length.

### Example 2: Expression of CLN3Δ678 and CLN3A78 in vitro

Plasmids comprising a C-terminal FLAG-tagged WT or mutant CLN3 gene were prepared using standard molecular biology techniques. The mutant CLN3 genes contained a deletion of exons 6, 7, and 8 (CLN3Δ678) or deletion of exons 7, 8, and 9 (CLN3Δ789). The WT and mutant CLN3 genes were under control of the CMV promoter. HeLa cells were transfected with plasmids containing WT CLN3, CLN3Δ78, CLN3Δ678, or empty plasmids (mock) using standard methods. 48 hours after transfection, the cells were fixed and stained with anti-FLAG M2 FITC antibody (F4049, Sigma), mounted with Prolong antifade with DAPI (Invitrogen) and visualized under an Olympus FV10i confocal microscope.

While none of the mock or *CLN3*Δ*78* transfected cells exhibited FITC fluorescence, many of the WT CLN3 and CLN3Δ678 transfected cells were fluorescent in the FITC channel. These results indicate that the CLN3Δ78 protein was not visibly expressed, but the wild type CLN3 and CLN3Δ678 proteins were visibly expressed.

### Example 3: Antisense Modulation of CLN3 Transcript Splicing

An antisense oligonucleotide targeted to the exon 6/intron 6 junction of human CLN3 pre-mRNA (MO-ASO ACACAGAACCACACACTCACCACAC, SEQ ID NO: 3) was synthesized by Gene Tools, LLC, and tested for its ability to modulate splicing of CLN3. The ASO targets the exon 6/intron 6 junction of the complement of GENBANK accession number NT_010393.16 truncated from nucleotides 28427600 to 28444620 (SEQ ID NO: 1). The ASO is a uniform morpholino ASO.

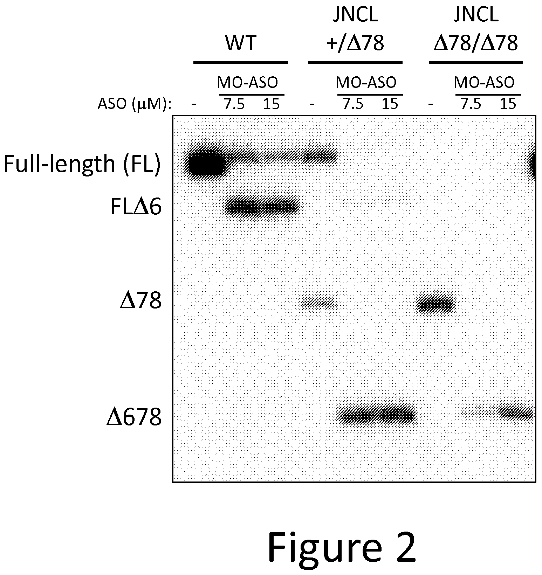

0 µM, 7.5 µM, 15 µM of the morpholino ASO targeted to the exon 6/intron 6 junction (MO-ASO) and Endo-porter transfection reagent (Gene Tools, LLC) were added to fibroblast cells of a JNCL patient homozygous for the CLN3Δ78 mutation, a heterozygous carrier of the CLN3Δ78 mutation, and a family member of the JNCL patient who is homozygous for WT CLN3. After 48 hours, total RNA was then isolated from the cells and CLN3 mRNA was detected via radioactive RT-PCR and PAGE. The gel is shown in Figure 2. Bands were quantitated by densitometry analysis using Image J software, and the results are shown in Table 1 below. In Table 1, 'Ht' means heterozygote, and 'Hm' means homozygote.

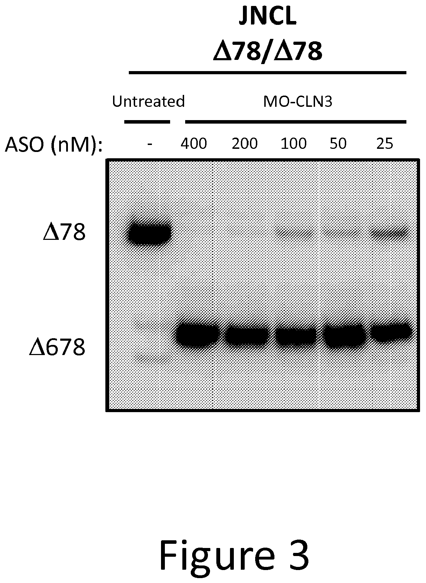

In a similar experiment, increasing concentrations of MO-ASO were incubated with homozygous CLN3Δ78/Δ78 patient fibroblasts. The lowest dose of 25 nM induced % exon 6 skipping, and the highest doses tested induced % exon 6 skipping. The results are shown in Table 2 below, and the gel is shown in Figure 3.

**Table 1: Modulation of Splicing of CLN3 pre-mRNA by MO-ASO as analyzed by RT-PCR**

| Genotype | MO-ASO Concentration | % of all Isoforms Lacking Exon 6 |
|---|---|---|
| WT | 0 µM (untreated control) | 0.0 |
| WT | 7.5 µM | 67.9 |
| WT | 15 µM | 73.7 |
| Ht | 0 µM (untreated control) | 0.0 |
| Ht | 7.5 µM | 92.8 |
| Ht | 15 µM | 93.4 |
| Hm | 0 µM (untreated control) | 0.0 |
| Hm | 7.5 µM | 74.6 |
| Hm | 15 µM | 93.3 |

**Table 2: MO-ASO Dose Response Analysis of CLN3 pre-mRNA Splicing in CLN3Δ78/Δ78 cells**

| MO-ASO concentration | % of all Isoforms Lacking Exon 6 |
|---|---|
| 0 nM (untreated control) | 4.0 |
| 25 nM | 82.9 |
| 50 nM | 94.5 |
| 100 nM | 89.7 |
| 200 nM | 98.4 |
| 400 nM | 99.4 |

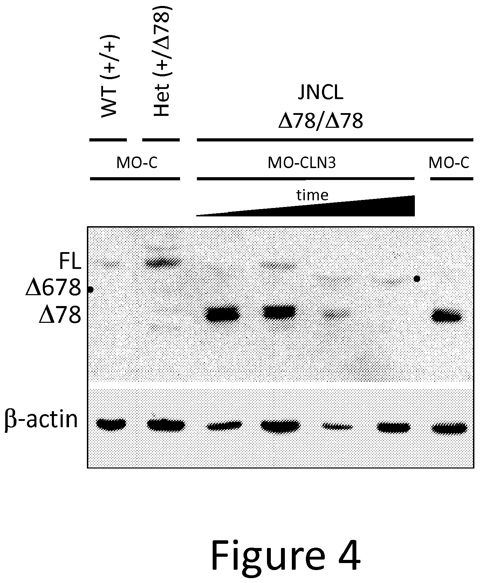

CLN3 protein levels were measured in a similar experiment in which a single concentration (7.5µM) of the MO-ASO or a control morpholino oligonucleotide (MO-C, AGCTGATCATATTCTACCTGGTGCT SEQ ID NO: 62) was used and protein levels in homozygous mutant CLN3Δ78 cells were analyzed at the following 0, 56, 144, or 192 hours following incubation. Protein levels were analyzed by western blot using a rabbit poly-clonal anti-CLN3 antibody (Proteintech) and anti-β-actin as a loading control. The blot is shown in Figure 4.

### Example 4: Design of Antisense Oligonucleotides for Induction of CLN3 Exon 6 Skipping

Antisense oligonucleotides complementary to exon 6 and/or the introns flanking exon 6 of the mouse CLN3 pre-mRNA transcript were synthesized and tested for their ability to modulate splicing of CLN3 by skipping exon 6. The oligonucleotides in Table 3 below are uniformly 2'-MOE modified, and all internucleoside linkages are phosphorothioate linkages. The cytosine bases are 5-methylcytosine. The ASOs target the complement of GENBANK accession number NT_039433.8, truncated from nucleotides 44319075 to 44333955 (SEQ ID NO: 2), and the ASO sequences are listed in Table 3 below. The start and stop sites associated with each oligonucleotide are the 5'- and 3'-positions, respectively, of the portion of SEQ ID NO: 2 that is complementary to the ASO.

**Table 3: Antisense Oligonucleotides for Induction of CLN3 Exon 6 Skipping**

| ISIS No. | Start Site | Stop Site | Sequence (5' to 3') | CLN3 Target Region | SEQ ID NO |
|---|---|---|---|---|---|
| 616693 | 5030 | 5047 | GAGAAGAGATGAGGAGGA | Intron 5 | 4 |
| 616694 | 5034 | 5051 | GGCTGAGAAGAGATGAGG | Intron 5/Exon 6 | 5 |
| 616695 | 5035 | 5052 | GGGCTGAGAAGAGATGAG | Intron 5/Exon 6 | 6 |
| 616696 | 5052 | 5069 | CACTGACGAGCACCCGGG | Exon 6 | 7 |
| 616697 | 5053 | 5070 | CCACTGACGAGCACCCGG | Exon 6 | 8 |
| 616698 | 5054 | 5071 | TCCACTGACGAGCACCCG | Exon 6 | 9 |
| 616699 | 5058 | 5075 | AAACTCCACTGACGAGCA | Exon 6 | 10 |
| 616700 | 5062 | 5079 | GAACAAACTCCACTGACG | Exon 6 | 11 |
| 616701 | 5066 | 5083 | AGCAGAACAAACTCCACT | Exon 6 | 12 |
| 616702 | 5070 | 5087 | TCCCAGCAGAACAAACTC | Exon 6 | 13 |
| 616703 | 5074 | 5091 | AAGCTCCCAGCAGAACAA | Exon 6 | 14 |
| 616704 | 5078 | 5095 | AACAAAGCTCCCAGCAGA | Exon 6 | 15 |
| 616705 | 5082 | 5099 | CCAGAACAAAGCTCCCAG | Exon 6 | 16 |
| 616706 | 5086 | 5103 | GCAACCAGAACAAAGCTC | Exon 6 | 17 |
| 616707 | 5090 | 5107 | GAAGGCAACCAGAACAAA | Exon 6 | 18 |
| 616708 | 5094 | 5111 | GAGAGAAGGCAACCAGAA | Exon 6 | 19 |
| 616709 | 5098 | 5115 | GACTGAGAGAAGGCAACC | Exon 6 | 20 |
| 616710 | 5102 | 5119 | CACTGACTGAGAGAAGGC | Exon 6 | 21 |
| 616711 | 5106 | 5123 | ACCCCACTGACTGAGAGA | Exon 6 | 22 |
| 616712 | 5110 | 5127 | CTTAACCCCACTGACTGA | Exon 6 | 23 |
| 616713 | 5114 | 5131 | CAGGCTTAACCCCACTGA | Exon 6 | 24 |
| 616714 | 5118 | 5135 | CACACAGGCTTAACCCCA | Exon 6 | 25 |
| 616715 | 5122 | 5139 | TCACCACACAGGCTTAAC | Exon 6/Intron 6 | 26 |
| 616716 | 5126 | 5143 | GTACTCACCACACAGGCT | Exon 6/Intron 6 | 27 |
| 616717 | 5130 | 5147 | CTTCGTACTCACCACACA | Exon 6/Intron 6 | 28 |
| 616718 | 5134 | 5151 | CCAGCTTCGTACTCACCA | Exon 6/Intron 6 | 29 |
| 616719 | 5138 | 5155 | TCTCCCAGCTTCGTACTC | Intron 6 | 30 |

### Example 5: Antisense Oligonucleotide Induction of CLN3 Exon 6 Skipping in vitro

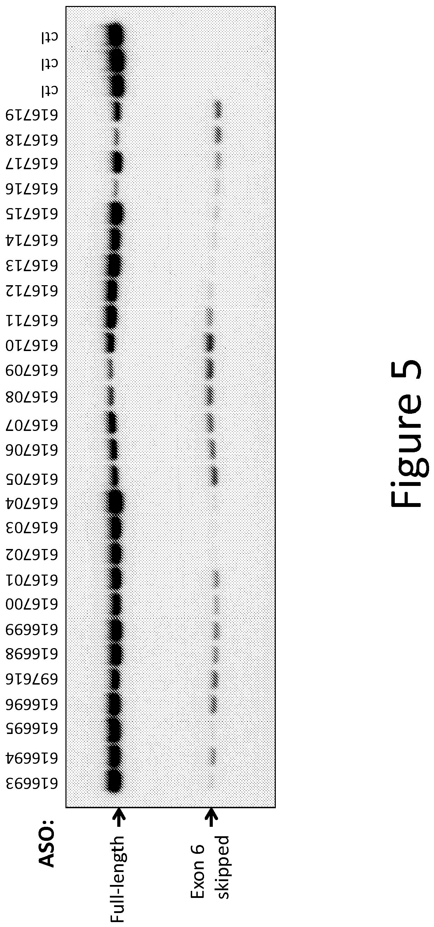

To test the ability of the antisense oligonucleotides to modulate splicing by skipping exon 6, mouse cell line 208ee, derived from C57/B16 mouse kidney, was transfected with 50 nM of the ASOs listed in Table 3 using Lipofectamine 2000 (Invitrogen). Untreated control cells received neither ASO nor Lipofectamine, while mock transfected cells received only Lipofectamine. After 48 hours, total RNA was collected from the cells and RT-PCR was used to identify CLN3 full-length and CLN3Δ6 transcripts. The PCR products were analyzed by PAGE and quantitated by phosphorimager analysis (Typhoon 9400, GE Healthcare). The gel is shown in Figure 5, and the results are shown in Table 4 below as the percentage of exon 6 skipped (Δ6) transcript out of the total full-length (FL) plus exon 6 skipped transcripts. Band intensities are reported in relative fluorescence units (RFU). This example illustrates that many ASOs induce CLN3 exon 6 skipping.

**Table 4: Induction of CLN3 Exon 6 Skipping in vitro**

| ISIS NO | Full-length CLN3 (RFU) | CLN3Δ6 (RFU) | Δ6/(FL + Δ6) (%) |
|---|---|---|---|
| untreated control | 6,823,783 | 762 | 0.01 |
| mock | 5,188,149 | 2,110 | 0.04 |
| 616693 | 6,723,467 | 253,542 | 3.63 |
| 616694 | 5,083,972 | 703,577 | 12.16 |
| 616695 | 6,278,156 | 225,133 | 3.46 |
| 616696 | 5,488,331 | 941,697 | 14.65 |
| 616697 | 3,262,413 | 1,067,086 | 24.65 |
| 616698 | 5,125,340 | 688,277 | 11.84 |
| 616699 | 4,999,204 | 727,472 | 12.70 |
| 616700 | 3,286,457 | 523332 | 13.74 |
| 616701 | 4,054,613 | 653,463 | 13.88 |
| 616702 | 4,047,520 | 73,846 | 1.79 |
| 616703 | 4,452,892 | 96,627 | 2.12 |
| 616704 | 7,603,794 | 152,903 | 1.97 |
| 616705 | 2,177,616 | 1,254,116 | 36.54 |
| 616706 | 2,222,239 | 903,504 | 28.91 |
| 616707 | 2,432,817 | 1,009,358 | 29.32 |
| 616708 | 1,182,124 | 1,120,453 | 48.66 |
| 616709 | 894,622 | 1,501,534 | 62.66 |
| 616710 | 2,315,933 | 1,597,682 | 40.82 |
| 616711 | 4,134,353 | 640,071 | 13.41 |
| 616712 | 3,928,470 | 229,565 | 5.52 |
| 616713 | 5,499,952 | 56,355 | 1.01 |
| 616714 | 3,618,665 | 180,643 | 4.75 |
| 616715 | 5,176,194 | 235,685 | 4.35 |
| 616716 | 692,245 | 335,115 | 32.62 |
| 616717 | 2,855,386 | 512,449 | 15.22 |
| 616718 | 730,706 | 832,631 | 53.26 |
| 616719 | 1,738,032 | 856,673 | 33.02 |

### Example 6: Design of Antisense Oligonucleotides for Induction of CLN3 Exon 9 Skipping

Antisense oligonucleotides complementary to exon 9 and/or the introns flanking exon 9 of the mouse CLN3 pre-mRNA transcript were synthesized and tested for their ability to modulate splicing of CLN3 by skipping exon 9. The oligonucleotides were uniformly modified to contain 2'-MOE, and all internucleoside linkages are phosphorothioate linkages. The cytosine bases are 5-methylcytosine. The ASOs target the complement of GENBANK accession number NT_039433.8 truncated from nucleotides 44319075 to 44333955 (SEQ ID NO: 2), and the ASO sequences are listed in Table 5 below. The start and stop sites associated with each oligonucleotide are the 5'- and 3'-positions, respectively, of the portion of SEQ ID NO: 2 that is complementary to the ASO.

**Table 5: Antisense Oligonucleotides for Induction of CLN3 Exon 9 Skipping**

| 3 | Start Site | Stop Site | Sequence (5' to 3') | CLN3 Target Region | SEQ ID NO |
|---|---|---|---|---|---|
| 616720 | 7338 | 7355 | GGGATGGAATGGAGAAGC | Intron 8 | 31 |
| 616721 | 7342 | 7359 | AGCTGGGATGGAATGGAG | Intron 8/Exon 9 | 32 |
| 616722 | 7346 | 7363 | AAATAGCTGGGATGGAAT | Intron 8/Exon 9 | 33 |
| 616723 | 7350 | 7367 | CAAGAAATAGCTGGGATG | Intron 8/Exon 9 | 34 |
| 616724 | 7354 | 7371 | GCAACAAGAAATAGCTGG | Intron 8/Exon 9 | 35 |
| 616725 | 7358 | 7375 | GTGAGCAACAAGAAATAG | Exon 9 | 36 |
| 616726 | 7362 | 7379 | AGACGTGAGCAACAAGAA | Exon 9 | 37 |
| 616727 | 7366 | 7383 | CAGGAGACGTGAGCAACA | Exon 9 | 38 |
| 616728 | 7370 | 7387 | GGTTCAGGAGACGTGAGC | Exon 9 | 39 |
| 616729 | 7371 | 7388 | GGGTTCAGGAGACGTGAG | Exon 9 | 40 |
| 616730 | 7387 | 7404 | CCCCTCCAGGGTCCAGGG | Exon 9 | 41 |
| 616731 | 7390 | 7407 | TTTCCCCTCCAGGGTCCA | Exon 9 | 42 |
| 616732 | 7394 | 7411 | TCGTTTTCCCCTCCAGGG | Exon 9 | 43 |
| 616733 | 7398 | 7415 | TGCCTCGTTTTCCCCTCC | Exon 9 | 44 |
| 616734 | 7402 | 7419 | TCTCTGCCTCGTTTTCCC | Exon 9 | 45 |
| 616735 | 7406 | 7423 | GCAGTCTCTGCCTCGTTT | Exon 9 | 46 |
| 616736 | 7410 | 7427 | GGCAGCAGTCTCTGCCTC | Exon 9 | 47 |
| 616737 | 7414 | 7431 | GCCGGGCAGCAGTCTCTG | Exon 9 | 48 |
| 616738 | 7418 | 7435 | GGCTGCCGGGCAGCAGTC | Exon 9 | 49 |
| 616739 | 7422 | 7439 | GAGAGGCTGCCGGGCAGC | Exon 9 | 50 |
| 616740 | 7426 | 7443 | CTATGAGAGGCTGCCGGG | Exon 9 | 51 |
| 616741 | 7430 | 7447 | GTGCCTATGAGAGGCTGC | Exon 9 | 52 |
| 616742 | 7434 | 7451 | CTCGGTGCCTATGAGAGG | Exon 9 | 53 |
| 616743 | 7438 | 7455 | GGGTCTCGGTGCCTATGA | Exon 9 | 54 |
| 616744 | 7454 | 7471 | CCTGGCTTTGACTCTGGG | Exon 9/Intron 9 | 55 |
| 616745 | 7458 | 7475 | CCTACCTGGCTTTGACTC | Exon 9/Intron 9 | 56 |
| 616746 | 7462 | 7479 | GCATCCTACCTGGCTTTG | Exon 9/Intron 9 | 57 |
| 616747 | 7466 | 7483 | CTTTGCATCCTACCTGGC | Exon 9/Intron 9 | 58 |
| 616748 | 7470 | 7487 | GGGCCTTTGCATCCTACC | Exon 9/Intron 9 | 59 |
| 616749 | 7474 | 7491 | GGGAGGGCCTTTGCATCC | Intron 9 | 60 |

### Example 7: Antisense Oligonucleotide Induction of CLN3 Exon 9 Skipping in vitro

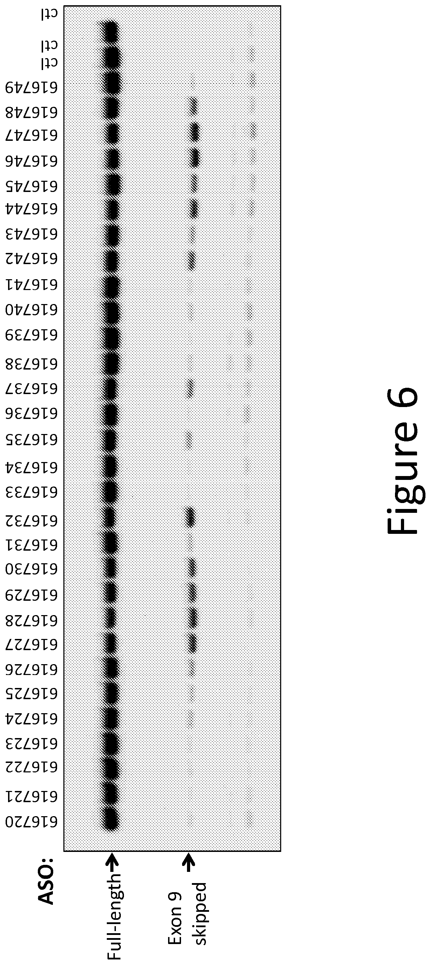

To test the ability of the antisense oligonucleotides to modulate splicing by skipping exon 9, mouse cell line 208ee, derived from C57/B16 mouse kidney, was transfected with 50 nM of the ASOs listed in Table 5 using Lipofectamine 2000 (Invitrogen). Untreated controls cells received neither ASO nor Lipofectamine, while mock transfected cells received only Lipofectamine. After 48 hours, total RNA was collected from the cells and RT-PCR was used to identify CLN3 full-length and CLN3Δ9 transcripts. The PCR products were analyzed by PAGE and quantitated by phosphorimager analysis (Typhoon 9400, GE Healthcare). The gel is shown in Figure 6, and the results are shown in Table 6 as the percentage of exon 9 skipped (Δ9) transcript out of the total full-length (FL) plus exon 9 skipped transcripts. Band intensities are reported in relative fluorescence units (RFU). This example illustrates that many ASOs induce CLN3 exon 9 skipping.

**Table 6: Induction of CLN3 Exon 9 Skipping in vitro**

| ISIS NO | Full-length CLN3 (RFU) | CLN3Δ9 (RFU) | Δ9/(FL + Δ9) (%) |
|---|---|---|---|
| untreated control | 9,181,862 | 123,203 | 1.32 |
| mock | 11,512,749 | 112,061 | 0.96 |
| 616720 | 8,594,979 | 360,150 | 4.02 |
| 616721 | 8,065,276 | 236,557 | 2.85 |
| 616722 | 9,180,522 | 336,620 | 3.54 |
| 616723 | 9,434,304 | 341,373 | 3.49 |
| 616724 | 8,253,838 | 790,632 | 8.74 |
| 616725 | 7,304,964 | 704,209 | 8.79 |
| 616726 | 7,557,368 | 1,175,984 | 13.47 |
| 616727 | 5,415,612 | 3,045,762 | 36.00 |
| 616728 | 4,294,429 | 3,391,413 | 44.13 |
| 616729 | 5,530,907 | 2,760,074 | 33.29 |
| 616730 | 5,419,114 | 2,431,785 | 30.97 |
| 616731 | 8,003,964 | 792,062 | 9.00 |
| 616732 | 5,661,965 | 3,374,571 | 37.34 |
| 616733 | 8,423,448 | 221,903 | 2.57 |
| 616734 | 7,865,161 | 226,765 | 2.80 |
| 616735 | 7,533,174 | 1,036,100 | 12.09 |
| 616736 | 8,566,723 | 261,101 | 2.96 |
| 616737 | 7,177,269 | 1,539,887 | 17.67 |
| 616738 | 9,536,684 | 433,505 | 4.35 |
| 616739 | 10,062,225 | 213,983 | 2.08 |
| 616740 | 9,458,260 | 522,205 | 5.23 |
| 616741 | 9,741,862 | 294,485 | 2.93 |
| 616742 | 6,988,392 | 1,994,800 | 22.21 |
| 616743 | 7,293,927 | 1,125,023 | 13.36 |
| 616744 | 6,063,867 | 2,719,387 | 30.96 |
| 616745 | 7,885,435 | 1,812,122 | 18.69 |
| 616746 | 6,220,132 | 3,850,348 | 38.23 |
| 616747 | 6,181,617 | 3,699,891 | 37.44 |
| 616748 | 7,778,346 | 2,210,461 | 22.13 |
| 616749 | 10,815,904 | 467,192 | 4.14 |

### Example 8: Antisense Oligonucleotide Induction of CLN3 Exon 6 Skipping in Batten Mice in vivo

A mouse model of Batten Disease, in which the CLN3Δ78 deletion is knocked-in to C57/BL6J mice, has been described (Cotman et al., Hum. Molec. Genet. (2002) 11, 2709-2721). The CLN3Δ78/Δ78 homozygous mice exhibit gliosis in the CNS, abnormal gait traces, clasping behavior indicating neurodegeneration, and early PT-10.

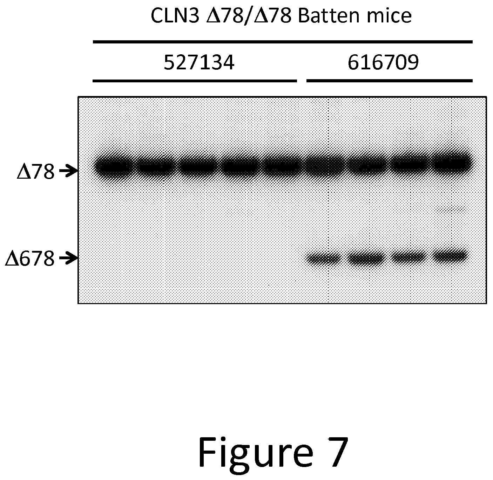

. 20 µg of Isis No. 616709 (see Table 3) or a control ASO, Isis No. 527134 (AGCTGATCATATTCTACC SEQ ID NO: 61) that is uniform 2'-MOE and contains uniform phosphorothioate internucleoside linkages, was administered to groups of six CLN3Δ78/Δ78 mice to test the ability of Isis No. 616709 to induce exon 6 skipping *in vivo.* The ASOs were administered by intracerebroventricular injection (ICV injection) on either post-natal day one or two. Four months after the single ICV injection, the mice were euthanized and RNA was isolated from brain tissue. CLN3Δ78 and CLN3Δ678 transcripts were analyzed by radiolabeled RT-PCR. The PCR products were separated by PAGE and quantitated by phosphorimager analysis (Typhoon 9400, GE Healthcare). A gel is shown in Figure 7, and the results for each group are presented in Table 7 below. As indicated by the results in Table 7 below, ISIS NO. 616709 induced skipping of CLN3 exon 6 in a mouse model of Batten Disease *in vivo.*

**Table 7: Induction of CLN3 Exon 6 Skipping in CLN3Δ78/Δ78 mice in vivo**

| ISIS NO. | Δ678/ (Δ678 + Δ78) (%) | Std Dev |
|---|---|---|
| 527134 | 3.70 | 5.30 |
| 616709 | 33.72 | 7.18 |

### Example 9: Antisense Oligonucleotide Rescue of Motor Coordination in CLN3Δ78/Δ78 Mice

In order to test whether ASOs that induce exon 6 skipping relieve symptoms of Batten Disease, homozygous CLN3Δ78/Δ78 and heterozygous CLN3+/Δ78 mice were divided into mixed gender and male only groups and treated with 20 µg of Isis NO. 616709 (SEQ ID NO. 20; *see* Table 3), control ASO, Isis No. 527134 (SEQ ID NO: 61; *see* Example 8), or no ASO (untreated). The ASOs were administered by intracerebroventricular injection on post-natal day one or two. Two or three months after the single injection, motor coordination of each mouse was measured based on the time each mouse spent on an accelerating rotarod. The results are presented in Tables 8-12 below.

**Table 8: Time Spent on an Accelerating Rotarod by Mixed Gender CLN3Δ78/Δ78 Mice, Age 2 Months**

| Isis No. | Genotype | No. of Mice in Group | Average Time Spent on Rotarod (seconds) | Std Dev. |
|---|---|---|---|---|
| Untreated | CLN3Δ78/Δ78 | 28 | 82.7 | 25.8 |
| 527134 | CLN3Δ78/Δ78 | 21 | 81.5 | 20.8 |
| 616709 | CLN3Δ78/Δ78 | 21 | 84.7 | 14.5 |
| Untreated | CLN3+/Δ78 | 22 | 91.6 | 19.7 |
| 527134 | CLN3+/Δ78 | 17 | 98.2 | 39.2 |
| 616709 | CLN3+/Δ78 | 31 | 99.8 | 22.9 |

**Table 9: Time Spent on an Accelerating Rotarod by Mixed Gender CLN3Δ78/Δ78 Mice, Age 3 Months**

| Isis No. | Genotype | No. of Mice in Group | Average Time Spent on Rotarod (seconds) | Std Dev. |
|---|---|---|---|---|
| Untreated | CLN3Δ78/Δ78 | 9 | 51.8 | 17.6 |
| 527134 | CLN3Δ78/Δ78 | 8 | 66.1 | 15.3 |
| 616709 | CLN3Δ78/Δ78 | 9 | 81.2 | 12.8 |
| Untreated | CLN3+/Δ78 | 8 | 70.3 | 14.6 |
| 527134 | CLN3+/Δ78 | 7 | 68.8 | 13.4 |
| 616709 | CLN3+/Δ78 | 13 | 96.0 | 29.3 |

**Table 10: Time Spent on an Accelerating Rotarod by Male CLN3Δ78/Δ78 Mice, Age 2 Months**

| Isis No. | Genotype | No. of Mice in Group | Average Time Spent on Rotarod (seconds) | Std Dev. |
|---|---|---|---|---|
| Untreated | CLN3Δ78/Δ78 | 13 | 68.1 | 19.3 |
| 527134 | CLN3Δ78/Δ78 | 5 | 74.3 | 15.2 |
| 616709 | CLN3Δ78/Δ78 | 12 | 84.1 | 14.3 |
| Untreated | CLN3+/Δ78 | 12 | 94.0 | 19.7 |
| 527134 | CLN3+/Δ78 | 8 | 108.2 | 47.5 |
| 616709 | CLN3+/Δ78 | 14 | 103.2 | 23.7 |

**Table 11: Time Spent on an Accelerating Rotarod by Male CLN3Δ78/Δ78 Mice, Age 3 Months**

| Isis No. | Genotype | No. of Mice in Group | Average Time Spent on Rotarod (seconds) | Std Dev. |
|---|---|---|---|---|
| Untreated | CLN3Δ78/Δ78 | 4 | 39.3 | 9.5 |
| 527134 | CLN3Δ78/Δ78 | 1 | 36.4 | n/a |
| 616709 | CLN3Δ78/Δ78 | 4 | 75.3 | 9.9 |
| Untreated | CLN3+/Δ78 | 8 | 70.3 | 14.6 |
| 527134 | CLN3+/Δ78 | 3 | 75.4 | 11.8 |
| 616709 | CLN3+/Δ78 | 7 | 117.4 | 15.8 |

The results in Tables 8-11 above indicate that the motor coordination deficit in the CLN3Δ78/Δ78 Batten mice worsens significantly between two and three months of age and is more severe in males than females. Furthermore, treatment with Isis No. 616709, which induces exon 6 skipping, increases the amount of time CLN3Δ78/Δ78 Batten mice are able to spend on an accelerating rotarod, thereby improving a motor coordination deficit that is symptomatic of Batten Disease.

### Example 10: Antisense Oligonucleotide Induction of Human CLN3 Exon 6 Skipping in vitro

Antisense oligonucleotides complementary to exon 6 and/or the introns flanking exon 6 of the human CLN3 pre-mRNA transcript were synthesized and tested for their ability to modulate splicing of CLN3 by skipping exon 6. The oligonucleotides in Table 12 below are uniformly 2'-MOE modified, and all internucleoside linkages are phosphorothioate linkages. The cytosine bases are 5-methylcytosine. The ASOs target the complement of GENBANK accession number NT_010393.16, truncated from nucleotides 28427600 to 28444620 (SEQ ID NO: 1), and the ASO sequences are listed in Table 12 below. The start and stop sites associated with each oligonucleotide are the 5'- and 3'-positions, respectively, of the portion of SEQ ID NO: 1 that is complementary to the ASO.

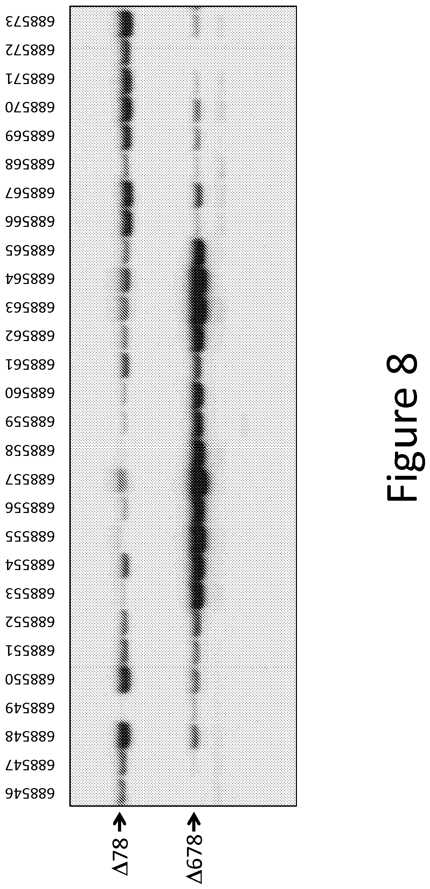

To test the ability of the antisense oligonucleotides to modulate splicing by skipping exon 6, human JNCL patient fibroblasts that are homozygous for the CLN3Δ78 deletion were transfected with 50 nM of an ASO using Cytofectin transfection reagent. After 48 hours, total RNA was collected from the cells and RT-PCR was used to identify the CLN3Δ78 and CLN3Δ678 mRNA transcripts. The PCR products were analyzed by PAGE and quantitated by phosphorimager analysis (Typhoon 9400, GE Healthcare). The gel is shown in Figure 8, and the results are shown in Table 12 below as the ratios of exon 6, 7, and 8 skipped (Δ678) transcript to exon 7 and 8 skipped (Δ78) transcript. The results below illustrate that many ASOs induced CLN3 exon 6 skipping.

**Table 12: CLN3 Exon 6 Skipping in patient fibroblasts**

| ISIS No. | Start Site | Stop Site | Sequence (5' to 3') | CLN3 Target Region | CLN3Δ678/ CLN3Δ78 | SEQ ID NO |
|---|---|---|---|---|---|---|
| 688546 | 5699 | 5716 | ACTCTCAGCATCTCAGCC | Intron 5 | 0.00 | 63 |
| 688547 | 5704 | 5721 | TCTCTACTCTCAGCATCT | Intron 5 | 0.00 | 64 |
| 688548 | 5709 | 5726 | GTCGGTCTCTACTCTCAG | Intron 5 | 0.25 | 65 |
| 688549 | 5714 | 5731 | GGAAGGTCGGTCTCTACT | Intron 5 | 1.38 | 66 |
| 688550 | 5734 | 5751 | GGTGAGAAGGGAAGGGAG | Intron 5 | 0.37 | 67 |
| 688551 | 5764 | 5781 | TCCCACTGACGAGAACCC | Exon 6 | 0.74 | 68 |
| 688552 | 5769 | 5786 | ACAAATCCCACTGACGAG | Exon 6 | 1.58 | 69 |
| 688553 | 5774 | 5791 | GCAGCACAAATCCCACTG | Exon 6 | 14.77 | 70 |
| 688554 | 5779 | 5796 | TTCCAGCAGCACAAATCC | Exon 6 | 3.68 | 71 |
| 688555 | 5784 | 5801 | GAAGCTTCCAGCAGCACA | Exon 6 | 31.07 | 72 |
| 688556 | 5789 | 5806 | AGGACGAAGCTTCCAGCA | Exon 6 | 9.28 | 73 |
| 688557 | 5794 | 5811 | CAACCAGGACGAAGCTTC | Exon 6 | 13.03 | 74 |
| 688558 | 5799 | 5816 | AAAGGCAACCAGGACGAA | Exon 6 | 24.37 | 75 |
| 688559 | 5804 | 5821 | TGAGAAAAGGCAACCAGG | Exon 6 | 10.29 | 76 |
| 688560 | 5809 | 5826 | CAGAATGAGAAAAGGCAA | Exon 6 | 8.44 | 77 |
| 688561 | 5814 | 5831 | CCCCACAGAATGAGAAAA | Exon 6 | 1.28 | 78 |
| 688562 | 5819 | 5836 | CTGGTCCCCACAGAATGA | Exon 6 | 7.32 | 79 |
| 688563 | 5824 | 5841 | ACAGGCTGGTCCCCACAG | Exon 6 | 8.32 | 80 |
| 688564 | 5829 | 5846 | ACCACACAGGCTGGTCCC | Exon 6/Intron 6 | 6.52 | 81 |
| 688565 | 5834 | 5851 | CACTCACCACACAGGCTG | Exon 6/Intron 6 | 4.56 | 82 |
| 688566 | 5839 | 5856 | CCACACACTCACCACACA | Exon 6/Intron 6 | 0.16 | 83 |
| 688567 | 5844 | 5861 | CAGAACCACACACTCACC | Intron 6 | 0.41 | 84 |
| 688568 | 5849 | 5866 | TGACACAGAACCACACAC | Intron 6 | 0.46 | 85 |
| 688569 | 5854 | 5871 | CCATCTGACACAGAACCA | Intron 6 | 0.33 | 86 |
| 688570 | 5859 | 5876 | GCTCCCCATCTGACACAG | Intron 6 | 0.27 | 87 |
| 688571 | 5879 | 5896 | CTCTGATGTGGTTCCTCG | Intron 6 | 0.11 | 88 |
| 688572 | 5884 | 5901 | AAATGCTCTGATGTGGTT | Intron 6 | 0.10 | 89 |
| 688573 | 5889 | 5906 | CCCACAAATGCTCTGATG | Intron 6 | 0.12 | 90 |

### Example 11: Antisense Oligonucleotide Induction of Human CLN3 Exon 9 Skipping in vitro

Antisense oligonucleotides complementary to exon 9 and/or the introns flanking exon 9 of the human CLN3 pre-mRNA transcript were synthesized and tested for their ability to modulate splicing of CLN3 by skipping exon 9. The oligonucleotides were uniformly modified to contain 2'-MOE, and all internucleoside linkages are phosphorothioate linkages. The cytosine bases are 5-methylcytosine. The ASOs target the complement of GENBANK accession number NT_010393.16, truncated from nucleotides 28427600 to 28444620 (SEQ ID NO: 1), and the ASO sequences are listed in Table 13 below. The start and stop sites associated with each oligonucleotide are the 5'- and 3'-positions, respectively, of the portion of SEQ ID NO: 1 that is complementary to the ASO.

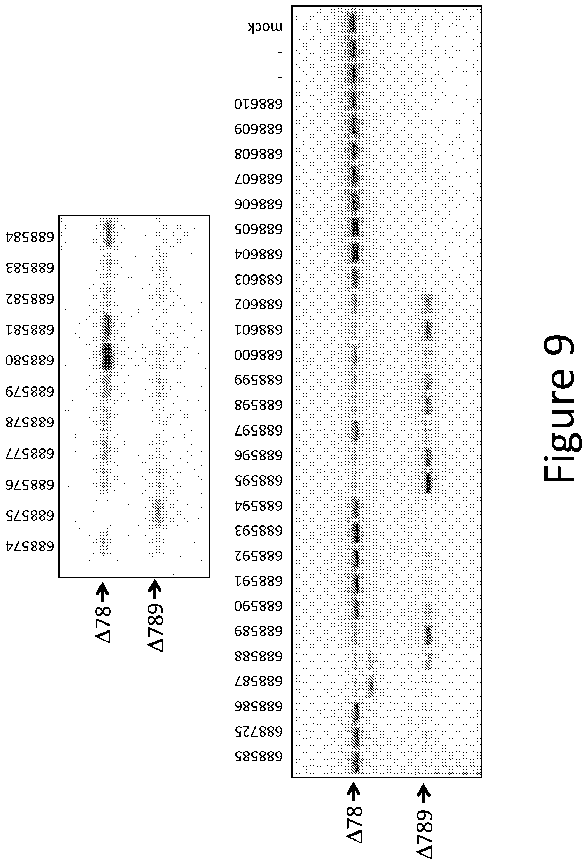

To test the ability of the antisense oligonucleotides to modulate splicing by skipping exon 9, human JNCL patient fibroblasts that are homozygous for the CLN3Δ78 deletion were transfected with 50 nM of an ASO using Cytofectin transfection reagent or were mock transfected. After 48 hours, total RNA was collected from the cells and RT-PCR was used to identify the CLN3Δ78 and CLN3Δ789 mRNA transcripts. The PCR products were analyzed by PAGE and quantitated by phosphorimager analysis (Typhoon 9400, GE Healthcare). The gel is shown in Figure 9, and the results are shown in Table 13 below as the ratios of exon 7, 8, and 9 skipped (Δ789) transcript to exon 7 and 8 skipped (Δ78) transcript. The results below illustrate that many ASOs induced CLN3 exon 9 skipping to a greater extent than the exon 9 skipping observed in mock transfected cells.

**Table 13: CLN3 Exon 9 Skipping in patient fibroblasts**

| ISIS No. | Start Site | Stop Site | Sequence (5' to 3') | CLN3 Target Region | CLN3Δ789/ CLN3Δ78 | SEQ ID NO |
|---|---|---|---|---|---|---|
| mock | n/a | n/a | n/a | n/a | 0.05 | n/a |
| 688574 | 9122 | 9139 | GGGAAGGTCCCCAGGGAC | Intron 8 | 1.14 | 91 |
| 688575 | 9127 | 9144 | TGGCTGGGAAGGTCCCCA | Intron 8 | 48.56 | 92 |
| 688576 | 9132 | 9149 | CCTACTGGCTGGGAAGGT | Intron 8 | 1.59 | 93 |
| 688577 | 9137 | 9154 | AAAACCCTACTGGCTGGG | Intron 8 | 0.46 | 94 |
| 688578 | 9142 | 9159 | GTCAGAAAACCCTACTGG | Intron 8 | 0.46 | 95 |
| 688579 | 9147 | 9164 | GCAGGGTCAGAAAACCCT | Intron 8 | 0.89 | 96 |
| 688580 | 9152 | 9169 | TGAAGGCAGGGTCAGAAA | Intron 8 | 0.20 | 97 |
| 688581 | 9157 | 9174 | TAGGATGAAGGCAGGGTC | Intron 8 | 0.08 | 98 |
| 688582 | 9162 | 9179 | AGGAGTAGGATGAAGGCA | Intron 8 | 0.79 | 99 |
| 688583 | 9167 | 9184 | TAGCTAGGAGTAGGATGA | Intron 8/Exon 9 | 1.05 | 100 |
| 688584 | 9172 | 9189 | AGAAATAGCTAGGAGTAG | Intron 8/Exon 9 | 0.22 | 101 |
| 688585 | 9177 | 9194 | CAACAAGAAATAGCTAGG | Intron 8/Exon 9 | 0.08 | 102 |
| 616725 | 9182 | 9199 | GTGAGCAACAAGAAATAG | Exon 9 | 0.29 | 36 |
| 688586 | 9187 | 9204 | GAGATGTGAGCAACAAGA | Exon 9 | 0.24 | 103 |
| 688587 | 9192 | 9209 | CTCAGGAGATGTGAGCAA | Exon 9 | 0.92 | 104 |
| 688588 | 9197 | 9214 | TGGGCCTCAGGAGATGTG | Exon 9 | 2.73 | 105 |
| 688589 | 9202 | 9219 | GGTCCTGGGCCTCAGGAG | Exon 9 | 2.73 | 106 |
| 688590 | 9207 | 9224 | TCCAGGGTCCTGGGCCTC | Exon 9 | 0.57 | 107 |
| 688591 | 9212 | 9229 | TCCCCTCCAGGGTCCTGG | Exon 9 | 0.14 | 108 |
| 688592 | 9217 | 9234 | CTTCTTCCCCTCCAGGGT | Exon 9 | 0.23 | 109 |
| 688593 | 9222 | 9239 | TGCTTCTTCTTCCCCTCC | Exon 9 | 0.06 | 110 |
| 688594 | 9227 | 9244 | CTCTCTGCTTCTTCTTCC | Exon 9 | 0.07 | 111 |
| 688595 | 9232 | 9249 | CTGCGCTCTCTGCTTCTT | Exon 9 | 8.80 | 112 |
| 688596 | 9237 | 9254 | CCGGGCTGCGCTCTCTGC | Exon 9 | 4.76 | 113 |
| 688597 | 9242 | 9259 | GGCTGCCGGGCTGCGCTC | Exon 9 | 0.51 | 114 |
| 688598 | 9262 | 9279 | GGGCCTCGGTTCTTATGA | Exon 9 | 3.74 | 115 |
| 688599 | 9282 | 9299 | CCTACCTGGCTTCGACTC | Exon 9/Intron 9 | 2.30 | 116 |
| 688600 | 9287 | 9304 | TGTCTCCTACCTGGCTTC | Exon 9/Intron 9 | 0.79 | 117 |
| 688601 | 9292 | 9309 | GTCTGTGTCTCCTACCTG | Exon 9/Intron 9 | 5.10 | 118 |
| 688602 | 9297 | 9314 | TGAGGGTCTGTGTCTCCT | Intron 9 | 1.75 | 119 |
| 688603 | 9302 | 9319 | CTCTCTGAGGGTCTGTGT | Intron 9 | 0.08 | 120 |
| 688604 | 9307 | 9324 | GTGACCTCTCTGAGGGTC | Intron 9 | 0.03 | 121 |
| 688605 | 9312 | 9329 | AGAAAGTGACCTCTCTGA | Intron 9 | 0.05 | 122 |
| 688606 | 9317 | 9334 | GAGAAAGAAAGTGACCTC | Intron 9 | 0.07 | 123 |
| 688607 | 9322 | 9339 | CCAGAGAGAAAGAAAGTG | Intron 9 | 0.05 | 124 |
| 688608 | 9327 | 9344 | CAAACCCAGAGAGAAAGA | Intron 9 | 0.08 | 125 |
| 688609 | 9332 | 9349 | AAGGCCAAACCCAGAGAG | Intron 9 | 0.02 | 126 |
| 688610 | 9337 | 9354 | AGGAAAAGGCCAAACCCA | Intron 9 | 0.01 | 127 |

### Example 12: Dose Response of Antisense Oligonucleotide Induction of Human CLN3 Exon Skipping

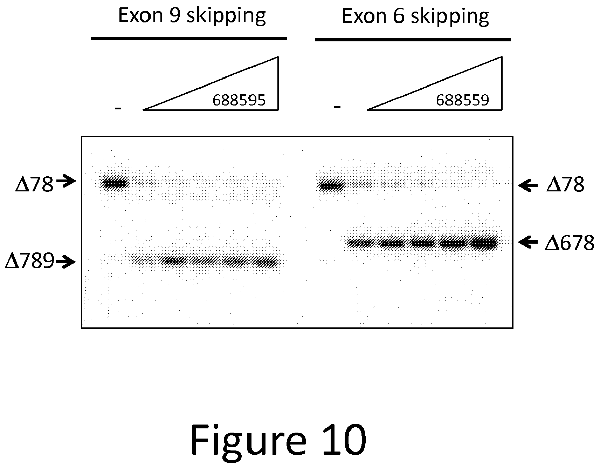

JNCL patient fibroblasts were transfected with Isis No. 688555, 688559 (see Table 12), or Isis No. 688595 (see Table 13) at a concentration listed in Table 14 below using Endo-porter transfection reagent (Gene Tools, LLC). After 48 hours, total RNA was isolated from the cells and CLN3 mRNA was detected as described in Examples 10 and 11. The gel is shown in Figure 10. The results are shown in Table 14 below and show that all three ASOs tested induced exon skipping in a dose dependent manner. "n/a" indicates that the ASO was not tested at the indicated concentration.

**Table 14: Dose Responses of an Exon 6 Skipping ASO and an Exon 9 Skipping ASO in Patient Cells**

| Concentration (nM) | CLN3Δ678/CLN3Δ 78 | | CLN3Δ789/CLN3Δ78 Isis No. 688595 |
|---|---|---|---|
| | Isis No. 688555 | Isis No. 688559 | |
| 0 | 0.00 | 0.01 | 0.04 |
| 0.39 | 0.37 | n/a | n/a |
| 0.78 | 1.13 | n/a | n/a |
| 1.56 | 4.31 | n/a | n/a |
| 3.125 | 15.24 | n/a | n/a |
| 12.5 | n/a | 3.46 | 3.84 |
| 25 | n/a | 7.36 | 19.34 |
| 50 | n/a | 11.88 | 23.16 |
| 100 | n/a | 23.66 | 21.31 |
| 200 | n/a | 70.91 | 27.9 |

### Example 13: Performance in a Water Maze by CLN3Δ78/Δ78 Mice Following ASO Treatment

In order to test whether an ASO that induces exon 6 skipping relieves symptoms of Batten Disease, homozygous CLN3Δ78/Δ78, heterozygous CLN3+/Δ78, and wild type mice were treated with 20 µg of Isis No. 616709 (see Table 3), Isis No. 527134 as a control (see Example 8), or no ASO (untreated). The ASOs were administered by intracerebroventricular injection on post-natal day one or two. Two or three months after the single injection, performance in the Morris water maze was measured based on the time each mouse took to swim to a hidden platform. Mice were placed individually in a circular pool, 48 inches in diameter, filled to a depth of 26 inches with 23 °C water. The pool walls were made opaque with white paint and placed in a room with prominent extra-maze cues at elast 16 inches from the pool edge. Four unique, proximal cues were affixed to the 8 cm high interior pool wall above water level at 0, 90, 180, and 270 degrees. Mice were placed in one of four starting quadrants facing the pool wall and allowed to swim until coming to rest atop a 4 inch square plexiglass platform submerged in 0.5 cm of water, or until a maximum of 60 seconds. Upon finding a platform, mice were left there for 20 seconds before reentry at the next start point or removal from the cage. Mice that did not find the platform within 60 seconds were guided to it by the experimenter. Trials were performed once at each starting quadrant point per session. Mice were tested for four consecutive days with two sessions of four trials each per day, and the time to reach a hidden platform was recorded. Each treatment group consisted of 13-35 mice. The results for each treatment group are shown in Table 15 below. The results show that CLN3Δ78/Δ78 homozygous mice that received an antisense oligonucleotide that induces exon 6 skipping performed similarly to WT or heterozygous mice and performed significantly better than CLN3Δ78/Δ78 homozygous mice that were untreated or received the control ASO. In particular, the differences between the control CLN3Δ78/Δ78 homozygous mice and the ASO treated CLN3Δ78/Δ78 homozygous mice and WT and heterozygous controls were significant, with p values ≤0.01 on days 2 and 4.

**Table 15: Time to Reach a Hidden Platform in a Morris Water Maze**

| Isis No. | Genotype | Number of mice in group | Trial day | Time to Platform(s) | Std. Dev. |
|---|---|---|---|---|---|
| Untreated | WT or CLN3+/Δ78 | 21 | 1 | 44.8 | 10.5 |
| | | | 2 | 24.9 | 12.0 |
| | | | 3 | 17.2 | 8.3 |
| | | | 4 | 13.5 | 7.6 |
| Untreated or 527134 | CLN3Δ78/Δ78 | 35 | 1 | 48.9 | 10.0 |
| | | | 2 | 35.9 | 14.5 |
| | | | 3 | 27.4 | 12.7 |
| | | | 4 | 26.0 | 14.2 |
| 616709 | CLN3Δ78/Δ78 | 13 | 1 | 43.0 | 7.6 |
| | | | 2 | 19.3 | 7.5 |
| | | | 3 | 24.1 | 12.9 |
| | | | 4 | 16.8 | 8.4 |

Further embodiments:
1. A compound comprising a modified oligonucleotide consisting of 8 to 30 linked nucleosides and having a nucleobase sequence comprising a complementary region, wherein the complementary region comprises at least 8 contiguous nucleobases and is complementary to an equal-length portion of a target region of a CLN3 transcript.
2. The compound of embodiment 1, wherein the target region of the CLN3 transcript comprises at least a portion of exon 6 of the CLN3 transcript.
3. The compound of embodiment 1, wherein the target region of the CLN3 transcript comprises at least a portion of exon 9 of the CLN3 transcript.
4. The compound of embodiment 1, wherein the target region of the CLN3 transcript comprises at least a portion of intron 5 of the CLN3 transcript.
5. The compound of embodiment 1, wherein the target region of the CLN3 transcript comprises at least a portion of intron 6 of the CLN3 transcript.
6. The compound of embodiment 1, wherein the target region of the CLN3 transcript comprises at least a portion of intron 9 of the CLN3 transcript.
7. The compound of embodiment 1, wherein the target region of the CLN3 transcript comprises at least a portion of intron 10 of the CLN3 transcript.
8. The compound of any of embodiments 1 to 7, wherein the complementary region of the modified oligonucleotide is 100% complementary to the target region.
9. The compound of any of embodiments 1 to 8, wherein the complementary region of the modified oligonucleotide comprises at least 10 contiguous nucleobases.
10. The compound of any of embodiments 1 to 8, wherein the complementary region of the modified oligonucleotide comprises at least 12 contiguous nucleobases.
11. The compound of any of embodiments 1 to 8, wherein the complementary region of the modified oligonucleotide comprises at least 14 contiguous nucleobases.
12. The compound of any of embodiments 1 to 8, wherein the complementary region of the modified oligonucleotide comprises at least 15 contiguous nucleobases.
13. The compound of any of embodiments 1 to 8, wherein the complementary region of the modified oligonucleotide comprises at least 16 contiguous nucleobases.
14. The compound of any of embodiments 1 to 8, wherein the complementary region of the modified oligonucleotide comprises at least 17 contiguous nucleobases.
15. The compound of any of embodiments 1 to 8, wherein the complementary region of the modified oligonucleotide comprises at least 18 contiguous nucleobases.
16. The compound of any of embodiments 1 to 8, wherein the complementary region of the modified oligonucleotide comprises at least 19 contiguous nucleobases.
17. The compound of any of embodiments 1 to 8, wherein the complementary region of the modified oligonucleotide comprises at least 20 contiguous nucleobases.
18. The compound of any of embodiments 1 to 17, wherein the nucleobase sequence of the oligonucleotide is at least 80% complementary to an equal-length region of the CLN3 transcript, as measured over the entire length of the oligonucleotide.
19. The compound of any of embodiments 1 to 17, wherein the nucleobase sequence of the oligonucleotide is at least 90% complementary to an equal-length region of the CLN3 transcript, as measured over the entire length of the oligonucleotide.
20. The compound of any of embodiments 1 to 17, wherein the nucleobase sequence of the oligonucleotide is 100%complementary to an equal-length region of the CLN3 transcript, as measured over the entire length of the oligonucleotide.
21. The compound of any of embodiments 1-2 or 8 to 20, wherein the target region is within nucleobase 5053 and nucleobase 5070 of SEQ ID NO.: 2.
22. The compound of any of embodiments 1-2 or 8 to 20, wherein the target region is within nucleobase 5082 and nucleobase 5119 of SEQ ID NO.: 2.
23. The compound of any of embodiments 1-2 or 8 to 20, wherein the target region is within nucleobase 5082 and nucleobase 5099 of SEQ ID NO.: 2.
24. The compound of any of embodiments 1-2 or 8 to 20, wherein the target region is within nucleobase 5086 and nucleobase 5103 of SEQ ID NO.: 2.
25. The compound of any of embodiments 1-2 or 8 to 20, wherein the target region is within nucleobase 5090 and nucleobase 5107 of SEQ ID NO.: 2.
26. The compound of any of embodiments 1-2 or 8 to 20, wherein the target region is within nucleobase 5094 and nucleobase 5111 of SEQ ID NO.: 2.
27. The compound of any of embodiments 1-2 or 8 to 20, wherein the target region is within nucleobase 5098 and nucleobase 5115 of SEQ ID NO.: 2.
28. The compound of any of embodiments 1-2 or 8 to 20, wherein the target region is within nucleobase 5102 and nucleobase 5119 of SEQ ID NO.: 2.
29. The compound of any of embodiments 1-2, 5, or 8 to 20, wherein the target region is within nucleobase 5126 and nucleobase 5143 of SEQ ID NO.: 2.
30. The compound of any of embodiments 1-2, 5 or 8 to 20, wherein the target region is within nucleobase 5134 and nucleobase 5155 of SEQ ID NO.: 2.
31. The compound of any of embodiments 1-2, 5, or 8 to 20, wherein the target region is within nucleobase 5134 and nucleobase 5151 of SEQ ID NO.: 2.
32. The compound of any of embodiments 1-2, 5 or 8 to 20, wherein the target region is within nucleobase 5138 and nucleobase 5155 of SEQ ID NO.: 2.
33. The compound of any of embodiments 1, 3, or 8 to 20, wherein the target region is within nucleobase 7366 and nucleobase 7411 of SEQ ID NO.: 2.
34. The compound of any of embodiments 1, 3, or 8 to 20, wherein the target region is within nucleobase 7366 and nucleobase 7383 of SEQ ID NO.: 2.
35. The compound of any of embodiments 1, 3, or 8 to 20, wherein the target region is within nucleobase 7370 and nucleobase 7387 of SEQ ID NO.: 2.
36. The compound of any of embodiments 1, 3, or 8 to 20, wherein the target region is within nucleobase 7371 and nucleobase 7388 of SEQ ID NO.: 2.
37. The compound of any of embodiments 1, 3, or 8 to 20, wherein the target region is within nucleobase 7387 and nucleobase 7404 of SEQ ID NO.: 2.
38. The compound of any of embodiments 1, 3, or 8 to 20, wherein the target region is within nucleobase 7394 and nucleobase 7411 of SEQ ID NO.: 2.
39. The compound of any of embodiments 1, 3, 6, or 8 to 20, wherein the target region is within nucleobase 7454 and nucleobase 7471 of SEQ ID NO.: 2.
40. The compound of any of embodiments 1, 3, 6, or 8 to 20, wherein the target region is within nucleobase 7462 and nucleobase 7483 of SEQ ID NO.: 2.
41. The compound of any of embodiments 1, 3, 6, or 8 to 20, wherein the target region is within nucleobase 7462 and nucleobase 7479 of SEQ ID NO.: 2.
42. The compound of any of embodiments 1, 3, 6, or 8 to 20, wherein the target region is within nucleobase 7466 and nucleobase 7483 of SEQ ID NO.: 2.
43. The compound of any of embodiments 1-42, wherein the nucleobase sequence of the antisense oligonucleotide comprises any one of SEQ ID NOs: 3 to 60.
44. The compound of any of embodiments 1-43, wherein the modified oligonucleotide comprises at least one modified nucleoside.
45. The compound of embodiment 44, wherein at least one modified nucleoside comprises a modified sugar moiety.
46. The compound of embodiment 45, wherein at least one modified sugar moiety is a 2'-substituted sugar moiety.
47. The compound of embodiment 46, wherein the 2'-substitutent of at least one 2'-substituted sugar moiety is selected from among: 2'-OMe, 2'-F, and 2'-MOE.
48. The compound of any of embodiments 44-47, wherein the 2'-substiuent of at least one 2'-substituted sugar moiety is a 2'-MOE.
49. The compound of any of embodiments 1-48, wherein at least one modified sugar moiety is a bicyclic sugar moiety.
50. The compound of embodiment 49, wherein at least one bicyclic sugar moiety is LNA or cEt.
51. The compound of any of embodiments 1-51, wherein at least one sugar moiety is a sugar surrogate.
52. The compound of embodiment 51, wherein at least one sugar surrogate is a morpholino.
53. The compound of embodiment 51, wherein at least one sugar surrogate is a modified morpholino.
54. The compound of any of embodiment 1-53, wherein the modified oligonucleotide comprises at least 5 modified nucleosides, each independently comprising a modified sugar moiety.
55. The compound of embodiment 54, wherein the modified oligonucleotide comprises at least 10 modified nucleosides, each independently comprising a modified sugar moiety.
56. The compound of embodiment 54, wherein the modified oligonucleotide comprises at least 15 modified nucleosides, each independently comprising a modified sugar moiety.
57. The compound of embodiment 54, wherein each nucleoside of the modified oligonucleotide is a modified nucleoside, each independently comprising a modified sugar moiety
58. The compound of any of embodiments 1-57, wherein the modified oligonucleotide comprises at least two modified nucleosides comprising modified sugar moieties that are the same as one another.
59. The compound of any of embodiments 1-57, wherein the modified oligonucleotide comprises at least two modified nucleosides comprising modified sugar moieties that are different from one another.
60. The compound of any of embodiments 1-59, wherein the modified oligonucleotide comprises a modified region of at least 5 contiguous modified nucleosides.
61. The compound of any of embodiments 1 to 60, wherein the modified oligonucleotide comprises a modified region of at least 10 contiguous modified nucleosides.
62. The compound of any of embodiments 1 to 61, wherein the modified oligonucleotide comprises a modified region of at least 15 contiguous modified nucleosides.
63. The compound of any of embodiments 1 to 61, wherein the modified oligonucleotide comprises a modified region of at least 20 contiguous modified nucleosides.
64. The compound of any of embodiments 58 to 63, wherein each modified nucleoside of the modified region has a modified sugar moiety independently selected from among: 2'-F, 2'-OMe, 2'-MOE, cEt, LNA, morpholino, and modified morpholino.
65. The compound of any of embodiments 58 to 64, wherein the modified nucleosides of the modified region each comprise the same modification as one another.
66. The compound of embodiment 65, wherein the modified nucleosides of the modified region each comprise the same 2'-substituted sugar moiety.
67. The compound of embodiment 65, wherein the 2'-substituted sugar moiety of the modified nucleosides of the region of modified nucleosides is selected from 2'-F, 2'-OMe, and 2'-MOE.
68. The compound of embodiment 67, wherein the 2'-substituted sugar moiety of the modified nucleosides of the region of modified nucleosides is 2'-MOE.
69. The compound of embodiment 65, wherein the modified nucleosides of the region of modified nucleosides each comprise the same bicyclic sugar moiety.
70. The compound of embodiment 69, wherein the bicyclic sugar moiety of the modified nucleosides of the region of modified nucleosides is selected from LNA and cEt.
71. The compound of embodiment 65, wherein the modified nucleosides of the region of modified nucleosides each comprises a sugar surrogate.
72. The compound of embodiment 71, wherein the sugar surrogate of the modified nucleosides of the region of modified nucleosides is a morpholino.
73. The compound of embodiment 71, wherein the sugar surrogate of the modified nucleosides of the region of modified nucleosides is a modified morpholino.
74. The compound of any of embodiments 1 to 73, wherein the modified nucleotide comprises no more than 4 contiguous naturally occurring nucleosides.
75. The compound of any of embodiments 1 to 74, wherein each nucleoside of the modified oligonucleotide is a modified nucleoside.
76. The compound of embodiment 75 wherein each modified nucleoside comprises a modified sugar moiety.
77. The compound of embodiment 76, wherein the modified nucleosides of the modified oligonucleotide comprise the same modification as one another.
78. The compound of embodiment 77, wherein the modified nucleosides of the modified oligonucleotide each comprise the same 2'-substituted sugar moiety.
79. The compound of embodiment 78, wherein the 2'-substituted sugar moiety of the modified oligonucleotide is selected from 2'-F, 2'-OMe, and 2'-MOE.
80. The compound of embodiment 78, wherein the 2'-substituted sugar moiety of the modified oligonucleotide is 2'-MOE.
81. The compound of embodiment 77, wherein the modified nucleosides of the modified oligonucleotide each comprise the same bicyclic sugar moiety.
82. The compound of embodiment 81, wherein the bicyclic sugar moiety of the modified oligonucleotide is selected from LNA and cEt.
83. The compound of embodiment 77, wherein the modified nucleosides of the modified oligonucleotide each comprises a sugar surrogate.
84. The compound of embodiment 83, wherein the sugar surrogate of the modified oligonucleotide is a morpholino.
85. The compound of embodiment 83, wherein the sugar surrogate of the modified oligonucleotide is a modified morpholino.
86. The compound of any of embodiments 1 to 85, wherein the modified oligonucleotide comprises at least one modified internucleoside linkage.
87. The compound of embodiment 86, wherein each internucleoside linkage is a modified internucleoside linkage.
88. The compound of embodiment 86 or 87, comprising at least one phosphorothioate internucleoside linkage.
89. The compound of embodiment 86, wherein each internucleoside linkage is a modified internucleoside linkage and wherein each internucleoside linkage comprises the same modification.
90. The compound of embodiment 89, wherein each internucleoside linkage is a phosphorothioate internucleoside linkage.
91. The compound of any of embodiments 1 to 90, comprising at least one conjugate.
92. The compound of any of embodiments 1 to 91 consisting of the modified oligonucleotide.
93. The compound of any of embodiments 1 to 92, wherein the compound modulates splicing of the CLN3 transcript.
94. The compound of any of embodiments 1 to 93, having a nucleobase sequence comprising any of the sequences as set forth in SEQ ID NOs. 3 to 60.
95. The compound of any of embodiments 1, 2, 4 to 5, or 8 to 93, having a nucleobase sequence comprising any of the sequences as set forth in SEQ ID NOs. 8, 16, 17, 18, 19, 20, 21, 27, 29, or 30.
96. The compound of any of embodiments 1, 3, or 6 to 93, having a nucleobase sequence comprising any of the sequences as set forth in SEQ ID NOs. 38, 39, 40, 41, 43, 55, 57, or 58.
97. The compound of any of embodiments 1, 2, or 8 to 93, having a nucleobase sequence comprising SEQ ID NO. 20.
98. A pharmaceutical composition comprising a compound according to any of embodiments 1-97 and a pharmaceutically acceptable carrier or diluent.
99. The pharmaceutical composition of embodiment 98, wherein the pharmaceutically acceptable carrier or diluent is sterile saline.
100. A method of modulating splicing of a CLN3 transcript in a cell comprising contacting the cell with a compound according to any of embodiments 1-99.
101. The method of embodiment 100, wherein the cell is in vitro.
102. The method of embodiment 100, wherein the cell is in an animal.
103. The method of any of embodiments 100 to 102, wherein the amount of CLN3 mRNA without exon 6 is increased.
104. The method of any of embodiments 100 to 102, wherein the amount of CLN3 mRNA without exon 9 is increased.
105. The method of any of embodiments 100 to 102, wherein the amount of CLN3 mRNA with exon 10 is increased.
106. The method of any of embodiments 100 to 105, wherein the CLN3 transcript is transcribed from a *CLN3*Δ*78* gene.
107. A method of modulating the expression of CLN3 in a cell, comprising contacting the cell with a compound according to any of embodiments 1-99.
108. The method of embodiment 84, wherein the cell is in vitro.
109. The method of embodiment 84, wherein the cell is in an animal.
110. A method comprising administering the compound according to any of embodiments 1-97 or the pharmaceutical composition of embodiments 98 or 99 to an animal.
111. The method of embodiment 108, wherein the administration is intracerebroventricular.
112. The method of embodiment 108, wherein the administration is into the central nervous sysem.
113. The method of any of embodiments 108-110, wherein the animal has one or more symptoms associated with Batten Disease.
114. The method of any of embodiments 108-110, wherein the administration results in amelioration of at least one symptom of Batten Disease.
115. The method of any of embodiments 108 to 112, wherein the animal is a mouse.
116. The method of any of embodiments 108 to 112, wherein the animal is a human.
117. A method of preventing or slowing one or more symptoms Batten Disease, comprising administering the compound according to any of embodiments 1-97 or the pharmaceutical composition of embodiments 98 or 99 to an animal in need thereof.
118. Use of the compound according to any of embodiments 1-97 or the pharmaceutical composition of embodiments 98 or 99 for the preparation of a medicament for use in the treatment of Batten Disease.
119. Use of the compound according to any of embodiments 1-97 or the pharmaceutical composition of embodiments 98 or 99 for the preparation of a medicament for use in the amelioration of one or more symptoms Batten Disease.
120. A compound comprising a modified oligonucleotide consisting of 8 to 30 linked nucleosides and having a nucleobase sequence comprising a complementary region, wherein the complementary region comprises at least 8 contiguous nucleobases and is complementary to an equal-length portion of a target region of a CLN3 transcript.
121. The compound of embodiment 120, wherein the CLN3 transcript comprises the nucleobase sequence of SEQ ID NO. 1.
122. The compound of embodiment 120 or 121, wherein the complementary region of the modified oligonucleotide is 100% complementary to the target region.
123. The compound of any of embodiments 120 to 122, wherein the complementary region of the modified oligonucleotide comprises at least 10 contiguous nucleobases.
124. The compound of any of embodiments 120 to 122, wherein the complementary region of the modified oligonucleotide comprises at least 12 contiguous nucleobases.
125. The compound of any of embodiments 120 to 122, wherein the complementary region of the modified oligonucleotide comprises at least 14 contiguous nucleobases.
126. The compound of any of embodiments 120 to 122, wherein the complementary region of the modified oligonucleotide comprises at least 15 contiguous nucleobases.
127. The compound of any of embodiments 120 to 122, wherein the complementary region of the modified oligonucleotide comprises at least 16 contiguous nucleobases.
128. The compound of any of embodiments 120 to 122, wherein the complementary region of the modified oligonucleotide comprises at least 17 contiguous nucleobases.
129. The compound of any of embodiments 120 to 122, wherein the complementary region of the modified oligonucleotide comprises at least 18 contiguous nucleobases.
130. The compound of any of embodiments 120 to 129, wherein the nucleobase sequence of the modified oligonucleotide is at least 80% complementary to an equal-length region of the CLN3 transcript, as measured over the entire length of the oligonucleotide.
131. The compound of any of embodiments 120 to 129, wherein the nucleobase sequence of the modified oligonucleotide is at least 90% complementary to an equal-length region of the CLN3 transcript, as measured over the entire length of the oligonucleotide.
132. The compound of any of embodiments 120 to 129, wherein the nucleobase sequence of the modified oligonucleotide is 100% complementary to an equal-length region of the CLN3 transcript, as measured over the entire length of the oligonucleotide.
133. The compound of any of embodiments 120-132, wherein the target region is within nucleobase 5704-5721, 5709-5726, 5714-5731, 5734-5751, 5764-5781, 5769-5786, 5774-5791, 5779-5796, 5784-5801, 5789-5806, 5794-5811, 5799-5816, 5804-5821, 5809-5826, 5814-5831, 5819-5836, 5824-5841, 5829-5846, 5834-5851, 5839-5856, 5844-5861, 5849-5866, 5854-5871, 5859-5876, 5879-5896, 5884-5901, or 5889-5906 of SEQ ID NO.: 1.
134. The compound of any of embodiments 120-133, wherein the target region is within nucleobase 5784- 5801 of SEQ ID NO.: 1.
135. The compound of any of embodiments 120-132, wherein the target region is within nucleobase 5804-5821 of SEQ ID NO.: 1.
136. The compound of any of embodiments 120-132, wherein the target region is within nucleobase 9122-9139, 9127-9144, 9132-9149, 9137-9154, 9142-9159, 9147-9164, 9152-9169, 9157-9174, 9162-9179, 9167-9184, 9172-9189, 9177-9194, 9182-9199, 9187-9204, 9192-9209, 9197-9214, 9202-9219, 9207-9224, 9212-9229, 9217-9234, 9222-9239, 9227-9244, 9232-9249, 9237-9254, 9242-9259, 9262-9279, 9282-9299, 9287-9304, 9292-9309, 9297-9314, 9302-9319, 9307-9324, 9312-9329, 9317-9334, 9322-9339, 9327-9344, 9332-9349, or 9337-9354 of SEQ ID NO.: 1.
137. The compound of any of embodiments 120-132, wherein the modified oligonucleotide has a nucleobase sequence comprising any of the sequences as set forth in SEQ ID NOs: 63, 64, 65, 66, 67, 68, 69, 70, 71, 72, 73, 74, 75, 76, 77, 78, 79, 80, 81, 82, 83, 84, 85, 86, 87, 88, 89, or 90.
138. The compound of any of embodiments 120-132, wherein the modified oligonucleotide has a nucleobase sequence consisting of the nucleobase sequence of any one of SEQ ID NOs: 63, 64, 65, 66, 67, 68, 69, 70, 71, 72, 73, 74, 75, 76, 77, 78, 79, 80, 81, 82, 83, 84, 85, 86, 87, 88, 89, or 90.
139. The compound of any of embodiments 120-132, wherein the modified oligonucleotide has a nucleobase sequence comprising the nucleobase sequence of SEQ ID NO. 72.
140. The compound of embodiment 138, wherein the modified oligonucleotide has a nucleobase sequence consisting of the nucleobase sequence of SEQ ID NO. 72.
141. The compound of any of embodiments 120-132, wherein the modified oligonucleotide has a nucleobase sequence comprising the nucleobase sequence of SEQ ID NO. 73.
142. The compound of embodiment 138, wherein the modified oligonucleotide has a nucleobase sequence consisting of the nucleobase sequence of SEQ ID NO. 73.
143. The compound of any of embodiments 120-132, wherein the modified oligonucleotide has a nucleobase sequence comprising any of the sequences as set forth in SEQ ID NOs: 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, or 127.
144. The compound of any of embodiments 120-132, wherein the modified oligonucleotide has a nucleobase sequence consisting of the nucleobase sequence of any one of SEQ ID NOs: 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, or 127.
145. The compound of any of embodiments 120-132, wherein the modified oligonucleotide has a nucleobase sequence comprising the nucleobase sequence of SEQ ID NO. 112.
146. The compound of embodiment 144, wherein the modified oligonucleotide has a nucleobase sequence consisting of the nucleobase sequence of SEQ ID NO. 112.
147. The compound of any of embodiments 120-146, wherein the modified oligonucleotide comprises at least one modified nucleoside.
148. The compound of any of embodiments 120-147, wherein each nucleoside of the modified oligonucleotide is a modified nucleoside selected from among: 2'-OMe, 2'-F, and 2'-MOE or a sugar surrogate.
149. The compound of embodiment 148, wherein the modified nucleoside is 2'-MOE.
150. The compound of embodiment 148, wherein the modified nucleoside is a morpholino.
151. The compound of embodiment 147, wherein at least one modified nucleoside comprises a modified sugar moiety.
152. The compound of embodiment 151, wherein at least one modified sugar moiety is a 2'-substituted sugar moiety.
153. The compound of embodiment 152, wherein the 2'-substitutent of at least one 2'-substituted sugar moiety is selected from among: 2'-OMe, 2'-F, and 2'-MOE.
154. The compound of any of embodiments 152-153, wherein the 2'-substiuent of at least one 2'-substituted sugar moiety is a 2'-MOE.
155. The compound of any of embodiments 120-154, wherein at least one modified sugar moiety is a bicyclic sugar moiety.
156. The compound of embodiment 155, wherein at least one bicyclic sugar moiety is LNA or cEt.
157. The compound of any of embodiments 120-1563, wherein at least one sugar moiety is a sugar surrogate.
158. The compound of embodiment 157, wherein at least one sugar surrogate is a morpholino.
159. The compound of embodiment 158, wherein at least one sugar surrogate is a modified morpholino.
160. The compound of any of embodiments 120-159, wherein the modified oligonucleotide comprises at least 5 modified nucleosides, each independently comprising a modified sugar moiety.
161. The compound of any of embodiments 120-159, wherein the modified oligonucleotide comprises at least 10 modified nucleosides, each independently comprising a modified sugar moiety.
162. The compound of any of embodiments 120-159, wherein the modified oligonucleotide comprises at least 15 modified nucleosides, each independently comprising a modified sugar moiety.
163. The compound of any of embodiments 120-159, wherein each nucleoside of the modified oligonucleotide is a modified nucleoside, each independently comprising a modified sugar moiety.
164. The compound of any of embodiments 120-163, wherein the modified oligonucleotide comprises at least two modified nucleosides comprising modified sugar moieties that are the same as one another.
165. The compound of any of embodiments 120-163, wherein the modified oligonucleotide comprises at least two modified nucleosides comprising modified sugar moieties that are different from one another.
166. The compound of any of embodiments 120-165, wherein the modified oligonucleotide comprises a modified region of at least 5 contiguous modified nucleosides.
167. The compound of any of embodiments 120-165, wherein the modified oligonucleotide comprises a modified region of at least 10 contiguous modified nucleosides.
168. The compound of any of embodiments 120-165, wherein the modified oligonucleotide comprises a modified region of at least 15 contiguous modified nucleosides.
169. The compound of any of embodiments 120-165, wherein the modified oligonucleotide comprises a modified region of at least 16 contiguous modified nucleosides.
170. The compound of any of embodiments 120-165, wherein the modified oligonucleotide comprises a modified region of at least 17 contiguous modified nucleosides.
171. The compound of any of embodiments 120-165, wherein the modified oligonucleotide comprises a modified region of at least 18 contiguous modified nucleosides.
172. The compound of any of embodiments 120-165, wherein the modified oligonucleotide comprises a modified region of at least 20 contiguous modified nucleosides.
173. The compound of any of embodiments 166 to1 72, wherein each modified nucleoside of the modified region has a modified sugar moiety independently selected from among: 2'-F, 2'-OMe, 2'-MOE, cEt, LNA, morpholino, and modified morpholino.
174. The compound of any of embodiments 166 to 173, wherein the modified nucleosides of the modified region each comprise the same modification as one another.
175. The compound of embodiment 174, wherein the modified nucleosides of the modified region each comprise the same 2'-substituted sugar moiety.
176. The compound of embodiment 174, wherein the 2'-substituted sugar moiety of the modified nucleosides of the region of modified nucleosides is selected from 2'-F, 2'-OMe, and 2'-MOE.
177. The compound of embodiment 174, wherein the 2'-substituted sugar moiety of the modified nucleosides of the region of modified nucleosides is 2'-MOE.
178. The compound of embodiment 174, wherein the modified nucleosides of the region of modified nucleosides each comprise the same bicyclic sugar moiety.
179. The compound of embodiment 178, wherein the bicyclic sugar moiety of the modified nucleosides of the region of modified nucleosides is selected from LNA and cEt.
180. The compound of embodiment 174, wherein the modified nucleosides of the region of modified nucleosides each comprises a sugar surrogate.
181. The compound of embodiment 180, wherein the sugar surrogate of the modified nucleosides of the region of modified nucleosides is a morpholino.
182. The compound of embodiment 180, wherein the sugar surrogate of the modified nucleosides of the region of modified nucleosides is a modified morpholino.
183. The compound of any of embodiments 120 to 182, wherein the modified nucleotide comprises no more than 4 contiguous naturally occurring nucleosides.
184. The compound of any of embodiments 120 to 183, wherein each nucleoside of the modified oligonucleotide is a modified nucleoside.
185. The compound of embodiment 184 wherein each modified nucleoside comprises a modified sugar moiety.
186. The compound of embodiment 185, wherein the modified nucleosides of the modified oligonucleotide comprise the same modification as one another.
187. The compound of embodiment 186, wherein the modified nucleosides of the modified oligonucleotide each comprise the same 2'-substituted sugar moiety.
188. The compound of embodiment 187, wherein the 2'-substituted sugar moiety of the modified oligonucleotide is selected from 2'-F, 2'-OMe, and 2'-MOE.
189. The compound of embodiment 187, wherein the 2'-substituted sugar moiety of the modified oligonucleotide is 2'-MOE.
190. The compound of embodiment 186, wherein the modified nucleosides of the modified oligonucleotide each comprise the same bicyclic sugar moiety.
191. The compound of embodiment 190, wherein the bicyclic sugar moiety of the modified oligonucleotide is selected from LNA and cEt.
192. The compound of embodiment 186, wherein the modified nucleosides of the modified oligonucleotide each comprises a sugar surrogate.
193. The compound of embodiment 192, wherein the sugar surrogate of the modified oligonucleotide is a morpholino.
194. The compound of embodiment 192, wherein the sugar surrogate of the modified oligonucleotide is a modified morpholino.
195. The compound of any of embodiments 120 to 194, wherein the modified oligonucleotide comprises at least one modified internucleoside linkage.
196. The compound of embodiment 195, wherein each internucleoside linkage is a modified internucleoside linkage.
197. The compound of embodiment 195 or 196, comprising at least one phosphorothioate internucleoside linkage.
198. The compound of embodiment 196, wherein each internucleoside linkage is a modified internucleoside linkage and wherein each internucleoside linkage comprises the same modification.
199. The compound of embodiment 198, wherein each internucleoside linkage is a phosphorothioate internucleoside linkage.
200. The compound of any of embodiments 120 to 200, comprising at least one conjugate.
201. The compound of any of embodiments 120 to 200, consisting of the modified oligonucleotide.
202. The compound of any of embodiments 120 to 201, wherein the compound modulates splicing of the CLN3 transcript.
203. A pharmaceutical composition comprising a compound according to any of embodiments 120-202 and a pharmaceutically acceptable carrier or diluent.
204. The pharmaceutical composition of embodiment 202, wherein the pharmaceutically acceptable carrier or diluent is sterile saline.
205. A method of modulating splicing of a CLN3 transcript in a cell comprising contacting the cell with a compound according to any of embodiments 120-204.
206. The method of embodiment 205, wherein the cell is in vitro.
207. The method of embodiment 205, wherein the cell is in an animal.
208. The method of any of embodiments 205 to 207, wherein the amount of CLN3 mRNA without exon 6 is increased.
209. The method of any of embodiments 205 to 207, wherein the amount of CLN3 mRNA without exon 9 is increased.
210. The method of any of embodiments 205 to 209, wherein the amount of CLN3 mRNA with exon 10 is increased.
211. The method of any of embodiments 205 to 210, wherein the CLN3 transcript is transcribed from a *CLN3*Δ*78* gene.
212. A method of modulating the expression of CLN3 in a cell, comprising contacting the cell with a compound according to any of embodiments 120-204.
213. The method of embodiment 212, wherein the cell is in vitro.
214. The method of embodiment 212, wherein the cell is in an animal.
215. A method comprising administering the compound of any of embodiments 120-204 to an animal.
216. The method of embodiment 215, wherein the administration is intracerebroventricular.
217. The method of embodiment 215, wherein the administration is into the central nervous sysem.
218. The method of any of embodiments 215 to 217, wherein the animal has one or more symptoms associated with Batten Disease.
219. The method of any of embodiments 215 to 217, wherein the administration results in amelioration of at least one symptom of Batten Disease.
220. The method of any of embodiments 215 to 219, wherein the animal is a mouse.
221. The method of any of embodiments 215 to 219, wherein the animal is a human.
222. A method of preventing or slowing one or more symptoms Batten Disease, comprising administering the compound according to any of embodiments 120-204 to an animal in need thereof.
223. The method of embodiment 222, wherein the animal is a human.
224. Use of the compound according to any of embodiments 120-204 for the preparation of a medicament for use in the treatment of Batten Disease.
225. Use of the compound according to any of embodiments 120-204 for the preparation of a medicament for use in the amelioration of one or more symptoms Batten Disease.

## Claims

1. An oligomeric compound comprising a modified oligonucleotide consisting of 12 to 30 linked nucleosides and having a nucleobase sequence comprising a complementary region, wherein:
(i) the complementary region comprises at least 8 contiguous nucleobases and is 100% complementary to an equal-length region of
exon 6, an intron adjacent to exon 6 and downstream of exon 6, an intron adjacent to exon 6 and upstream of exon 6, or an intron-exon splice junction adjacent to exon 6, of a CLN3 transcript, or
exon 9, an intron adjacent to exon 9 and downstream of exon 9, an intron adjacent to exon 9 and upstream of exon 9, or an intron-exon splice junction adjacent to exon 9, of a CLN3 transcript;
(ii) the nucleobase sequence of the modified oligonucleotide is at least 90% complementary to an equal-length region of the CLN3 transcript, as measured over the entire length of the oligonucleotide;
(iii) the modified oligonucleotide does not comprise more than 4 contiguous unmodified 2'-deoxynucleosides; and
(iv) the modified oligonucleotide comprises at least one modified nucleoside comprising a modified sugar moiety, or at least one modified internucleoside linkage.

2. The oligomeric compound of claim 1, wherein the nucleobase sequence of the modified oligonucleotide comprises a portion of at least 12 contiguous nucleobases, wherein the portion is complementary to:
(i) an equal length portion of nucleobases 5699-5751 or 5764- 5906 of SEQ ID NO: 1, or an equal length portion of nucleobases 5030-5155 of SEQ ID NO: 2; or
(ii) an equal length portion of nucleobases 9122-9279 or 9282-9354 of SEQ ID NO: 1, or an equal length portion of nucleobases 7338-7491 of SEQ ID NO: 2.

3. The oligomeric compound of claim 1 or claim 2, wherein the modified oligonucleotide has a nucleobase sequence comprising at least 12 contiguous nucleobases of any of the nucleobase sequences of SEQ ID NOs: 3-60 and 63-127.

4. The oligomeric compound of any of claims 1-3, wherein:
a. the nucleobase sequence of the modified oligonucleotide is 100% complementary to an equal length portion of the CLN3 transcript, as measured over the entire length of the oligonucleotide;
b. the CLN3 transcript has the nucleobase sequence of SEQ ID NO: 1 or SEQ ID NO: 2; and/or
c. the modified oligonucleotide consists of 12 to 20, 12 to 25, 13 to 20, 13 to 25, 13 to 30, 14 to 20, 14 to 25, 14 to 30, 15 to 20, 15 to 25, 15 to 30, 16 to 20, 16 to 25, 16 to 30, 17 to 20, 17 to 25, 17 to 30, 18 to 20, 18 to 25, or 18 to 30 linked nucleosides.

5. The oligomeric compound of any of claims 1-4, consisting of a single-stranded modified oligonucleotide.

6. The oligomeric compound of any of claims 1-5, wherein the modified oligonucleotide comprises at least one modified nucleoside, optionally wherein the at least one modified nucleoside comprises a modified sugar moiety.

7. The oligomeric compound of any of claims 1-6, wherein the modified oligonucleotide comprises at least 5, at least 10, at least 15, at least 16, at least 17, or 18 modified nucleosides, each independently comprising a modified sugar moiety.

8. The oligomeric compound of claim 6 or claim 7, wherein at least one modified sugar moiety is a 2'-substituted sugar moiety, optionally wherein the 2'-substituent of the 2'-substituted sugar moiety is selected from among: 2'-OMe, 2'-F, and 2'-MOE.

9. The oligomeric compound of any of claims 6-8, wherein at least one modified sugar moiety is a bicyclic sugar moiety, optionally wherein the bicyclic sugar moiety is LNA or cEt.

10. The oligomeric compound of any of claims 6-9, wherein at least one sugar moiety is a morpholino.

11. The oligomeric compound of any of claims 1-10, wherein the modified oligonucleotide comprises at least one modified internucleoside linkage, optionally wherein the at least one modified internucleoside linkage is a phosphorothioate internucleoside linkage.

12. A pharmaceutical composition comprising an oligomeric compound according to any of claims 1-11, and a pharmaceutically acceptable carrier or diluent, optionally wherein the pharmaceutically acceptable carrier or diluent is sterile saline.

13. The pharmaceutical composition of claim 12, wherein the pharmaceutical composition comprises a pharmaceutically acceptable salt of the compound, optionally wherein the pharmaceutically acceptable salt is a sodium salt or a potassium salt.

14. The oligomeric compound of any of claims 1-11, or the pharmaceutical composition of claim 12 or claim 13, for use in the treatment of Batten Disease or for use in delaying or preventing the onset of Batten Disease.

15. The oligomeric compound or pharmaceutical composition for use according to claim 14, wherein the oligomeric compound or pharmaceutical composition ameliorates at least one symptom of Batten disease, optionally wherein the at least one symptom of Batten disease is loss of motor function, seizures, vision loss, loss of cognitive function, and premature death.
